# EUROPEAN PATENT APPLICATION

(11) **EP 2 308 840 A1**
(43) Date of publication of application: **13.04.2011**
(21) Application number: 10178649.9
(22) Date of filing: 29.06.2006
(51) Int. Cl.: C07D 211/08, C07D 211/36, C07D 401/04, C07D 401/06, C07D 401/10, C07D 413/10, A61K 31/506, A61P 3/10

(54) **GPCR agonists**

(30) Priority: 30.06.2005 GB 0513277; 27.03.2006 GB 0605946
(62) Divisional of application: 06744358.0
(71) Applicant: PROSIDION LTD, Oxford, Oxfordshire OX4 6LT (GB)
(72) Inventor: Bradley, Stuart Edward, Oxford, Oxfordshire OX4 6LT (GB); Fyfe, Matthew Colin Thor, Oxford, Oxfordshire OX4 6LT (GB); Bertram, Lisa Sarah, Oxford, Oxfordshire OX4 6LT (GB); Gattrell, William, Oxford, Oxfordshire OX4 6LT (GB); Jeevaratnam, Revathy Perpetua, Oxford, Oxfordshire OX4 6LT (GB); Keily, John, Oxford, Oxfordshire OX4 6LT (GB); Procter, Martin James, Oxford, Oxfordshire OX4 6LT (GB); Rasamison, Chrystelle Marie, Oxford, Oxfordshire OX4 6LT (GB); Rushworth, Philip John, Oxford, Oxfordshire OX4 6LT (GB); Sambrook-Smith, Colin Peter, Oxford, Oxfordshire OX4 6LT (GB); Stonehouse, David French, Oxford, Oxfordshire OX4 6LT (GB); Swain, Simon Andrew, Oxford, Oxfordshire OX4 6LT (GB); Williams, Geoffrey Martyn, Oxford, Oxfordshire OX4 6LT (GB)
(74) Representative: Crump, Julian Richard John

(57) **Abstract**

Compounds of formula (I): or pharmaceutically acceptable salts thereof, are GPCR agonists and are useful as for the treatment of obesity and diabetes.

## Description

### BACKGROUND OF THE INVENTION

The present invention is directed to G-protein coupled receptor (GPCR) agonists. In particular, the present invention is directed to GPCR agonists that are useful for the treatment of obesity, e.g. as regulators of satiety, and for the treatment of diabetes.

Obesity is characterized by an excessive adipose tissue mass relative to body size. Clinically, body fat mass is estimated by the body mass index (BMI; weight(kg)/height(m)²), or waist circumference. Individuals are considered obese when the BMI is greater than 30 and there are established medical consequences of being overweight. It has been an accepted medical view for some time that an increased body weight, especially as a result of abdominal body fat, is associated with an increased risk for diabetes, hypertension, heart disease, and numerous other health complications, such as arthritis, stroke, gallbladder disease, muscular and respiratory problems, back pain and even certain cancers.

Pharmacological approaches to the treatment of obesity have been mainly concerned with reducing fat mass by altering the balance between energy intake and expenditure. Many studies have clearly established the link between adiposity and the brain circuitry involved in the regulation of energy homeostasis. Direct and indirect evidence suggest that serotonergic, dopaminergic, adrenergic, cholinergic, endocannabinoid, opioid, and histaminergic pathways in addition to many neuropeptide pathways (e.g. neuropeptide Y and melanocortins) are implicated in the central control of energy intake and expenditure. Hypothalamic centres are also able to sense peripheral hormones involved in the maintenance of body weight and degree of adiposity, such as insulin and leptin, and fat tissue derived peptides.

Drugs aimed at the pathophysiology associated with insulin dependent Type I diabetes and non-insulin dependent Type II diabetes have many potential side effects and do not adequately address the dyslipidaemia and hyperglycaemia in a high proportion of patients. Treatment is often focused at individual patient needs using diet, exercise, hypoglycaemic agents and insulin, but there is a continuing need for novel antidiabetic agents, particularly ones that may be better tolerated with fewer adverse effects.

Similarly, metabolic syndrome (syndrome X) which is characterized by hypertension and its associated pathologies including atherosclerosis, lipidemia, hyperlipidemia and hypercholesterolemia have been associated with decreased insulin sensitivity which can lead to abnormal blood sugar levels when challenged. Myocardial ischemia and microvascular disease is an established morbidity associated with untreated or poorly controlled metabolic syndrome.

There is a continuing need for novel antiobesity and antidiabetic agents, particularly ones that are well tolerated with few adverse effects.

GPR119 (previously referred to as GPR116) is a GPCR identified as SNORF25 in WO00/50562 which discloses both the human and rat receptors, US 6,468,756 also discloses the mouse receptor (accession numbers: AAN95194 (human), AAN95195 (rat) and ANN95196 (mouse)).

In humans, GPR119 is expressed in the pancreas, small intestine, colon and adipose tissue. The expression profile of the human GPR119 receptor indicates its potential utility as a target for the treatment of obesity and diabetes.

International patent application WO2005/061489 (published after the priority date of the present application) discloses heterocyclic derivatives as GPR119 receptor agonists.

The present invention relates to agonists of GPR119 which are useful for the treatment of obesity e.g. as peripheral regulators of satiety, and for the treatment of diabetes.

### SUMMARY OF THE INVENTION

Compounds of formula (I): 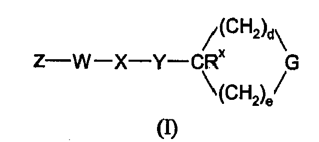 or pharmaceutically acceptable salts thereof, are agonists of GPR119 and are useful for the prophylactic or therapeutic treatment of obesity and diabetes.

### DETAILED DESCRIPTION OF THE INVENTION

The present invention is directed to a compound of formula (I), or a pharmaceutically acceptable salt thereof: 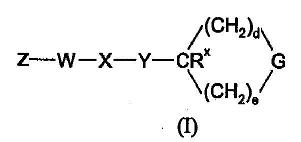 wherein Z is phenyl or a 5- or 6-membered heteroaryl group containing up to four heteroatoms selected from O N and S, any of which may be optionally substituted by one or more substituents selected from halo, C₁₋₄ alkyl, C₁₋₄ fluoroalkyl, C₁₋₄ hydroxyalkyl, C₂₋₄ alkenyl, C₂₋₄ alkynyl, C₃₋₇ cycloalkyl, aryl, OR¹, CN, NO₂ -(CH₂)ⱼ-S(O)mR¹, -(CH₂)ⱼ-C(O)NR¹R¹¹, NR¹R¹¹, NR²C(O)R¹, NR²C(O)NR¹R¹¹, NR²SO₂R¹, SO₂NR¹R¹¹, C(O)R², C(O)OR2, -P(O)(CH₃)₂, -(CH₂)ⱼ₋(4- to 7-membered heterocyclyl) or -(CH₂)ⱼ-(5- to 6-membered heteroaryl); provided that Z is not optionally substituted 3- or 4-pyridyl;
m is 0, 1 or 2;
j is 0, 1 or 2;
W and Y are independently a bond, an unbranched or a branched C₁₋₄ alkylene optionally substituted by hydroxy or C₁₋₃alkoxy, or an unbranched or a branched C₂₋₄ alkenylene;
X is selected from CH₂, O S, CH(OH), CH(halogen), CF₂, C(O), C(O)O, C(O)S, SC(O), C(O)CH₂S, C(O)CH₂C(OH), C(OH)CH₂C(O), C(O)CH₂C(O), OC(O), NR⁵, CH(NR⁵R⁵⁵), C(O)NR², NR² C(O), S(O) and S(O)₂;
R^{x} is hydrogen or hydroxy;
G is CHR³, N-C(O)OR⁴, N-C(O)NR⁴R⁵, N-C₁₋₄alkylene-C(O)OR⁴, N-C(O)C(O)OR⁴, N-S(O)₂R⁴, N-C(O)R⁴ or N-P(O)(O-Ph)₂; or N-heterocyclyl or N-heteroaryl, either of which may optionally be substituted by one or two groups selected from C₁₋₄ alkyl, C₁₋₄ alkoxy or halogen;
R¹ and R¹¹ are independently hydrogen, C₁₋₄ alkyl, which may optionally be substituted by halo, hydroxy, C₁₋₄ alkoxy-, aryloxy-, arylC₁₋₄ alkoxy-, C₁₋₄alkylS(O)ₘ-, C₃₋₇ heterocyclyl,
-C(O)OR⁷ or N(R²)₂; or may be C₃₋₇ cycloalkyl, aryl, heterocyclyl or heteroaryl, wherein the cyclic groups may be substituted with one or more substituents selected from halo, C₁₋₄ alkyl, C₁₋₄ fluoroalkyl, OR⁶, CN, SO₂CH₃, N(R²)₂ and NO₂; or taken together R¹ and R¹¹ may form a 5- or 6-membered heterocyclic ring optionally substituted by hydroxy, C₁₋₄ alkyl or C₁₋₄ hydroxyalkyl and optionally containing a further heteroatom selected from O and NR²; or R¹¹ is C₁₋₄ alkyloxy-;
R² are independently hydrogen or C₁₋₄ alkyl; or a group N(R²)₂ may form a 4- to 7-membered heterocyclic ring optionally containing a further heteroatom selected from O and NR²;
R³ is C₃₋₆ alkyl;
R⁴ is C₁₋₈ alkyl, C₂₋₈ alkenyl or C₂₋₈ alkynyl, any of which may be optionally substituted by one or more substituents selected from halo, NR⁵R⁵⁵, OR⁵, C(O)OR⁵, OC(O)R⁵ and CN, and may contain a CH₂ group that is replaced by O or S; or a C₃₋₇cycloalkyl, aryl, heterocyclyl, heteroaryl, C₁₋₄alkyleneC₃₋₇cycloalkyl, C₁₋₄alkylenearyl, C₁₋₄alkyleneheterocyclyl or C_{1- 4}alkyleneheteroaryl, any of which may be substituted with one or more substituents selected from halo, C₁₋₄ alkyl, C₁₋₄ fluoroalkyl, OR⁵, CN, NR⁵R⁵⁵, SO₂Me, NO₂ and C(O)OR⁵;
R⁵ and R⁵⁵ are independently hydrogen or C₁₋₄alkyl; or taken together R⁵ and R⁵⁵ may form a 5- or 6-membered heterocyclic ring; or a group NR⁵ may represent NS(O)₂-(2-NO₂-C₆H₄);
R⁶ is hydrogen, C₁₋₂alkyl or C₁₋₂ fluoroalkyl;
R⁷ is hydrogen or C₁₋₄ alkyl;
d is 0, 1, 2 or 3; and
e is 1, 2, 3, 4 or 5, provided that d + e is 2, 3, 4 or 5.

The molecular weight of the compounds of formula (I) is preferably less than 800, more preferably less than 600, even more preferably less than 500.

Suitably Z represents phenyl or a 5- or 6-membered heteroaryl group containing up to four heteroatoms selected from O, N and S, any of which may be optionally substituted by one or more substituents selected from halo, C₁₋₄ alkyl, C₁₋₄ fluoroalkyl, C₁₋₄ hydroxyalkyl, C₂₋₄ alkenyl, C₂₋₄ alkynyl, C₃₋₇ cycloakyl, aryl, OR¹, CN, NO₂, S(O)ₘR¹, C(O)NR¹R¹¹, NR¹R¹¹, NR²C(O)R¹, NR²SO₂R¹, SO₂NR¹R¹¹, COR², C(O)OR², a 4- to 7-membered heterocyclyl group or a 5- or 6-membered heteroaryl group; provided that Z is not optionally substituted 3- or 4-pyridyl. More suitably Z represents phenyl or a 6-membered heteroaryl group containing up to four heteroatoms selected from O, N and S, any of which may be optionally substituted.

In one embodiment of the invention Z is phenyl or a 5- or 6-membered heteroaryl group containing up to four heteroatoms selected from O, N and S, any of which may be optionally substituted by one or more substituents selected from halo, C₁₋₄ alkyl, C₁₋₄ fluoroalkyl, C₁₋₄ hydroxyalkyl, C₂₋₄ alkenyl, C₂₋₄ alkynyl, C₃₋₇ cycloalkyl, aryl, OR¹, CN, NO₂, S(O)ₘR¹, C(O)NR¹R¹¹, NR¹R¹¹, NR²C(O)R¹, NR²SO₂R¹, SO₂NR¹R¹¹, C(O)R², C(O)OR², 4- to 7-membered heterocyclyl or 5- to 6-membered heteroaryl; provided that Z is not optionally substituted 3- or 4-pyridyl.

Z is preferably phenyl or a 6-membered heteroaryl group containing up to two N heteroatoms either of which may optionally be substituted, more preferably optionally substituted phenyl and especially substituted phenyl. Examples of Z heteroaryl groups include oxazolyl, isoxazolyl, thienyl, pyrazolyl, imidazolyl, furanyl, pyridazinyl or 2-pyridyl. Preferred substituent groups for Z are halo, C₁₋₄ alkyl, C₁₋₄ fluoroalkyl, C₂₋₄ alkenyl, C₂₋₄ alkynyl, CN, S(O)m)R¹, NR²C(O)NR¹R¹¹, C(O)NR¹R¹¹, SO₂NR¹R¹¹, COR², COOR² or a 5- or 6-membered heteroaryl group; especially halo e.g. fluoro or chloro, C₁₋₄ alkyl, C₁₋₄ fluoroalkyl, C₂₋₄ alkenyl, C₂₋₄ alkynyl, CN, S(O)ₘR¹, NR²C(O)NR¹R¹¹, C(O)NR¹R¹¹, SO₂NR¹R¹¹ or a 5-membered heteroaryl group; in particular fluoro, chloro, methyl, S(O)ₘR¹ e.g. where m is 1 or 2, NR²C(O)NR¹R¹¹, C(O)NR¹R¹¹, SO₂NR¹R¹¹ or a 5-membered heteroaryl group.

In one embodiment, suitable substituent groups for Z are halo, C₁₋₄ alkyl, C₁₋₄ fluoroalkyl, C₂₋₄ alkenyl, C₂₋₄ alkynyl, CN, S(O)ₘR¹, C(O)NR¹R¹¹, SO₂NR¹R¹¹, COR², COOR² or a 5- or 6-membered heteroaryl group; especially halo (e.g. fluoro or chloro), C₁₋₄alkyl, C₁₋₄ fluoroalkyl, C₂₋₄ alkenyl, C₂₋₄ alkynyl, CN, S(O)ₘR¹, C(O)NR¹R¹¹, SO₂NR¹R¹¹; in particular fluoro, chloro, methyl, S(O)ₘR¹ (e.g. where m is 1 or 2), C(O)NR¹R¹¹ or SO₂NR¹R¹¹.

Suitably, j is 0 or 1. In one embodiment of the invention j represents 0. In a second embodiment of the invention j represents 1.

Suitably W and Y are independently a bond, an unbranched or a branched C₁₋₄ alkylene optionally substituted by hydroxy, or an unbranched or a branched C₂₋₄ alkenylene.

In one embodiment of the invention W and Y are independently a bond, an unbranched or a branched C₁₋₄ alkylene, or an unbranched or a branched C₂₋₄ alkenylene.

Preferably W and Y do not both represent a bond.

Preferably W is a bond.

Preferably Y is an Y is unbranched or a branched C₃₋₄ alkylene optionally substituted by hydroxy or C₁₋₃ alkoxy, e.g an unsubstituted unbranched or a branched C₃₋₄ alkylene.

In certain embodiments of the invention -W-X-Y- represents a chain of 2 to 6 atoms in length. -W-X-Y- preferably represents a 4 or 5 atom chain.

When W is C₂₋₃ alkenylene, the stereochemistry at the double bond is preferably (*E*).

Suitably, X is selected from CH₂, O, S, CH(OH), CH(halogen), CF₂, C(O), C(O)O, C(O)S, SC(O), C(O)CH₂S, C(O)CH₂C(OH), C(O)CH₂C(O), OC(O), NR⁵, CH(NR⁵R⁵⁵), C(O)NR², S(O) and S(O)₂. More suitably X is selected from CH₂, O, S, CH(OH), CH(halogen), C(O), C(O)O, C(O)S, SC(O), C(O)CH₂S, C(O)CH₂C(OH), C(O)CH₂C(O), OC(O), NR⁵, CH(NR⁵R⁵⁵), C(O)NR², S(O) and S(O)₂.

X is preferably CH₂, CF₂, O or NR⁵ e.g. NH, in particular CH₂, O or NR⁵, especially O. R^{x} is preferably hydrogen.

G is preferably N-C(O)R⁴, N-C(O)NR⁴R⁵, N-C₁₋₄alkylene-C(O)OR⁴, N-C(O)C(O)OR⁴, N-heterocyclyl, N-heteroaryl, N-S(O)₂R⁴_{,} N-C(O)R⁴ or N-P(O)(O-Ph)₂; especially N-C(O)R⁴, N-C(O)NR⁴R⁵, N-C₁₋₄alkylene-C(O)OR⁴, N-heteroaryl, N-S(O)₂R⁴ or N-C(O)R⁴; in particular N-C(O)OR⁴, N-C(O)NR⁴R⁵, N-heteroaryl, N-S(O)₂R⁴ or N-C(O)R⁴. More preferably, G is N-C(O)R⁴, N-C(O)NR⁴R⁵ or N-heteroaryl. G is most preferably N-C(O)OR⁴. When G is N-heteroaryl the heteroaryl ring is preferably a 5- or 6-membered heteroaryl ring containing up to three heteroatoms selected from O, N and S, for example pyridin-2-yl, oxadiazolyl, or pyrimidinyl, especially pyrimidin-2-yl. Alternatively, G is CHR³.

Suitably, R¹ and R¹¹ are independently hydrogen, C₁₋₄ alkyl, which may optionally be substituted by halo e.g. fluoro, hydroxy, C₁₋₄ alkyloxy-, aryloxy-, arylC₁₋₄ alkyloxy-, C₁₋₄ alkylS(O)ₘ-, C₃₋₇ heterocyclyl or N(R²)₂; or may be C₃₋₇ cycloalkyl, aryl, heterocyclyl or heteroaryl, wherein the cyclic groups may be substituted with one or more substituents selected from halo, C₁₋₄ alkyl, C₁₋₄ fluoroalkyl, OR⁶, CN, SO₂CH₃, N(R²)₂ and NO₂; or taken together R¹ and R¹¹ may form a 5- or 6-membered heterocyclic ring optionally containing a further heteroatom selected from O and NR².

In one embodiment of the invention R¹ and R¹¹ are independently hydrogen, C₁₋₄ alkyl, which may optionally be substituted by halo (e.g. fluoro), hydroxy, C₁₋₄ alkyloxy-, C₁₋₄ alkylthio-C₃₋₇ heterocyclyl or N(R²)₂; or may be C₃₋₇ cycloalkyl, aryl, heterocyclyl or heteroaryl, wherein the cyclic groups may be substituted with one or more substituents selected from halo, C₁₋₄ alkyl, C₁₋₄ fluoroalkyl, OR⁶, CN, SO₂CH₃, N(R²)₂ and NO₂.

Suitably R² is hydrogen, methyl or tert-butyl.

Exemplary R³ groups include *n*-pentyl.

Exemplary R⁴ groups include methyl, ethyl, propyl, iso-propyl, sec-butyl, tert-butyl, butynyl, cyclobutyl, pentyl, 2,2-dimethylpropyl, cyclopentyl, hexyl, cyclohexyl, trifluoroethyl, trichloroethyl, phenyl, methoxyphenyl, tolyl, fluorophenyl, chlorophenyl, trifluoromethylphenyl, nitrophenyl, naphthalenyl, chlorobenzyl, methylsulfanylethyl- and tetrahydrofuranmethyl-.

Preferably R⁴ represents C₁₋₈ alkyl, C₂₋₈ alkenyl or C₂₋₈ alkynyl optionally substituted by one or more halo atoms or cyano, and may contain a CH₂ group that is replaced by O or S; or a C₃₋₇ cycloalkyl, aryl or C₁₋₄ alkylC₃₋₇ cycloalkyl, any of which may be substituted with one or more substituents selected from halo, C₁₋₄ alkyl, C₁₋₄ fluoroalkyl, OR⁵, CN, NR⁵R⁵⁵, NO₂ and C(O)OC₁₋₄alkyl. More preferably R⁴ represents C₁₋₈ alkyl, C₂₋₈ alkenyl or C₂₋₈ alkynyl optionally substituted by one or more halo atoms or CN, and may contain a CH₂ group that is replaced by O or S; or a C₃₋₇ cycloalkyl or aryl, either of which may be substituted with one or more substituents selected from halo, C₁₋₄ alkyl, C₁₋₄ fluoroalkyl, OR⁵, CN, NR⁵R⁵⁵, NO₂ and C(O)OC₁₋₄alkyl. Most preferred R⁴ groups are C₂₋₅ alkyl, e.g. C₃₋₅alkyl, optionally substituted by one or more halo or CN groups, and which may contain a CH₂ group that is replaced by O or S, or C₃₋₅ cycloalkyl optionally substituted by C₁₋₄ alkyl. In one embodiment of the invention the group represented by R⁴ is unsubstituted.

In one embodiment of the invention d + e is 2, 3, or 4. Suitably, d is 1 or 2 and e is 1 or 2. In a preferred embodiment of the invention d and e each represent 1. In a more preferred embodiment of the invention d and e each represent 2.

Suitably R⁵ and R⁵⁵ are independently hydrogen or C₁₋₄alkyl; or taken together R⁵ and R⁵⁵ may form a 5- or 6-membered heterocyclic ring; in particular R⁵ represents hydrogen or methyl, especially methyl.

Suitably R⁶ is C₁₋₄ alkyl or C₁₋₄ fluoroalkyl.

A group of compounds according to the invention which may be mentioned are the compounds of formula (Ia), and pharmaceutically acceptable salts thereof: 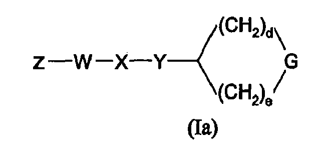 wherein Z is phenyl or a 5- or 6-membered heteroaryl group containing up to four heteroatoms selected from O N and S, any of which may be optionally substituted by one or more substituents selected from halo, C₁₋₄ alkyl, C₁₋₄ fluoroalkyl, C₁₋₄ hydroxyalkyl, C₂₋₄ alkenyl, C₂₋₄ alkynyl, C₃₋₇ cycloalkyl, aryl, OR¹, CN, NO₂, S(O)ₘR¹, NR²C(O)NR¹R¹¹, C(O)NR¹R¹¹, NR¹R¹¹, NR²C(O)R¹, NR²SO₂R¹, SO₂NR¹R¹¹, COR², C(O)OR², a 4- to 7-membered heterocyclyl group or a 5- or 6-membered heteroaryl group; provided that Z is not optionally substituted 3- or 4-pyridyl;
m is 0, 1 or 2;
W and Y are independently a bond, an unbranched or a branched C₁₋₃ alkylene or an unbranched or a branched C₂₋₃ alkenylene;
X is selected from CH₂, O, S, CH(OH), CH(halogen), C(O), C(O)O, C(O)S, SC(O), C(O)CH₂S, C(O)CH₂C(OH), C(O)CH₂C(O), OC(O), NR⁵, CH(NR⁵R⁵⁵), C(O)NR², S(O) and S(O)₂;
G is CHR³, N-C(O)OR⁴, N-C(O)NR⁴R⁵, N-C₁₋₄alkylene-C(O)OR⁴, N-C(O)C(O)OR⁴, N-S(O)₂R⁴, N-C(O)R⁴ or N-P(O)(O-Ph)₂; or N-heterocyclyl or N-heteroaryl, either of which may optionally be substituted by one or two groups selected from C₁₋₄alkyl, C₁₋₄alkoxy or halogen;
R¹ and R¹¹ are independently hydrogen, C₁₋₄ alkyl, which may optionally be substituted by halo e.g. fluoro, hydroxy, C₁₋₄ alkoxy-, C₁₋₄ alkylthio-, C₃₋₇ heterocyclyl or N(R²)₂; or may be C₃₋₇ cycloalkyl, aryl, heterocyclyl or heteroaryl, wherein the cyclic groups may be substituted with one or more substituents selected from halo, C₁₋₄ alkyl, C₁₋₄ fluoroalkyl, OR⁶, CN, SO₂CH₃, N(R²)₂ and NO₂;
R² are independently hydrogen or C₁₋₄ alkyl; or a group N(R²)₂ may form a 4- to 7-membered heterocyclic ring optionally containing a further heteroatom selected from O and NR²;
R³ is C₃₋₆ alkyl;
R⁴ is C₁₋₈ alkyl, C₂₋₈ alkenyl or C₂₋₈ alkynyl, any of which may be optionally substituted by one or more halo atoms, NR⁵R⁵⁵, OR⁵, C(O)OR⁵, OC(O)R⁵ or cyano, and may contain a CH₂ group that is replaced by O or S; or a C₃-₇cycloalkyl, aryl, heterocyclyl, heteroaryl, C_{1- 4}alkyleneC₃₋₇cycloalkyl, C₁₋₄alkylenearyl, C₁₋₄alkyleneheterocyclyl or C₁₋₄alkyleneheteroaryl, any of which may be substituted with one or more substituents selected from halo, C₁₋₄ alkyl, C_{1- 4} fluoroalkyl, OR⁵, CN, NR⁵R⁵⁵, SO₂Me, NO₂ or C(O)OR⁵;
R⁵ and R⁵⁵ are independently hydrogen or C₁₋₄alkyl; or taken together R⁵ and R⁵⁵ may form a 5 or 6 membered heterocyclic ring;
R⁶ is hydrogen, C₁₋₂ alkyl or C₁₋₂ fluoroalkyl;
d is 0,1,2 or 3;
e is 1, 2, 3, 4 or 5; and
with the proviso that d + e is 2, 3, 4 or 5.

One group of compounds of interest are those of formula (Ib): 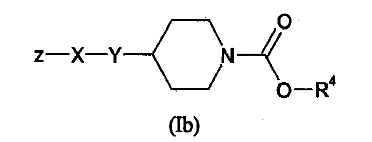 wherein:
Y represents an unbranched or a branched C₃₋₄ alkylene group;
Z, X, and R⁴ are as described previously for compounds of formula (I).

A group of compounds of formula (Ib) of particualar interest are those of formula (Ic): 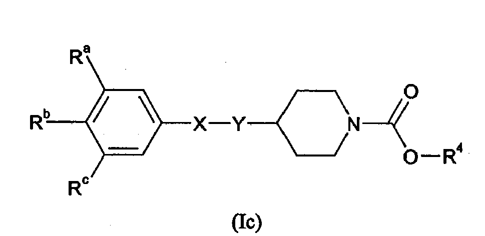 wherein:
R^{a} and R^{c} independently represent hydrogen, fluorine, chlorine, methyl or CN;
R^{b} represents S(O)ₘR¹, C(O)NR¹R¹¹, SO₂NR¹R¹¹, NR²C(O)R¹, NR²SO₂R¹, NR²C(O)NR¹R¹¹ or 5-membered heteroaryl;
X represents CH₂, CF₂, O NH or C(O);
Y represents an unbranched or a branched C₃₋₄ alkylene group;
R⁴ represents C₂₋₅ alkyl or C₃₋₆ cycloalkyl which may optionally be substituted by methyl;
m represents 1 or 2;
R¹ and R¹¹ independently represent hydrogen or C₁₋₄ alkyl which may optionally be substituted by hydroxyl or NH₂, alternatively R¹ and R¹¹ taken together may form a heterocyclic ring, e.g. a 5- or 6-membered heterocyclic ring, optionally substituted with OH or CH₂OH; and
R² are independently hydrogen or C₁₋₄ alkyl; or a group N(R²)₂ may form a 4- to 7-membered heterocyclic ring optionally containing a further heteroatom selected from O and NR².

For compounds of formula (Ic), suitably R^{b} represents S(O)mR⁶, C(O)NR⁶R⁶⁶ or SO₂NR⁶R⁶⁶, NR¹⁰C(O)NR⁶R⁶⁶ or 5-membered heteroaryl. Alternatively for compounds of formula (Ic), suitably R^{b} represents NR¹⁰C(O)R⁶ or NR¹⁰SO₂R⁶.

While the preferred groups for each variable have generally been listed above separately for each variable, preferred compounds of this invention include those in which several or each variable in formula (I) to (Ic) is selected from the preferred, more preferred or particularly listed groups for each variable. Therefore, this invention is intended to include all combinations of preferred, more preferred and particularly listed groups.

Specific compounds of the invention which may be mentioned are those included in the Examples and pharmaceutically acceptable salts thereof.

The following provisos may optionally be used (individually or in any combination) to exclude certain compounds from the scope of the invention:
i) when G is N-(CH₂)₃-C(O)OR⁴, d represents 2 and e represents 2, suitably R⁴ does not represent ethyl or trichloroethyl.
ii) when d represents 2 and e represents 2, suitably Z is not a 5-membered heteroaryl group substituted by -(CH₂)ⱼ-pyrid-4-yl.
iii) suitably Z is not a 5-membered heteroaryl group substituted by -(CH₂)ⱼ-pyrid-4-yl.
iv) when d represents 2 and e represents 2 and -W-X-Y- represents -O-, suitably Z does not represent pyrimidine or optionally substituted pyrimidine.
v) when d represents 2 and e represents 2, G represents N-C(O)O-t-butyl and Z represents bromophenyl, suitably -W-X-Y- does not represent -C(O)- or -C(NH₂)-.
vi) when d represents 2 and e represents 2, G represents N-C(O)O-t-butyl or N-C(O)-CH₂-(*N-*decahydroquinoline), suitably -W-X-Y- does not represent -CH=CH-, -CH₂₋CH=CH- or -C(O)-CH=CH-.
vii) when -W-X-Y- represents -C(O)-, d represents 2 and e represents 2, suitably Z does not represent phenyl which is monosubstituted in the 4-position by F, Cl or methyl.
viii) when -W-X-Y- represents -CH₂-C(O)-, d represents 2 and e represents 2, suitably Z does not represent phenyl which is monosubstituted in the 4-position by F, Cl or methyl.

As used herein, unless stated otherwise, "alkyl" as well as other groups having the prefix "alk" such as, for example, alkenyl, alkynyl, and the like, means carbon chains which may be linear or branched or combinations thereof. Examples of alkyl groups include methyl, ethyl, propyl, isopropyl, butyl, sec- and tert-butyl, pentyl, hexyl, heptyl and the like. "Alkenyl", "alkynyl" and other like terms include carbon chains having at least one unsaturated carbon-carbon bond.

The term "fluoroalkyl," includes alkyl groups substituted by one or more fluorine atoms, e.g. CH₂F, CHF₂ and CF₃.

The term "cycloalkyl" means carbocycles containing no heteroatoms, and includes monocyclic and bicyclic saturated and partially saturated carbocycles. Examples of cycloalkyl groups include cyclopropyl, cyclobutyl, cyclopentyl, cyclohexyl and cycloheptyl. Examples of partially saturated cycloalkyl groups include cyclohexene and indane. Cycloalkyl. groups will typically contain 3 to 10 ring carbon atoms in total (e.g. 3 to 6, or 8 to 10).

The term "halo" includes fluorine, chlorine, bromine, and iodine atoms (in particular fluorine or chlorine).

The term "aryl" includes phenyl and naphthyl, in particular phenyl.

Unless otherwise indicated the term "heterocyclyl" and "heterocyclic ring" includes 4-to 10-membered monocyclic and bicyclic saturated rings, e.g. 4- to 7-membered monocyclic saturated rings, containing up to three heteroatoms selected from N, O and S. Examples of heterocyclic rings include oxetane, tetrahydrofuran, tetrahydropyran, oxepane, oxocane, thietane, tetrahydrothiophene, tetrahydrothiopyran, thiepane, thiocane, azetidine, pyrrolidine, piperidine, azepane, azocane, [1,3]dioxane, oxazolidine, piperazine, and the like. Other examples of heterocyclic rings include the oxidised forms of the sulfur-containing rings. Thus, tetrahydrothiophene 1-oxide, tetrahydrothiophene 1,1-dioxide, tetrahydrothiopyran 1-oxide, and tetrahydrothiopyran 1,1-dioxide are also considered to be heterocyclic rings.

Unless otherwise stated, the term "heteroaryl" includes mono- and bicyclic 5- to 10-membered, e.g. monocyclic 5- or 6-membered, heteroaryl rings containing up to 4 heteroatoms selected from N, O and S. Examples of such heteroaryl rings are furyl, thienyl, pyrrolyl, pyrazolyl, imidazolyl, oxazolyl, isoxazolyl, thiazolyl, isothiazolyl, triazolyl, oxadiazolyl, thiadiazolyl, tetrazolyl, pyridinyl, pyridazinyl, pyrimidinyl, pyrazinyl and triazinyl. Bicyclic heteroaryl groups include bicyclic heteroaromatic groups where a 5- or 6-membered heteroaryl ring is fused to a phenyl or another heteroaromatic group. Examples of such bicyclic heteroaromatic rings are benzofuran, benzothiophene, indole, benzoxazole, benzothiazole, indazole, benzimidazole, benzotriazole, quinoline, isoquinoline, quinazoline, quinoxaline and purine. Preferred heteroaryl groups are monocyclic 5- or 6-membered, heteroaryl rings containing up to 4 heteroatoms selected from N, O and S.

Compounds described herein may contain one or more asymmetric centers and may thus give rise to diastereomers and optical isomers. The present invention includes all such possible diastereomers as well as their racemic mixtures, their substantially pure resolved enantiomers, all possible geometric isomers, and pharmaceutically acceptable salts thereof. The above formula (I) is shown without a definitive stereochemistry at certain positions. The present invention includes all stereoisomers of formula (I) and pharmaceutically acceptable salts thereof. Further, mixtures of stereoisomers as well as isolated specific stereoisomers are also included. During the course of the synthetic procedures used to prepare such compounds, or in using racemization or epimerization procedures known to those skilled in the art, the products of such procedures can be a mixture of stereoisomers.

When a tautomer of the compound of formula (I) exists, the present invention includes any possible tautomers and pharmaceutically acceptable salts thereof, and mixtures thereof, except where specifically drawn or stated otherwise.

When the compound of formula (I) and pharmaceutically acceptable salts thereof exist in the form of solvates or polymorphic forms, the present invention includes any possible solvates and polymorphic forms. A type of a solvent that forms the solvate is not particularly limited so long as the solvent is pharmacologically acceptable. For example, water, ethanol, propanol, acetone or the like can be used.

The term "pharmaceutically acceptable salts" refers to salts prepared from pharmaceutically acceptable non-toxic bases or acids. When the compound of the present invention is acidic, its corresponding salt can be conveniently prepared from pharmaceutically acceptable non-toxic bases, including inorganic bases and organic bases. Salts derived from such inorganic bases include aluminum, ammonium, calcium, copper (ic and ous), ferric, ferrous, lithium, magnesium, potassium, sodium, zinc and the like salts. Particularly preferred are the ammonium, calcium, magnesium, potassium and sodium salts. Salts derived from pharmaceutically acceptable organic non-toxic bases include salts of primary, secondary, and tertiary amines, as well as cyclic amines and substituted amines such as naturally occurring and synthesized substituted amines. Other pharmaceutically acceptable organic non-toxic bases from which salts can be formed include arginine, betaine, caffeine, choline, *N',N'-*dibenzylethylenediamine, diethylamine, 2-diethylaminoethanol, 2-dimethylaminoethanol, ethanolamine, ethylenediamine, *N*-ethylmorpholine, *N*-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, its corresponding salt can be conveniently prepared from pharmaceutically acceptable non-toxic acids, including inorganic and organic acids. Such acids include, for example, acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid and the like

Since the compounds of formula (I) are intended for pharmaceutical use they are preferably provided in substantially pure form, for example at least 60% pure, more suitably at least 75% pure, especially at least 98% pure (% are on a weight for weight basis).

The compounds of formula (I) can be prepared as described below, in which Z, d, e, W, X, Y and G are as defined above. The Schemes are illustrated using compounds wherein R^{x} is hydrogen, compounds wherein R^{x} is hydroxy may be prepared using analogous methods.

Compounds of formula (I) in which X is CO₂, COS, or CONR² can be prepared by condensing the appropriate acid (II) with an alcohol, thiol, or amine (III), as shown in Scheme 1 where E is O, S, or NR², using a typical reagent for such a condensation reaction, e.g., EDCI (Pottorf, R. S.; Szeto, P. In Handbook of Reagents for Organic Synthesis: Activating Agents and Protecting Groups; Pearson, A. J., Roush, W. R., Eds.; Wiley: Chichester,1999; pp 186-188). The acids (II) and alcohols, thiols, and amines (III) are either commercially available or are prepared easily using known techniques.

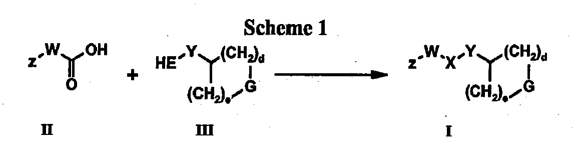

Compounds of formula (I) in which X is SCO or OCO can be prepared by condensing the appropriate thiol or alcohol (IV) with the appropriate acid (V), as shown in Scheme 2 where E is S or O, employing a reagent typically used for effecting such reactions, e.g., EDCI (Pottorf, R S.; Szeto, P. In Handbook of Reagents for Organic Synthesis: Activating Agents and Protecting Groups; Pearson, A. J., Roush, W. R., Eds.; Wiley: Chichester, 1999; pp 186-188). The alcohols and thiols (IV), as well as acids (V), are either commercially available or are prepared straightforwardly using known techniques.

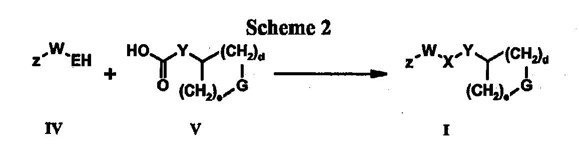

Compounds of formula (I) in which X is S or O can be prepared by alkylating the appropriate thiol or alcohol (IV) with the appropriate alkyl halide or sulfonate ester (VI), as shown in Scheme 3 where E is S or O and LG is chloro, bromo, iodo, alkanesulfonate, or arenesulfonate. The reaction is typically carried out using a base, e.g., potassium *tert*-butoxide (Hall, S. E., et al. J. Med. Chem. 1989, 32, 974-984). The alcohols and thiols (IV), as well as the alkyl halides or sulfonates (VI), are either commercially available or are made easily using known techniques. The compounds of formula (I) where X is SO or SO₂ can easily be obtained from the compounds of formula (I) where X is S by oxidation with, for example, *m*CPBA (Fyfe, M. C. T. et al. International Patent Publication WO 04/72031).

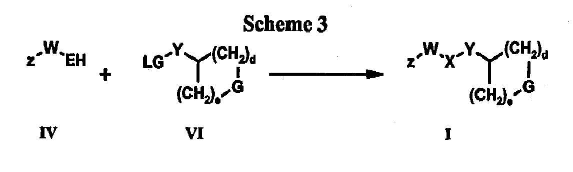

Compounds of formula (I) in which W is C₂₋₃ alkenylene can be prepared by a Wittig reaction between the appropriate phosphonium salt (VII) and the appropriate aldehyde (VIII), as indicated in Scheme 4 where m is 1 or 2 and n is 0 or 1 with the proviso that m + n < 3. As an alternative, to the approach described in Scheme 4, the compounds of formula (I) in which W is C₂₋₃ alkenylene can be prepared by a Wittig reaction between the appropriate aldehyde (IX) and the appropriate phosphonium salt (X), as indicated in Scheme 5 where q is 0 or 1 and r is 1 or 2 with the proviso that q + r < 3. The reactions are carried out in the presence of a suitable base, e.g., NaOMe or LiHMDS (March, J. Advanced Organic Chemistry, 4th edn.; Wiley: New York, 1992; pp 956-963). The phosphonium salts (VII) and (X), as well as the aldehydes (VIII) and (IX), are either commercially available or are made easily using known techniques. The compounds of formula (I) where W is C₂₋₃ alkylene can easily be synthesized from the compounds of formula (I) where W is C₂₋₃ alkenylene by a hydrogenation reaction using, for example, palladium on charcoal as a catalyst.

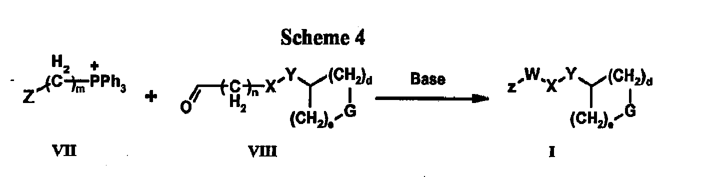

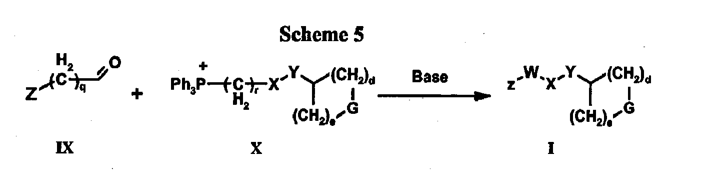

Compounds of the formula (I) where W is a bond, X is S or O, and the group Z is unsubstituted or substituted by CN can be prepared by condensation of the appropriate heteroaryl halide (XI), where with the appropriate alcohol or thiol (III), as depicted in Scheme 6 where Hal represents a halogen and E is S or O. The reaction is carried out in the presence of a suitable basic system, e.g., potassium hydroxide and potassium carbonate in the presence of tris(3,6-dioxaheptyl)amine (Ballesteros, P.; Claramunt, R. M.; Elguero, J. Tetrahedron 1987, 43, 2557-2564). The heteroaryl halides (XI) and alcohols/thiols (III) are either commercially available or are made easily using known techniques.

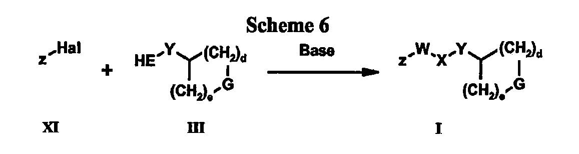

Compounds of the formula (I) where G is NC(O)OR⁴, NC(O)NR⁴R⁵, NC(O)R⁴, or N-C(O)C(O)OR⁴ can be prepared by the route shown in Scheme 7, where an amine of formula (XII) is condensed with an acyl chloride of formula (XIII) where A is O, NR⁵, a bond, or C(O)O. The reaction is carried out in the presence of a suitable base, such as triethylamine (Picard, F., et al. J. Med. Chem. 2002, 45, 3406-3417). Compounds of the formula (I) where G is NCONR⁴R⁵ and R⁵ is hydrogen may also be prepared by reacting the amine (XII) with a suitable isocyanate O=C=N-R⁴ (Boswell, R. F., Jr., et al. J. Med Chem. 1974, 17, 1000-1008). Compounds of the formula (I) where G is N-C₁₋₄alkylene-C(O)OR⁴ may be prepared by akylating the amine (XII) with the appropriate α-haloester (Rooney, C. S. et al. J. Med. Chem. 1983, 26, 700-714). The amine (XII) is generally derived from its *N-tert*-butoxycarbonyl precursor (prepared by one of the routes outlined in Schemes 1-6) by deprotection with an acid, e.g., trifluoroacetic acid (Fyfe, M. C. T. et al. International Patent Publication WO 04/72031).

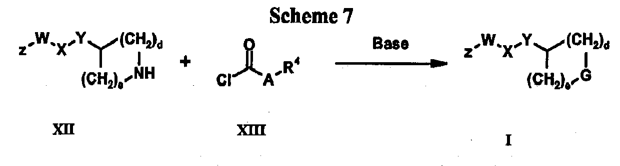

Compounds of the formula (I) where G is N-heteroaryl may be prepared by condensation of amine (XII) with a heteroaryl chloride of formula (XIV), as illustrated in Scheme 8 (Barillari, C. et al. Eur. J. Org. Chem. 2001, 4737-4741; Birch, A. M. et al. J. Med. Chem. 1999, 42, 3342-3355).

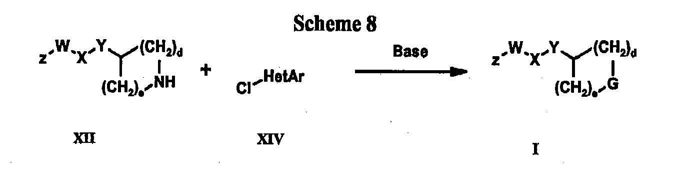

Compounds of the formula (I) where where the group Z is substituted by CN can be prepared from the corresponding unsubstituted Z group by the Reissert reaction (Fife, W. K. J. Org. Chem. 1983, 48, 1375-1377). Similar reactions can be used to prepare the compounds where Z is substituted by halogen (Walters, M. A.; Shay, J. J. Tetrahedron Lett. 1995, 36, 7575-7578). The compounds where Z is substituted by halogen can be transformed into the corresponding compounds where Z is substituted by C₁₋₄ alkyl by transition metal-catalysed cross-coupling reactions (Fürstner, A., et al. J. Am. Chem. Soc. 2002, 124,13856-13863).

Other compounds of formula (I) may be prepared by methods analogous to those described above or by methods known *per se.*

Further details for the preparation of the compounds of formula (I) are found in the examples.

The compounds of formula (I) may be prepared singly or as compound libraries comprising at least 2, for example 5 to 1,000, compounds and more preferably 10 to 100 compounds of formula (I). Compound libraries may be prepared by a combinatorial "split and mix" approach or by multiple parallel synthesis using either solution or solid phase chemistry, using procedures known to those skilled in the art.

During the synthesis of the compounds of formula (I), labile functional groups in the intermediate compounds, e.g. hydroxy, carboxy and amino groups, may be protected. The protecting groups may be removed at any stage in the synthesis of the compounds of formula (I) or may be present on the final compound of formula (I). A comprehensive discussion of the ways in which various labile functional groups may be protected and methods for cleaving the resulting protected derivatives is given in, for example, Protective Groups in Organic Chemistry, T.W. Greene and P.G.M. Wuts, (1991) Wiley-Interscience, New York, 2nd edition.

Any novel intermediates, such as those defined above, may be of use in the synthesis of compounds of formula (I) and are therefore also included within the scope of the invention, for example compounds of formula (XII): 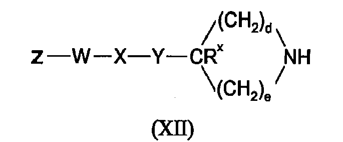
or a salt or protected derivative thereof, wherein the groups Z, W, X, Y, R^{x}, d and e are as defined above for compounds of formula (I).

As indicated above the compounds of formula (I) are useful as GPR119 agonists, e.g. for the treatment and/or prophylaxis of obesity and diabetes. For such use the compounds of formula (I) will generally be administered in the form of a pharmaceutical composition.

The invention also provides a compound of formula (I), or a pharmaceutically acceptable salt thereof, for use as a pharmaceutical.

The invention also provides a pharmaceutical composition comprising a compound of formula (I), in combination with a pharmaceutically acceptable carrier.

Preferably the composition is comprised of a pharmaceutically acceptable carrier and a non-toxic therapeutically effective amount of a compound of formula (I), or a pharmaceutically acceptable salt thereof.

Moreover, the invention also provides a pharmaceutical composition for the treatment of disease by modulating GPR119, resulting in the prophylactic or therapeutic treatment of obesity, e.g. by regulating satiety, or for the treatment of diabetes, comprising a pharmaceutically acceptable carrier and a non-toxic therapeutically effective amount of compound of formula (I), or a pharmaceutically acceptable salt thereof.

The pharmaceutical compositions may optionally comprise other therapeutic ingredients or adjuvants. The compositions include compositions suitable for oral, rectal, topical, and parenteral (including subcutaneous, intramuscular, and intravenous) administration, although the most suitable route in any given case will depend on the particular host, and nature and severity of the conditions for which the active ingredient is being administered. The pharmaceutical compositions may be conveniently presented in unit dosage form and prepared by any of the methods well known in the art of pharmacy.

In practice, the compounds of formula (I), or pharmaceutically acceptable salts thereof, can be combined as the active ingredient in intimate admixture with a pharmaceutical carrier according to conventional pharmaceutical compounding techniques. The carrier may take a wide variety of forms depending on the form of preparation desired for administration, e.g. oral or parenteral (including intravenous).

Thus, the pharmaceutical compositions can be presented as discrete units suitable for oral administration such as capsules, cachets or tablets each containing a predetermined amount of the active ingredient. Further, the compositions can be presented as a powder, as granules, as a solution, as a suspension in an aqueous liquid, as a non-aqueous liquid, as an oil-in-water emulsion, or as a water-in-oil liquid emulsion. In addition to the common dosage forms set out above, the compound of formula (I), or a pharmaceutically acceptable salt thereof, may also be administered by controlled release means and/or delivery devices. The compositions may be prepared by any of the methods of pharmacy. In general, such methods include a step of bringing into association the active ingredient with the carrier that constitutes one or more necessary ingredients. In general, the compositions are prepared by uniformly and intimately admixing the active ingredient with liquid carriers or finely divided solid carriers or both. The product can then be conveniently shaped into the desired presentation.

The compounds of formula (I), or pharmaceutically acceptable salts thereof, can also be included in pharmaceutical compositions in combination with one or more other therapeutically active compounds.

The pharmaceutical carrier employed can be, for example, a solid, liquid, or gas. Examples of solid carriers include lactose, terra alba, sucrose, talc, gelatin, agar, pectin, acacia, magnesium stearate, and stearic acid. Examples of liquid carriers are sugar syrup, peanut oil, olive oil, and water. Examples of gaseous carriers include carbon dioxide and nitrogen.

In preparing the compositions for oral dosage form, any convenient pharmaceutical media may be employed. For example, water, glycols, oils, alcohols, flavoring agents, preservatives, coloring agents, and the like may be used to form oral liquid preparations such as suspensions, elixirs and solutions; while carriers such as starches, sugars, microcrystalline cellulose, diluents, granulating agents, lubricants, binders, disintegrating agents, and the like may be used to form oral solid preparations such as powders, capsules and tablets. Because of their ease of administration, tablets and capsules are the preferred oral dosage units whereby solid pharmaceutical carriers are employed. Optionally, tablets may be coated by standard aqueous or nonaqueous techniques.

A tablet containing the composition of this invention may be prepared by compression or molding, optionally with one or more accessory ingredients or adjuvants. Compressed tablets may be prepared by compressing, in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent. Each tablet preferably contains from about 0.05mg to about 5g of the active ingredient and each cachet or capsule preferably containing from about 0.05mg to about 5g of the active ingredient.

For example, a formulation intended for the oral administration to humans may contain from about 0.5mg to about 5g of active agent, compounded with an appropriate and convenient amount of carrier material which may vary from about 5 to about 95 percent of the total composition. Unit dosage forms will generally contain between from about 1mg to about 2g of the active ingredient, typically 25mg, 50mg, 100mg, 200mg, 300mg, 400mg, 500mg, 600mg, 800mg, or 1000mg.

Pharmaceutical compositions of the present invention suitable for parenteral administration may be prepared as solutions or suspensions of the active compounds in water. A suitable surfactant can be included such as, for example, hydroxypropylcellulose. Dispersions can also be prepared in glycerol, liquid polyethylene glycols, and mixtures thereof in oils. Further, a preservative can be included to prevent the detrimental growth of microorganisms.

Pharmaceutical compositions of the present invention suitable for injectable use include sterile aqueous solutions or dispersions. Furthermore, the compositions can be in the form of sterile powders for the extemporaneous preparation of such sterile injectable solutions or dispersions. In all cases, the final injectable form must be sterile and must be effectively fluid for easy syringability. The pharmaceutical compositions must be stable under the conditions of manufacture and storage; thus, preferably should be preserved against the contaminating action of microorganisms such as bacteria and fungi. The carrier can be a solvent or dispersion medium containing, for example, water, ethanol, polyol (e.g. glycerol, propylene glycol and liquid polyethylene glycol), vegetable oils, and suitable mixtures thereof.

Pharmaceutical compositions of the present invention can be in a form suitable for topical use such as, for example, an aerosol, cream, ointment, lotion, dusting powder, or the like. Further, the compositions can be in a form suitable for use in transdermal devices. These formulations may be prepared, using a compound of formula (I), or a pharmaceutically acceptable salt thereof, via conventional processing methods. As an example, a cream or ointment is prepared by admixing hydrophilic material and water, together with about 5wt% to about 10wt% of the compound, to produce a cream or ointment having a desired consistency.

Pharmaceutical compositions of this invention can be in a form suitable for rectal administration wherein the carrier is a solid. It is preferable that the mixture forms unit dose suppositories. Suitable carriers include cocoa butter and other materials commonly used in the art. The suppositories may be conveniently formed by first admixing the composition with the softened or melted carrier(s) followed by chilling and shaping in molds.

In addition to the aforementioned carrier ingredients, the pharmaceutical formulations described above may include, as appropriate, one or more additional carrier ingredients such as diluents, buffers, flavoring agents, binders, surface-active agents, thickeners, lubricants, preservatives (including anti-oxidants) and the like. Furthermore, other adjuvants can be included to render the formulation isotonic with the blood of the intended recipient. Compositions containing a compound of formula (I), or pharmaceutically acceptable salts thereof, may also be prepared in powder or liquid concentrate form.

Generally, dosage levels on the order of 0.01 mg/kg to about 150mg/kg of body weight per day are useful in the treatment of the above-indicated conditions, or alternatively about 0.5mg to about 7g per patient per day. For example, obesity may be effectively treated by the administration of from about 0.01 to 50mg of the compound per kilogram of body weight per day, or alternatively about 0.5mg to about 3.5g per patient per day.

It is understood, however, that the specific dose level for any particular patient will depend upon a variety of factors including the age, body weight, general health, sex, diet, time of administration, route of administration, rate of excretion, drug combination and the severity of the particular disease undergoing therapy.

The compounds of formula (I) may be used in the treatment of diseases or conditions in which GPR119 plays a role.

Thus the invention also provides a method for the treatment of a disease or condition in which GPR119 plays a role comprising a step of administering to a subject in need thereof an effective amount of a compound of formula (I), or a pharmaceutically acceptable salt thereof. Diseases or conditions in which GPR119 plays a role include obesity and diabetes. In the context of the present application the treatment of obesity is intended to encompass the treatment of diseases or conditions such as obesity and other eating disorders associated with excessive food intake e.g. by reduction of appetite and body weight, maintenance of weight reduction and prevention of rebound and diabetes (including Type 1 and Type 2 diabetes, impaired glucose tolerance, insulin resistance and diabetic complications such as neuropathy, nephropathy, retinopathy, cataracts, cardiovascular complications and dyslipidaemia). And the treatment of patients who have an abnormal sensitivity to ingested fats leading to functional dyspepsia. The compounds of the invention may also be used for treating metabolic diseases such as metabolic syndrome (syndrome X), impaired glucose tolerance, hyperlipidemia, hypertriglyceridemia, hypercholesterolemia, low HDL levels and hypertension.

The compounds of the invention may offer advantages over compounds acting via different mechanisms for the treatment of the above mentioned disorders in that they may offer beta-cell protection, increased cAMP and insulin secretion and also slow gastric emptying.

The invention also provides a method for the regulation of satiety comprising a step of administering to a subject in need thereof an effective amount of a compound of formula (I), or a pharmaceutically acceptable salt thereof.

The invention also provides a method for the treatment of obesity comprising a step of administering to a subject in need thereof an effective amount of a compound of formula (I), or a pharmaceutically acceptable salt thereof.

The invention also provides a method for the treatment of diabetes, including Type 1 and Type 2 diabetes, particularly type 2 diabetes, comprising a step of administering to a patient in need thereof an effective amount of a compound of formula (1), or a pharmaceutically acceptable salt thereof.

The invention also provides a method for the treatment of metabolic syndrome (syndrome X), impaired glucose tolerance, hyperlipidemia, hypertriglyceridemia, hypercholesterolemia, low HDL levels or hypertension comprising a step of administering to a patient in need thereof an effective amount of a compound of formula (I), or a pharmaceutically acceptable salt thereof.

The invention also provides a compound of formula (I), or a pharmaceutically acceptable salt thereof, for use in the treatment of a condition as defined above.

The invention also provides the use of a compound of formula (I), or a pharmaceutically acceptable salt thereof, in the manufacture of a medicament for the treatment of a condition as defined above.

In the methods of the invention the term "treatment" includes both therapeutic and prophylactic treatment.

The compounds of formula (I), or pharmaceutically acceptable salts thereof, may be administered alone or in combination with one or more other therapeutically active compounds. The other therapeutically active compounds may be for the treatment of the same disease or condition as the compounds of formula (I) or a different disease or condition. The therapeutically active compounds may be administered simultaneously, sequentially or separately.

The compounds of formula (I) may be administered with other active compounds for the treatment of obesity and/or diabetes, for example insulin and insulin analogs, gastric lipase inhibitors, pancreatic lipase inhibitors, sulfonyl ureas and analogs, biguanides, α2 agonists, glitazones, PPAR-γ agonists, mixed PPAR-α/γ agonists, RXR agonists, fatty acid oxidation inhibitors, α-glucosidase inhibitors, dipeptidyl peptidase IV inhibitors, GLP-1 agonists e.g. GLP-1 analogues and mimetics, β-agonists, phosphodiesterase inhibitors, lipid lowering agents, glycogen phosphorylase inhibitors, antiobesity agents e.g. pancreatic lipase inhibitors, MCH-1 antagonists and CB-1 antagonists (or inverse agonists), amylin antagonists, lipoxygenase inhibitors, somostatin analogs, glucokinase activators, glucagon antagonists, insulin signalling agonists, PTP1B inhibitors, gluconeogenesis inhibitors, antilypolitic agents, GSK inhibitors, galanin receptor agonists, anorectic agents, CCK receptor agonists, leptin, serotonergic/dopaminergic antiobesity drugs, reuptake inhibitors e.g. sibutramine, CRF antagonists, CRF binding proteins, thyromimetic compounds, aldose reductase inhibitors, glucocorticoid receptor antagonists, NHE-1 inhibitors or sorbitol dehydrogenase inhibitors.

Combination therapy comprising the administration of a compound of formula (I), or a pharmaceutically acceptable salt thereof, and at least one other antiobesity agent represents a further aspect of the invention.

The present invention also provides a method for the treatment of obesity in a mammal, such as a human, which method comprises administering an effective amount of a compound of formula (I), or a pharmaceutically acceptable salt thereof, and another antiobesity agent, to a mammal in need thereof.

The invention also provides the use of a compound of formula (I), or a pharmaceutically acceptable salt thereof, and another antiobesity agent for the treatment of obesity.

The invention also provides the use of a compound of formula (I), or a pharmaceutically acceptable salt thereof, in the manufacture of a medicament for use in combination with another antiobesity agent, for the treatment of obesity.

The compound of formula (I), or a pharmaceutically acceptable salt thereof, and the other antiobesity agent(s) may be co-administered or administered sequentially or separately.

Co-administration includes administration of a formulation which includes both the compound of formula (I), or a pharmaceutically acceptable salt thereof, and the other antiobesity agent(s), or the simultaneous or separate administration of different formulations of each agent. Where the pharmacological profiles of the compound of formula (I), or a pharmaceutically acceptable salt thereof, and the other antiobesity agent(s) allow it, coadministration of the two agents may be preferred.

The invention also provides the use of a compound of formula (I), or a pharmaceutically acceptable salt thereof, and another antiobesity agent in the manufacture of a medicament for the treatment of obesity.

The invention also provides a pharmaceutical composition comprising a compound of formula (I), or a pharmaceutically acceptable salt thereof, and another antiobesity agent, and a pharmaceutically acceptable carrier. The invention also encompasses the use of such compositions in the methods described above.

GPR119 agonists are of particular use in combination with centrally acting antiobesity agents.

The other antiobesity agent for use in the combination therapies according to this aspect of the invention is preferably a CB-1 modulator, e.g. a CB-1 antagonist or inverse agonist. Examples of CB-1 modulators include SR141716 (rimonabant) and SLV-319 ((4*S*)-(-)-3-(4-chlorophenyl)-*N*-methyl-*N*-[(4-chlorophenyl)sulfonyl]-4-phenyl-4,5-dihydro-1H-pyrazole-1-carboxamide); as well as those compounds disclosed in EP576357, EP656354, WO 03/018060, WO 03/020217, WO 03/020314, WO 03/026647, WO 03/026648, WO 03/027076, WO 03/040105, WO 03/051850, WO 03/051851, WO 03/053431, WO 03/063781, WO 03/075660, WO 03/077847, WO 03/078413, WO 03/082190, WO 03/082191, WO 03/082833, WO 03/084930, WO 03/084943, WO 03/086288, WO 03/087037, WO 03/088968, WO 04/012671, WO 04/013120, WO 04/026301, WO 04/029204, WO 04/034968, WO 04/035566, WO 04/037823 WO 04/052864, WO 04/058145, WO 04/058255, WO 04/060870, WO 04/060888, WO 04/069837, WO 04/069837, WO 04/072076, WO 04/072077, WO 04/078261 and WO 04/108728, and the references disclosed therein.

Other diseases or conditions in which GPR119 has been suggested to play a role include those described in WO 00/50562 and US 6,468,756, for example cardiovascular disorders, hypertension, respiratory disorders, gestational abnormalities, gastrointestinal disorders, immune disorders, musculoskeletal disorders, depression, phobias, anxiety, mood disorders and Alzheimer's disease.

All publications, including, but not limited to, patents and patent application cited in this specification, are herein incorporated by reference as if each individual publication were specifically and individually indicated to be incorporated by reference herein as fully set forth.

The invention will now be described by reference to the following examples which are for illustrative purposes and are not to be construed as a limitation of the scope of the present invention.

### EXAMPLES

### Materials and methods

Column chromatography was carried out on SiO₂ (40-63 mesh) unless specified otherwise. LCMS data were obtained as follows: Atlantis 3µ C₁₈ column (3.0 × 20.0 mm, flow rate = 0.85 mL/min) eluting with a H₂O-CH₃CN solution containing 0.1 % HCO₂H over 6 min with UV detection at 220 nm. Gradient information: 0.0-0.3 min 100% H₂O; 0.3-4.25 min: Ramp up to 10% H₂O-90% CH₃CN; 4.25-4.4 min: Ramp up to 100% CH₃CN; 4.4-4.9 min: Hold at 100% CH₃CN; 4.9-6.0 min: Return to 100% H₂O. The mass spectra were obtained using an electrospray ionisation source in either the positive (ES⁺) or negative (ES⁻) ion modes.

Abbreviations and acronyms: Ac: Acetyl; *n*-Bu: *n*-Butyl; *t*-Bu: *tert*-Butyl; dba: dibenzylideneacetone; DBU: 1,8-diazabicyclo[5.4.0]undec-7-ene; DME: 1,2-dimethoxyethane; DMF: Dimethylformamide; Et: Ethyl; HATU: *O*-(7-azabenzotriazol-1-yl)-*N,N,N',N-*tetramethyluronium hexafluorophosphate; HBTU: *O*-benzotriazol-1-yl-*N,N,N',N-*tetramethyluronium hexafluorophosphate; h: hour(s); IH: Isohexane; *m*CPBA: 3-Chloroperoxybenzoic acid; Me: Methyl; Ph: Phenyl; RP-HPLC: Reverse phase-high performance liquid chromatography; rt: Room temperature; RT: Retention time; THF: Tetrahydrofuran; XantPhos: 9,9-Dimethyl-4,5-bis(diphenylphosphino)xanthene.

The syntheses of the following compounds have been described elsewhere: 1-(2-Bromoethyl)-4-methylsulfanylbenzene: Avery M. A., et al, J. Med. Chem., 2003, 46, 4244-4258; 2-Chloro-5-methoxypyrimidine: Chesterfield J. H., et al., Pyrimidines. Part XI, J. Chem. Soc., 1960, 4590-4596; 2-Chloro-5-methylpyrimidine: US2002/0165241; 4-(2-Ethoxycarbonyl-1-methylethyl)piperidine-1-carboxylic acid *tert*-butyl ester: Kaneko, T., et al., US Patent 6,518,423; Ethyl(3-fluoro-4-methylsulfanylphenyl)acetate: Fyfe, M. C. T., et al., WO 04/072031; 3-Fluoro-4-methylsulfanylphenol and 3-Fluoro-4-methylsulfanylaniline: WO2002/083643; 4-Hydroxy-4-(3-hydroxypropyl)piperidine-1-carboxylic acid *tert*-butyl ester: Cooper L. C., et al., Bioorg. Med. Chem. Lett., 2002, 12, 1759-1763; 4-(3-Hydroxy-2-methylpropyl)piperidine-1-carboxylic acid *tert*-butyl ester: Caldwell, C., et al., WO 00/059503; 4-(3-Hydroxypropyl)piperidine-1-carboxylic acid *tert*-butyl ester: Siegel M. G., et al., Tetrahedron, 1999, 55, 11619-11640; (4-Methoxycarbonylbenzyl)triphenylphosphonium bromide: Gross J., et al., Angew. Chem.(GE), 1995,107, 523-526; (4-Methylsulfanylbenzyl)phosphonic acid diethyl ester and (4-Methanesulfonylbenzyl)phosphonic acid diethyl ester: Ulman A., et al., J. Am. Chem. Soc.,1990, 112, 7083-7090; 4-(3-Oxopropyl)piperidine-1-carboxylic acid *tert*-butyl ester: Keenan, R M., et al., J. Med. Chem. 1999, 42, 545-559; 3-Piperidin-4-ylpropyl acetate: Askew, B., et al. US Patent 5,559,127. All other compounds were available from commercial sources.

### Preparation 1: 4-[4-(4-Methanesulfonylphenyl)butyl]piperidine

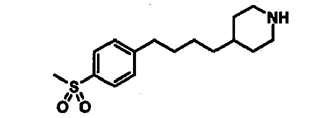

Trifluoroacetic acid (3.0 mL) was added to a stirred solution of 4-[4-(4-methanesulfonylphenyl)butyl]piperidine-1-carboxylic acid *tert*-butyl ester (**Example 46**, 500 mg, 1.27 mmol) in CH₂Cl₂ (10 mL). After 0.5 h, the solvent was removed and the residue partitioned between CH₂Cl₂ (20 mL) and saturated aqueous Na₂CO₃ (10 mL). The aqueous phase was re-extracted with CH₂Cl₂ (20 mL), the combined organics washed with brine (10 mL) and dried (MgSO₄). Removal of the solvent afforded the title compound: RT = 2.29 min, *m*/*z* (ES⁺) = 296.2 [*M* + H]⁺.

### Preparation 2: 4-[3-(Diethoxyphosphoryl)propyl]piperidine-1-carboxylic acid tert-butyl ester

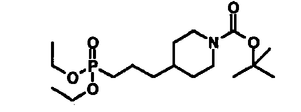

4-(2-Oxoethyl)piperidine-1-carboxylic acid *tert*-butyl ester (1 g, 4.4 mmol) and bis(diethoxyphosphinyl)methane (1.09 mL, 4.4 mmol) were dissolved in CH₂Cl₂ (6.4 mL) and a 50% w/v aqueous NaOH solution (6.4 mL) added. After stirring for 10 min the reaction mixture was diluted with water (15 mL) and extracted with EtOAc (4 × 30 mL). The combined organic phases were dried (MgSO₄) and evaporated to afford 4-[(*E*)-3-(diethoxyphosphoryl)allyl]-piperidine-1-carboxylic acid *tert*-butyl ester: δ_{H} (CDCl₃) 1.08 (q, 2H) 1.32 (t, 6H), 1.45 (s, 9H), 1.55-1.67 (m, 3H), 2.17 (t, 2H), 2.67 (t, 2H), 4.03-4.19 (m, 6H), 5.66 (dd, 1H), 6.72 (m, 1H). A sample of this olefin (1.64 g, 4.54 mmol) was dissolved in EtOH (15 mL) and Pd (10% on C, 164 mg, 155 µmol) added. The atmosphere was exchanged for H₂ and the mixture stirred for 60 h. Filtration through a pad of celite, washing through with MeOH (4 × 5 mL), removal of the solvent and purification of the residue by column chromatography (IH-EtOAc 1:4) afforded the title phosphonate: δ_{H} (CDCl₃) 1.08 (dq, 2H), 1.30-1.47 (m, 3H), 1.32 (t, 6H), 1.45 (s, 9H), 1.61-1.73 (m, 6H), 2.05 (t, 2H), 4.04-4.13 (m, 6H).

### Preparation 3: 4-Methylsulfanyl-3-trifluoromethylbenzaldehyde

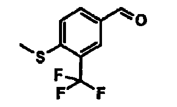

A solution of 4-fluoro-3-trifluoromethylbenzaldehyde (1.0 g, 5.21 mmol) in anhydrous DMF (7 mL) was treated with sodium thiomethoxide (365 mg, 5.21 mmol) and the resulting solution stirred for 18 h at rt. The DMF was evaporated, the residue taken up in Et₂O (80 mL) and this ethereal solution washed with water (30 mL) and saturated aqueous Na₂CO₃ (30 mL) then dried (MgSO₄). The solvent was removed and the residue purified by column chromatography (IH-EtOAc 20:1) to give the title compound: δ_{H} (CDCl₃) 2.61 (s, 3H), 7.46 (d, 1H), 7.98 (d, 1H), 8.11 (s, 1H), 9.99 (s, 1H).

The aldehydes listed in **Table 1** were prepared from the corresponding aryl fluorides using the method described in **Preparation 3.**

**Table 1**

| **Prep** | **Structure** | **Name** | δ**_{H} (CDCl₃)** |
|---|---|---|---|
| **4** | 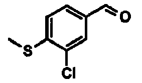 | 3-Chloro-4-methylsulfanyl benzaldehyde | 2.55 (s, 3H), 7.27 (d, 1H), 7.75 (dd, 1H), 1.83 (d, 1H), 9.91 (s, 1H) |
| **5** | 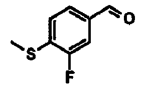 | 3-Fluoro-4-methylsulfanyl benzaldehyde | 2.54 (s, 3H), 7.32 (t, 1H), 7.51 (dd, 1H), 7.64 (dd, 1H), 9.92 (s, 1H) |
| **6** | 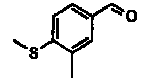 | 3-Methyl-4-methylsulfanyl benzaldehyde | 2.38 (s, 3H), 2.55 (s, 3H), 7.24 (d, 1H), 7.63 (s, 1H), 7.69 (d, 1H), 9.92 (s, 1H) |
| **7** | 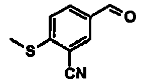 | 5-Formyl-2-methylsulfanyl benzonitrile | 2.65 (s, 3H), 7.41 (d, 1H), 8.02 (d, 1H), 8.07 (s, 1H), 9.95 (s, 1H) |

### Preparation 8: N-(4-Methylsulfanylphenyl)-2-nitrobenzenesulfonamide

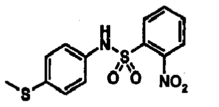

A solution of 2-nitrobenzenesulfonyl chloride (1.145 g, 5.17 mmol) in anhydrous CH₂Cl₂ (5 mL) was added in a dropwise manner to a stirred solution of 4-(methylthio)aniline (0.685 g, 4.92 mmol) and pyridine (540 µL, 6.64 mmol) in anhydrous CH₂Cl₂ (15 mL). After 1.5 h the solution was evaporated to dryness and the residue partitioned between EtOAc (120 mL) and saturated aqueous NH₄Cl (50 mL). The organic phase was separated, washed with brine (20 mL) and dried (MgSO₄). The solvent was reduced to a small volume and passed through a short silica plug, eluting with EtOAc. The volume of EtOAc was adjusted to 15 mL and IH (50 mL) added to induce crystallisation of the title compound: δ_{H} (CDCl₃) 2.47 (s, 3H), 7.15 (d, 2H), 7.17 (d, 2H), 7.22 (s, 1H), 7.62 (t, 1H), 7.73 (t, 1H), 7.84 (d, 1H), 7.89 (d, 1H).

### Preparation 9: 4-(3-Methanesulfonyloxypropyl)piperidine-1-carboxylic acid tert-butyl ester

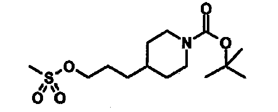

A stirred solution of 4-(3-hydroxypropyl)piperidine-1-carboxylic acid *tert*-butyl ester (244 mg, 1 mmol) and dry NEt₃ (280 µL, 2 mmol) in anhydrous CH₂Cl₂ (4.5 mL) was cooled to 0°C and methanesulfonyl chloride (93 µL, 1.2 mmol) introduced in a dropwise manner. The reaction was brought to rt and stirring continued for 30 min. The mixture was diluted with EtOAc (50 mL), washed with 0.5 M aqueous HCl (10 mL), water (10 mL), saturated aqueous sodium bicarbonate (10 mL) and brine (10 mL) then dried (MgSO₄). Evaporation of the solvent afforded the title compound: δ_{H} (CDCl₃) 1.10 (dq, 2H), 1.33-1.41 (m; 3H), 1.46 (s, 9H), 1.65 (d, 2H), 1.78 (dt, 2H), 2.68 (t, 2H), 3.01 (s, 3H), 4.09 (m, 2H), 4.22 (t, 2H).

### Preparation 10: 4-(2-Methanesulfonyloxyethyl)piperidine-1-carboxylic acid tert-butyl ester

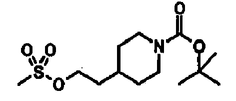

Using the same procedure as that described in **Preparation 9**, 4-(2-hydroxyethyl)-piperidine-1-carboxylic acid *tert*-butyl ester was converted to the corresponding mesylate: δ_{H} (CDCl₃) 1.14 (dq, 2H), 1.46 (s, 9H), 1.62-1.73 (m, 5H), 2.70 (t, 2H), 3.02 (s, 3H), 4.10 (m, 2H), 4.29 (t, 2H).

### Preparation 11: 4-(4-Oxobutyl)piperidine-1-carboxylic acid tert-butyl ester

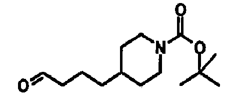

A stirred suspension of Dess-Martin periodinane (165 mg, 391 µmol) in CH₂Cl₂ (3 mL) was cooled on ice and a solution of 4-(4-hydroxybutyl)piperidine-1-carboxylic acid *tert*-butyl ester in CH₂Cl₂ (2 mL) added. Stirring was continued for 1 h at rt whereupon the solvent was evaporated and the residue taken up in ether (20 mL). The organic phase was washed with brine (3 mL), dried (MgSO₄) and evaporated. The residue was purified by column chromatography (IH-Et₂O 2:1) to afford the title aldehyde: δ_{H} (CDCl₃) 1.09 (dq, 2H), 1.25-1.30 (m, 2H), 1.36-1.45 (m, 1H), 1.46 (s, 9H), 1.62-1.70 (m, 4H), 2.44 (t, 2H), 2.68 (t, 2H), 4.08 (m, 2H), 9.78 (s, 1H).

### Preparation 12: 4-Methanesulfonylbenzenethiol

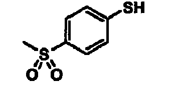

Solid sodium hydrosulfide monohydrate (0.88 g, 11.95 mmol) was added in one portion to a solution of 4-fluorophenylmethyl sulfone (1.74 g, 9.99 mmol) in DMF (10 mL). After stirring for 18 h, water (20 mL) and 2 M aqueous HCl (20 mL) were added and the resulting acidic solution extracted with EtOAc (2 × 100 mL). The organic phase was washed with water (2 × 50 mL) and brine (100 mL) then dried (MgSO₄) and evaporated. Ether (60 mL) was added to the brown residue and the mixture extracted with 2 M aqueous NaOH (50 mL). The aqueous phase was separated and acidified to pH 1 with cone HCl and extracted with EtOAc (2 × 50 mL). After drying (MgSO₄), the organics were evaporated to afford the title compound: δ_{H} (CDCl₃) 3.06 (s, 3H), 3.73 (s, 1H), 7.43 (d, 2H), 7.80 (d, 2H).

### Preparation 13: 2-Hydroxy-1-oxa-8-azaspiro[4.5]decane-8-carboxylic acid tert-butyl ester

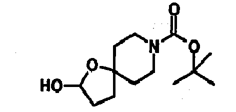

A stirred solution of 4-hydroxy-4-(3-hydroxypropyl)piperidine-1-carboxylic acid *tert-*butyl ester (1.0 g, 3.86 mmol) in CH₂Cl₂ (60 mL) was cooled on an ice bath and Dess-Martin periodinane (1.8 g, 4.24 mmol) added. After 1 h, the reaction mixture was diluted with ether (120 mL) and washed with 2 M aqueous NaOH (70 mL). The aqueous phase was extracted with ether (60 mL) and the combined organics washed with water (50 mL) and brine (50 mL) then dried (MgSO₄). The solvent was removed and the residue purified by column chromatography (IH-EtOAc 3:2) to afford the title compound: δ_{H} (CDCl₃) 1.48 (s, 9H), 1.48-1.62 (m, 2H), 1.62-1.77 (m, 3H), 1.91-2.08 (m, 3H), 3.35 (m, 2H), 3.56 (m, 2H), 5.51 (d, 1H).

### Preparation 14: 4-(3-Ethoxycarbonyl-2-oxopropyl)piperidine-1-carboxylic acid tert-butyl ester

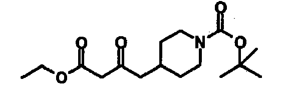

A solution of 2-(1-*tert*-butoxycarbonylpiperidine-4-yl)acetic acid (5 g, 20.58 mmol) in THF (40 mL) and carbonyldiimidazole (3.94 g, 24.28 mmol) was was stirred at rt for 5 h then added to a previously prepared mixture of the potassium salt of ethyl malonate (4.66 g, 27.37 mmol) and magnesium chloride (1.96 g, 20.58 mmol) that had been heated under reflux in THF (50 mL) for 5 h. Stirring of the resulting reaction mixture was continued at rt for 18 h, after which time it was poured into ice-water (250 mL). Sufficient conc HCl was added to bring the aqueous phase to pH 7. This was then extracted with EtOAc (2 × 300 mL), the combined organics washed with brine (50 mL) and dried (MgSO₄). Evaporation of the solvent and purification of the residue by column chromatography (IH-EtOAc 4:1) gave the title compound: δ_{H} (CDCl₃) 1.12 (dq, 2H), 1.29 (t, 3H), 1.46 (s, 9H), 1.68 (d, 2H), 2.03 (m, 1H) 2.48 (d, 2H), 2.73 (t, 2H), 3.42 (s, 2H), 4.07 (m, 2H), 4.20 (q, 2H).

### Preparation 15: 2-Fluoro-4-hydroxybenzoic acid methyl ester

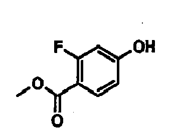

Trimethylsilyldiazomethane (7 mL of a 2 M solution in hexane, 14 mmol) was added in a dropwise manner to a stirred solution of 2-fluoro-4-hydroxybenzoic acid (2.0 g, 12.8 mmol) in toluene (15 mL). After 5 min, AcOH (75 µL) and water (20 mL) were added and the aqueous phase separated and extracted with EtOAc (50 mL). The combined organics were extracted with 1 M aqueous NaOH (3 × 30 mL) and the combined aqueous extracts acidified to pH 2 using aqueous HCl. The resulting suspension was extracted into EtOAc (100 mL), dried (MgSO₄) and evaporated to afford the title ester: δ_{H} (DMSO) 3.78 (s, 3H), 6.63 (dd, 1H), 6.70 (dd, 1H), 7.75 (t, 1H), 10.79 (s, 1H).

### Preparation 16: 4-(2-Oxoethoxy)piperidine-1-carboxylic acid tert-butyl ester

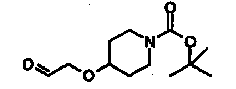

Borane (11.58 mL of a 1.0 M solution in THF, 11.58 mmol) was added slowly to a stirred solution of 4-carboxymethoxypiperidine-1-carboxylic acid tert-butyl ester (1.50 g, 5.79 mmol) in anhydrous THE (30 mL) at 0°C. After 4 h, the reaction was quenched by the slow addition of saturated aqueous NaHCO₃ (30 mL) and extracted with EtOAc (2 × 50 mL). The combined organics were washed with brine (50 mL), dried (MgSO₄), evaporated and purified by column chromatography (EtOAc) to afford 4-(2-hydroxyethoxy)piperidine-1-carboxylic acid *tert*-butyl ester: δ_{H} (CDCl₃) 1.46 (s, 9H), 1.52 (m, 2H), 1.86 (m, 2H), 2.06 (t, 1H), 3.08 (ddd, 2H), 3.50 (tt, 1H), 3.58 (t, 2H), 3.71-3.80 (m, 4H). A solution of this alcohol (200 mg, 820 µmol) in CH₂Cl₂ (5 mL) was treated with Dess-Martin periodinane (381 mg, 900 µmol) added in one portion. After stirring at rt for 2 h, the reaction mixture was partitioned between CH₂Cl₂ (30 mL) and saturated aqueous NaHCO₃ (15 mL). The aqueous phase was extracted further with CH₂Cl₂ (2 × 30 mL), the combined organics washed with brine (10 mL) and dried (MgSO₄). The solvent was removed and the residue purified by column chromatography (IH-EtOAc 1:1) to afford the title compound: δ_{H} (CDCl₃) 1.44 (s, 9H), 1.56 (m, 2H), 1.85 (m, 2H), 3.09 (ddd, 2H), 3.53 (tt, 1H), 3.77 (m, 2H), 4.10 (s, 2H), 9.72 (s, 1H).

### Preparation 17: 4-[4-(4-Aminophenyl)butyl]piperidine-1-carboxylic acid tert-butyl ester

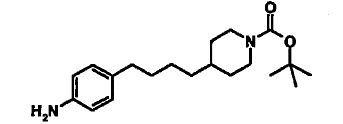

A stirred solution of diethyl(4-nitrobenzyl)phosphonate (798 mg, 2.90 mmol) in anhydrous dimethoxyethane (15 mL) was treated portionwise with NaH (116 mg of a 60% dispersion in oil, 2.90 mmol). After 5 min, a solution of 4-(3-oxopropyl)piperidine-1-carboxylic acid *tert*-butyl ester (500 mg, 2.07 mmol) in dimethoxyethane (4 mL) was added and stirring continued for 1.5 h. The reaction was quenched by addition of saturated aqueous NH₄Cl (10 mL) and the mixture extracted with EtOAc (2 × 80 mL). The combined organic phases were washed with brine (15 mL), dried (MgSO₄), evaporated and the residue purified by column chromatography (IH-EtOAc 4:1) to give 4-[4-(4-nitrophenyl)but-3-enyl]piperidine-1-carboxylic acid *tert*-butyl ester: RT = 4.45 min; *m*/*z* (ES⁺) = 361.2 [*M* + H]⁺. A sample of this olefin (613 mg, 1.70 mmol) was dissolved in a mixture of EtOH (30 mL) and EtOAc (30 mL) and a slurry of Pd (10% on C, 61 mg, 58 µmol) in EtOH (1 mL) added. An atmosphere of H₂ was introduced and the reaction stirred 18 h at rt. After filtration through a pad of Celite, the solvent was removed to give the title aniline: RT = 3.19 min; *m*/*z* (ES⁺) = 333.2 [*M* + H]⁺.

### Preparation 18: 4-[3-(4-Aminophenyl)propyl]piperidine-1-carboxylic acid tert-butyl ester

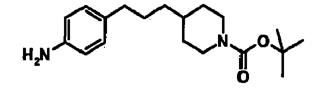

The title compound was prepared from diethyl(4-nitrobenzyl)phosphonate and 4-(2-oxoethyl)piperidine-1-carboxylic acid *tert*-butyl ester using the method described in **Preparation 17:** RT = 3.01 min; *mlz* (ES⁺) = 319.2 [*M* + H]⁺.

### Preparation 19: 4-[3-(4-Ethylsulfanyl-3-fluorophenoxy)propyl]piperidine-1-carboxylic acid tert-butyl ester

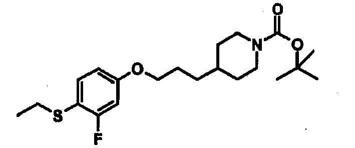

A mixture of 4-bromo-3-fluorophenol (2.0 g, 10.5 mmol), di-*tert*-butylazodicarboxylate (3.6 g, 15.7 mmol), PPh₃ (4.1 g, 15.7 mmol), and 4-(3-hydroxypropyl)piperidine-1-carboxylic acid *tert*-butyl ester (2.5 g, 10.5 mmol) in anhydrous PhMe (80 mL) was stirred at 20°C for 16 h. The reaction was quenched with 1M HCl, the organic phase washed with saturated aqueous Na₂CO₃ and brine, then dried (MgSO₄). The solution was filtered and concentrated to give an oil that was treated with IH and Et₂O to precipitate out Ph₃PO. The Ph₃PO was removed by filtration, the solution concentrated and the residue purified by column chromatography (EtOAc-IH, 1:9) to furnish 4-[3-(4-bromo-3-fluorophenoxy)-propyl]piperidine-1-carboxylic acid *tert*-butyl ester: *m*/*z* (ES⁺) = 416.0 [*M* + *H*]⁺. A mixture of this aryl bromide (107 mg, 260 µmol), Pd₂(dba)₃ (24 mg, 26 µmol), and XantPhos (17 mg, 29 µmol) in anhydrous xylene (1.5 mL) was stirred under argon for 20 min. NaSEt (26 mg, 310 µmol) was added and the reaction heated under reflux for 22 h. On cooling to rt, the reaction was diluted with EtOAc and washed with H₂O and brine. The solution was dried (MgSO₄), filtered, and concentrated to give a gum that was purified by column chromatography (CH2Cl₂-IH [1:1], then CH₂Cl₂, then CH₂Cl₂-EtOAc [9:1]) to afford the title compound: *mlz* (ES⁺) = 398.1 [*M* + H]⁺.

The aryl bromides listed in **Table 2** were prepared via Mitsunobu reactions, using methods similar to those described in **Preparation 19.**

**Table 2**

| **Prep** | **Structure** | **Name** | **m/z (ES⁺)** |
|---|---|---|---|
| **20** | 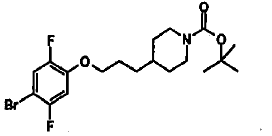 | 4-[3-(4-Bromo-2,5-difluorophenoxy)propyl]-piperidine-1-carboxylic acid *tert*-butyl ester | 434.0 [*M* + H]⁺ |
| **21** | 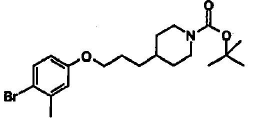 | 4-[3-(4-Bromo-3-methyl-phenoxy)propyl]-piperidine-1-carboxylic acid *tert*-butyl ester | 412.0 [*M* + H]⁺ |
| **22** | 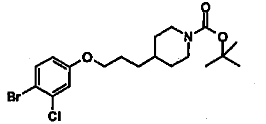 | 4-[3-(4-Bromo-3-chloro-phenoxy)propyl]-piperidine-1-carboxylic acid *tert*-butyl ester | 434.0 [*M* + H]⁺ |
| **23** | 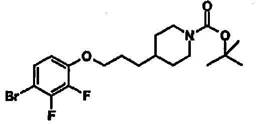 | 4-[3-(4-Bromo-2,3-difluorophenoxy)propyl]-piperidine-1-carboxylic acid *tert*-butyl ester | 434.0 [*M* + H]⁺ |

### Preparation 24: 4-(2-Bromoethyl)-2-fluoro-1-methylsulfanylbenzene

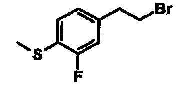

A solution of ethyl(3-fluoro-4-methylsulfanylphenyl)acetate (2.00 g, 8.8 mmol) in anhydrous THF (13 mL) was added to a stirred suspension of LiAlH₄ (0.35 g, 9.2 mmol) in anhydrous THF (20 mL) at 0°C. The reaction was warmed to rt, before being stirred for 30 min. More LiAlH₄ (16 mg, 0.4 mmol) was added and stirring continued for another 30 min. The reaction was cooled to 0°C, before being quenched with saturated aqueous Na₂SO₄ (25 mL) and filtered through celite. The remainder was concentrated in vacuo and the residue partitioned between H₂O and EtOAc. The aqueous phase was further extracted with EtOAc, the combined organic extracts dried (MgSO₄), filtered and concentrated. Column chromatography yielded 2-(3-fluoro-4-methylsulfanylphenyl)ethanol: *m*/*z* (ES⁺) = 187.0 [*M* + H]⁺. A stirred solution of this alcohol (1.16 g, 6.24 mmol) in anhydrous Et₂O (47 mL) and MeCN (16 mL) was treated with PPh₃ (4.91 g, 18.7 mmol) and imidazole (1.27 g, 18.7 mmol), followed by Br₂ (0.96 mL, 18.7 mmol). After 2 h, the solvents were removed in vacuo and the residue purified by column chromatography (EtOAc-IH, 1:99) to furnish the title compound: RT = 3.92 min.

### Preparation 25: 2-Fluoro4-hydroxybenzenesulfonamide

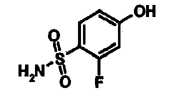

4-Amino-2-fluorobenzenesulfonamide (150 mg, 0.79 mmol) was added portionwise to a stirred mixture of ice (2 g) and H₂SO₄ (100 µL). A solution of NaNO₂ (54 mg, 0.79 mmol) in ice water (1 mL) was added dropwise to the reaction, the temperature of the reaction being maintained between 0 and 10°C. After 0.5 h, the reaction mixture was transferred to a dropping funnel containing H₂O (3 mL), before being added dropwise to a boiling solution of CuSO₄ (500 mg, 3.15 mmol) in water (5 mL) and H₂SO₄ (0.5 mL). The reaction was heated at 135°C (bath) for 0.5 h, before being cooled and extracted with EtOAc (2 × 150 mL). The combined organic layers were extracted with 2M NaOH (2 × 40 mL). The aqueous extracts were acidified to pH 3 with 12M HCl, before being extracted with EtOAc (2 × 150 mL). The organic extracts were dried (MgSO₄), filtered, and concentrated and the residue purified by column chromatography (IH-EtOAc, 9:11) to furnish the title compound: δ_{H} ((CD₃)₂SO) 6.68-6.75 (m, 2H), 7.39 (s, 2H), 7.60 (t, 1H); *m*/*z* (ES⁻) = 190.1 [*M* - H]⁻.

### Preparation 26: 4-(3-Hydroxypropyl)piperidine-1-carboxylic acid isopropyl ester

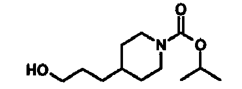

*i*-PrOCOCl (1M in PhMe, 28.1 mL, 28.1 mmol) was added to a solution of 3-piperidin-4-yl-propyl acetate (10.0 g, 54.0 mmol) and NEt₃ (8.1 g, 80.2 mmol) in anhydrous CH₂Cl₂ (100 mL) over 5 min. The reaction was stirred for 3 h, then the mixture was washed with 1M HCl (2x), saturated aqueous Na₂CO₃, and brine and dried (MgSO₄). The solution was filtered and concentrated, before being taken up in MeOH (50 mL). 2M NaOH was added and the reaction stirred for 4 h. The MeOH was removed under reduced pressure and the remainder extracted with EtOAc. The organic extracts were dried (MgSO₄) filtered and concentrated to give an oil that was purified by flash chromatography (EtOAc-CH₂Cl₂, 1:1) to afford the title compound: δ_{H} (CDCl₃) 1.05-1.15 (m, 2H), 1.23 (d, 6H), 1.25-1.35 (m, 2H), 1.40-1.50 (m, 1H), 1.55-1.60 (m, 2H), 1.65-1.70 (m, 2H), 2.65-2.75 (m, 2H), 3.60-3.67 (m, 2H), 4.05-4.15 (br m, 2H), 4.90 (sept, 1H).

### Preparation 27: 4-(3-Methanesulfonyloxypropyl)piperidine-1-carboxylic acid isopropyl ester

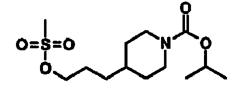

Using the same procedure as that described in **Preparation 9**, 4-(3-hydroxypropyl)piperidine-1-carboxylic acid isopropyl ester **(Preparation 26)** was converted into the corresponding mesylate: *m*/*z* (ES⁺) = 308.1 [*M* + H]⁺.

### Preparation 28: 4-[3-(3-Fluoro4-methylsulfanylphenylamino)propyl]piperidine-1-carboxylic acid isopropyl ester

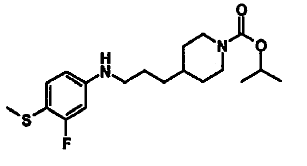

3-Fluoro-4-methylsulfanylaniline was reacted with 4-(3-methanesulfonyloxypropyl)-piperidine-1-carboxylic acid isopropyl ester (**Preparation 27**), employing a procedure similar to that outlined in **Example 161**, to furnish the title compound: *mlz* (ES⁺) = 369.0 [*M* + H]⁺.

### Preparation 29: 4-(3-Hydroxy-1-methylpropyl)piperidine-1-carboxylic acid tert-butyl ester

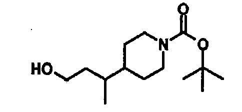

LiAlH₄ was added portionwise to a stirred solution of 4-(2-ethoxycarbonyl-1-methylethyl)piperidine-1-carboxylic acid *tert*-butyl ester (1.0 g, 3.3 mmol) in anhydrous THF (10 mL) at 0°C. The mixture was warmed to rt and stirred for 1 h. The reaction was quenched with H₂O (2.5 mL), 2M NaOH (0.25 mL), and H₂O (2.5 mL), before being extracted with EtOAc (2x). The organic extracts were washed with brine (3x), dried (MgSO₄), filtered and concentrated to yield the title compound: δ_{H} (CDCl₃) 0.90 (d, 3H), 1.15-1.45 (m, 5H), 1.49 (s, 9H), 1.50-1.80 (m, 3H), 2.20 (s, 1H), 2.60-2.70 (br m, 2H), 3.60-3.80 (m, 2H), 4.05-4.20 (br m, 2H).

### Preparation 30: 4-(3-Hydroxybutyl)piperidine-1-carboxylic acid tert-butyl ester

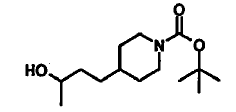

MeMgCl (3.0M in THF, 1.41 mL, 4.24 mmol) was added to a stirred solution of 4-(3-oxopropyl)piperidine-1-carboxylic acid *tert*-butyl ester (0.93 g, 3.86 mmol) in anhydrous THF (10 mL) at -40°C. After 1 h, more MeMgCl (3.0M in THF, 1.41 mL, 4.24 mmol) was added and stirring continued at -40°C for an additional hour. The reaction was quenched with saturated aqueous NH₄Cl, warmed to rt and extracted with CH₂Cl₂. The organic extracts were dried (MgSO₄), filtered, concentrated and the residue purified by flash chromatography (EtOAc-IH, 2:3) to yield the title compound: δ_{H} (CDCl₃) 1.05-1.45 (m, 10H), 1.49 (s, 9H), 1.65-1.72 (m, 2H), 2.65-2.75 (m, 2H), 3.77-3.85 (m, 1H), 4.05-4.20 (m, 2H).

### Preparation 31: 4-[3-(4-Amino-3-fluorophenoxy)propyl]piperidine-1-carboxylic acid tert-butyl ester

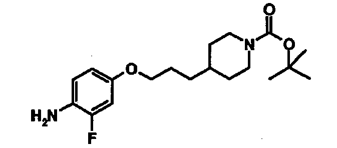

Mitsunobu condensation of 3-fluoro4-nitrophenol with 4-(3-hydroxypropyl)piperidine-1-carboxylic acid *tert*-butyl ester, utilizing a procedure similar to that outlined in **Example 156,** furnished 4-[3-(3-fluoro-4-nitrophenoxy)propyl]piperidine-1-carboxylic acid *tert*-butyl ester: δ_{H} (CDCl₃) 1.10-1.20 (m, 2H), 1.40-1.55 (m, 12H), 1.67-1.75 (m, 2H), 1.82-1.92 (m, 2H), 2.65-2.80 (m, 2H), 4.00-4.20 (m, 4H), 6.73-6.80 (m, 2H), 8.13 (t, 1H). A solution of this compound (1.85 g) in EtOH (20 mL) was treated with Pd (10% on C, 0.25 g) and the reaction stirred under a hydrogen atmosphere for 20 h, before being filtered through celite. The filtrate was concentrated in vacuo to provide the title compound: *m*/*z* (ES⁺) = 353.2 [*M* + H]⁺.

### Example 1: 4-[(E)-4-(4-Methoxycarbonylphenyl)but-3-enyl]piperidine-1-carboxylic acid tert-butyl ester

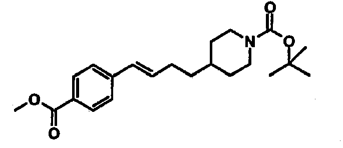

A suspension of (4-methoxycarbonylbenzyl)triphenylphosphonium bromide (2.28 g, 4.64 mmol) was suspended in dimethoxyethane (13 mL) and sodium hydride (186 mg of a 60% dispersion in oil, 4.64 mmol) added portionwise. After stirring for 20 min, a solution of 4-(3-oxopropyl)piperidine-1-carboxylic acid *tert*-butyl ester (800 mg, 3.31 mmol) in DME (6.5 mL) was introduced and the resulting reaction stirred for 20 h at rt. Saturated aqueous NH₄Cl (30 mL) was added and the mixture extracted with EtOAc (2 × 30 mL). The combined organics were dried (MgSO₄), the solvent removed and the residue purified by column chromatography (IH-EtOAc 95:5, 9:1 and 4:1) to give the title compound: RT = 4.51 min; *mlz* (ES⁺) = 374.2 [*M* + H]⁺.

### Example 2: 4-[4-(4-Methoxycarbonylphenyl)butyl]piperidine-1-carboxylic acid tert-butyl ester

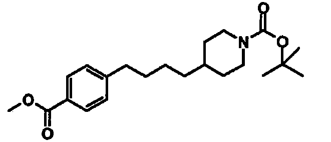

A slurry of Pd (10% on C, 28 mg, 27 µmol) in EtOAc (1 mL) was added to a solution of 4-[(*E*)-4-(4-methoxycarbonylphenyl)but-3-enyl]piperidine-1-carboxylic acid *tert*-butyl ester (**Example 1**, 280 mg, 750 µmol) in EtOH (15 mL) and stirred vigorously under an atmosphere of H₂. After 60 h the mixture was filtered through a pad of Celite, washing through with EtOAc (3 × 10 mL), and the combined filtrates evaporated to afford the title compound: RT = 4.67 min; *mlz* (ES⁺) = 376.2 [*M* + H]⁺.

### Example 3: 4-[4-(4-Carboxyphenyl)butyl]piperidine-1-carboxylic acid tert-butyl ester

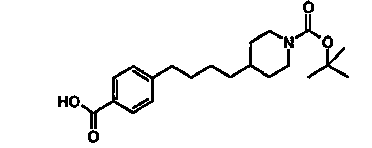

A solution of 4-[4-(4-methoxycarbonylphenyl)butyl]piperidine-1-carboxylic acid *tert-*butyl ester **(Example 2**, 376 mg, 1 mmol) in THF (5 mL) was treated with 2 M aqueous sodium hydroxide (1.6 mL, 3.1 mmol). Sufficient MeOH was added to ensure homogeneity of the stirred mixture. After 18 h the solvent was reduced to a small volume, the mixture diluted with water (15 mL) and washed with EtOAc (5 mL). The aqueous phase was acidified to pH 4 with 2 M aqueous HCl, saturated with NaCl, and extracted with EtOAc (50 mL). The organic phase was dried (MgSO₄) and evaporated to afford the title acid: RT = 4.16 min; *mlz* (ES⁺) = 360.4 [*M* + H]⁺.

### Example 4: 4-[4-(4-Ethylcarbamoylphenyl)butyl]piperidine-1-carboxylic acid tert-butyl ester

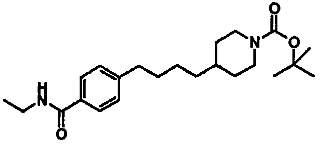

Diisopropylethylamine (28.5 µL, 165 µmol), ethylamine (110 µL of a 2 M solution in THF, 220 µmol) and HBTU (63 mg, 165 µmol) were added sequentially to a solution of 4-[4-(4-carboxyphenyl)butyl]piperidine-1-carboxylic acid *tert*-butyl ester (**Example 3,** 40 mg, 110µmol) in dimethylacetamide (0.5 mL). After stirring for 18 h, the solvent was removed, the residue taken up in EtOAc (2 mL) and washed with saturated aqueous Na₂CO₃ (2 × 2 mL) and brine (2 mL). After drying (MgSO₄), the solvent was removed to afford the title compound: RT = 4.01 min; *mlz* (ES⁺) = 389.2 [*M* + H]⁺.

The compounds listed in **Table 3** were prepared by reaction of the appropriate amine with 4-[4-(4-carboxyphenyl)butyl]piperidine-1-carboxylic acid *tert*-butyl ester **(Example 3)** using the method described in **Example 4.**

**Table 3**

| **Eg** | **Structure** | **Name** | **RT (min)** | ***mlz* (ES⁺)** |
|---|---|---|---|---|
| **5** | 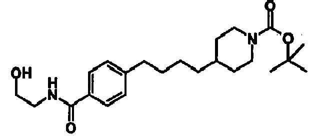 | 4-{4-[4-(2-Hydroxyethyl carbamoyl)phenyl]butyl} piperidine-1-carboxylic acid *tert*-butyl ester | 3.69 | 405.2 [*M* + H]⁺ |
| **6** | 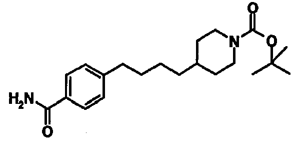 | 4-[4-(4-Carbamoylphenyl)butyl] piperidine-1-carboxylic acid *tert-*butyl ester | 3.76 | 361.2 [*M* + H]⁺ |
| **7** | 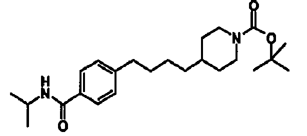 | 4-[4-(4-Isopropylcarbamoyl phenyl)butyl]piperidine-1-carboxylic acid *tert*-butyl ester | 4.19 | 403.3 [*M* + H]⁺ |
| **8** | 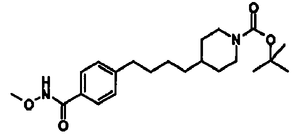 | 4-[4-(4-Methoxycarbamoyl phenyl)butyl]piperidine-1-carboxylic acid *tert*-butyl ester | 3.94 | 391.3 [*M* + H]⁺ |
| **9** | 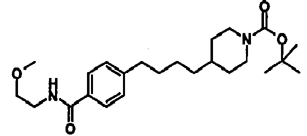 | 4-{4-[4-(2-Methoxyethyl carbamoyl)phenyl]butyl} piperidine-1-carboxylic acid *tert*-butyl ester | 3.99 | 419.3 [*M* + H]⁺ |
| **10** | 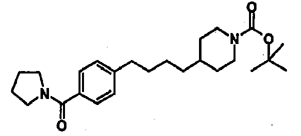 | 4-{4-[4-(Pyrrolidine-1-carbonyl) phenyl]butyl}piperidine-1-carboxylic acid *tert*-butyl ester | 4.05 | 415.3 [*M* + H]⁺ |
| **11** | 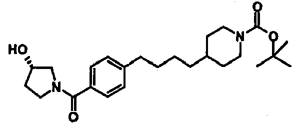 | 4-{4-[4-((*S*)-3-Hydroxy pyrrolidine-1-carbonyl)phenyl] butyl}piperidine-1-carboxylic acid *tert*-butyl ester | 3.61 | 431.3 [*M* + H]⁺ |
| **12** | 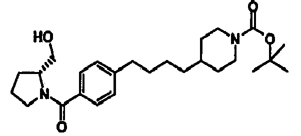 | 4-{4-[4-((*R*)-2-Hydroxymethyl pyrrolidine-1-carbonyl)phenyl] butyl}piperidine-1-carboxylic acid *tert*-butyl ester | 3.81 | 445.3 [*M* + H]⁺ |
| **13** | 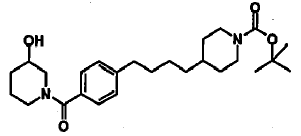 | 4-{4-[4-(3-Hydroxypiperidine-1-carbonyl)phenyl]butyl}piperidine -1-carboxylic acid *tert*-butyl ester | 3.86 | 445.3 [*M* + H]⁺ |
| **14** | 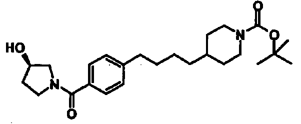 | 4-{4-[4-((*R*)-3-Hydroxy pyrrolidine-1-carbonyl)phenyl] butyl}piperidine-1-carboxylic acid *tert*-butyl ester | 3.67 | 431.3 [*M* + H]⁺ |
| **15** | 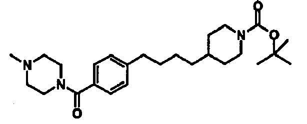 | 4-{4-[4-(4-Methylpiperazine-1-carbonyl)phenyl]butyl}piperidine -1-carboxylic acid *tert*-butyl ester | 3.07 | 444.3 [*M* + H]⁺ |
| **16** | 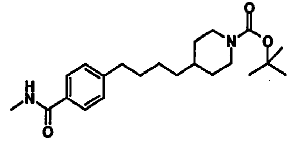 | 4-[4-(4-Methylearbamoylphenyl) butyl]piperidine-1-carboxylic acid *tert*-butyl ester | 3.99 | 375.3 [*M* + H]⁺ |
| **17** | 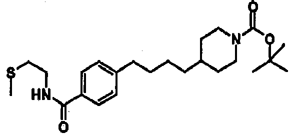 | 4-{4-[4-(2-Methylsulfanylethyl carbamoyl)phenyl]butyl} piperidine-1-carboxylic acid *tert-* butyl ester | 4.16 | 435.3 [*M* + H]⁺ |

### Examples 18 and 19: 4-{4-[4-(2-Methanesulfinylethylcarbamoyl)phenyl]butyl}piperidine-1-carboxylic acid tert-butyl ester and 4-{4-[4-(2-medianesulfonylethylcarbamoyl)phonyl]butyl}-piperidine-1-carboxylic acid tert-butyl ester

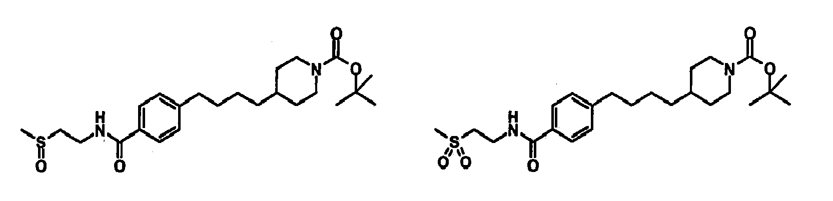

A sample of 4-{4-[4-(2-methylsulfanylethylcarbamoyl)phenyl]butyl}piperidine-1-carboxylic acid *tert*-butyl ester (**Example 17**, 30 mg, 69 µmol) was dissolved in CH₂Cl₂ (1 mL) and *m*CPBA (23.3 mg of 77% purity, 104 µmol) added. After stirring overnight the reaction mixture was diluted with CH₂Cl₂ (5 mL) and saturated aqueous Na₂CO₃ (2 mL) added. The organic phase was separated, evaporated and the residue purified by column chromatography (IH-EtOAc 6:4 then EtOAc and finally MeOH-EtOAc 1:9) to give the title sulfoxide: RT = 3.65 min; *mlz* (ES⁺) = 451.3 [*M* + H]⁺ and the title sulfone: RT = 3.79 min; *mlz* (ES⁺) = 467.3 [*M* + H]⁺.

Oxidation using one equivalent of *m*CPBA under the above conditions afforded exclusively the sulfoxide; oxidation using two equivalents of *m*CPBA gave the corresponding sulfone as the sole product.

### Example 20: 4-[4-(4-Acetylaminophenyl)butyl]piperidine-1-carboxylic acid tert-butyl ester

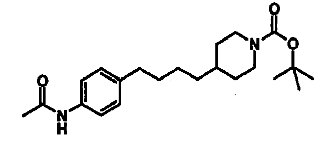

A stirred solution of 4-[4-(4-aminophenyl)butyl]piperidine-1-carboxylic acid *tert*-butyl ester (**Preparation 17**, 40 mg, 135 µmol) and NEt₃ (53 µL, 380 µmol) in dry THF (2 mL) was treated with acetyl chloride (18 µL, 250 µmol). After 18 h the mixture was diluted with EtOAc (15 mL), washed with water (4 mL), saturated aqueous NaHCO₃ (4 mL) and brine (4 mL) and dried (MgSO₄). The solvent was removed and the residue purified by column chromatography (IH-EtOAc 7:3) to give the title amide: RT = 3.97 min; *mlz* (ES⁺) = 375.2 [*M* + H]⁺*.*

The amides listed in **Table 4** were prepared by reaction of the appropriate acid chloride with either 4-[4-(4-aminophenyl)butyl]piperidine-1-carboxylic acid *tert*-butyl ester **(Preparation 17)** or 4-[3-(4-aminophenyl)propyl]piperidine-1-carboxylic acid *tert*-butyl ester **(Preparation 18)** using methods analogous to that outlined in **Example 20.**

**Table 4**

| **Eg** | **Structure** | **Name** | **RT (min)** | ***m*/*z* (ES⁺)** |
|---|---|---|---|---|
| **21** | 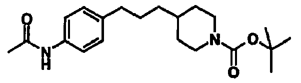 | 4-[3-(4-Acetylaminophenyl) propyl]piperidine-1-carboxylic acid *tert*-butyl ester | 3.84 | 361.2 [*M* + H]⁺ |
| **22** | 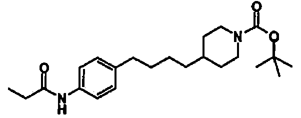 | 4-[4-(4-Propionylaminophenyl) butyl]piperidine-1-carboxylic acid *tert*-butyl ester | 4.16 | 389.2 [*M* + H]⁺ |
| **23** | 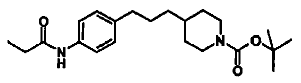 | 4-[3-(4-Propionylaminophenyl) propyl]piperidine-1-carboxylic acid *tert*-butyl ester | 3.95 | 375.2 [*M* + H]⁺ |
| **24** | 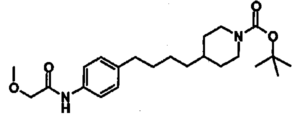 | 4-{4-[4-(2-Methoxyacetylamino) phenyl]butyl}piperidine-1-carboxylic acid *tert*-butyl ester | 4.57 | 405.3 [*M* + H]⁺ |
| **25** | 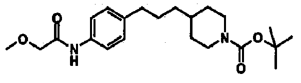 | 4-{3-[4-(2-Methoxyacetylamino) phenyl]propyl}piperidine-1-carboxylic acid *tert*-butyl ester | 4.02 | 391.2 [*M* + H]⁺ |
| **26** | 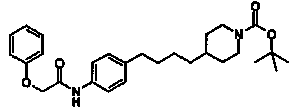 | 4-{4-[4-(2-Phenoxyacetylamino) phenyl]butyl}piperidine-1-carboxylic acid *tert*-butyl ester | 4.52 | 467.2 [*M* + H]⁺ |
| **27** | 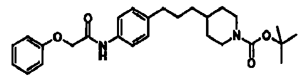 | 4-{3-[4-(2-Phenoxyacetylamino) phenyl]propyl}piperidine-1-carboxylic acid *tert*-butyl ester | 4.44 | 453.2 [*M* + H]⁺ |
| **28** | 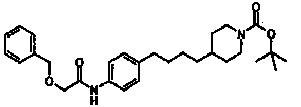 | 4-{4-[4-(2-Benzyloxyacetyl amino)phenyl]butyl}piperidine-1-carboxylic acid *tert*-butyl ester | 4.54 | 481.3 [*M* + H]⁺ |
| **29** | 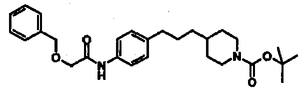 | 4-{3-[4-(2-Benzyloxyacetyl amino)phenyl]propyl}piperidine-1-carboxylic acid *tert*-butyl ester | 4.44 | 467.3 [*M* + H]⁺ |
| **30** | 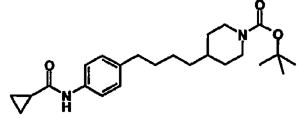 | 4-{4-[4-(Cyclopropanecarbonyl amino)phenyl]butyl}piperidine-1-carboxylic acid *tert*-butyl ester | 4.14 | 401.3 [*M* + H]⁺ |
| **31** | 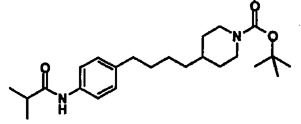 | 4-[4-(4-Isobutyrylaminophenyl) butyl]piperidine-1-carboxylic acid *tert*-butyl ester | 4.19 | 403.3 [*M* + H]⁺ |
| **32** | 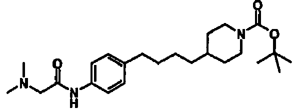 | 4-{4-[4-(2-Dimethylaminoacetyl amino)phenyl]butyl}piperidine-1-carboxylic acid *tert*-butyl ester | 3.09 | 418.3 [*M* + H]⁺ |

### Example 33: 4-{4-[4-(2-Hydroxyacetylamino)phenyl]butyl}piperidine-1-carboxylic acid tert-butyl ester

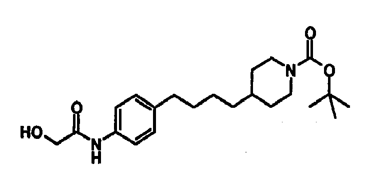

A slurry of Pd (10% on C, 10 mg,1 µmol) in EtOH (1 mL) was added to a solution of 4-{4-[4-(2-benzyloxyacetylamino)phenyl]butyl}piperidine-1-carboxylic acid *tert*-butyl ester (**Example 28,** 30 mg, 62 µmol) in EtOH (4 mL) and the mixture stirred for 18 h under an atmosphere of H₂. Filtration though a plug of celite and removal of the solvent afforded the title alcohol: RT = 3.86 min; *mlz* (ES⁺) = 391.3 [*M*+H]⁺.

### Example 34:4-{3-[4-(2-Hydroxyacetylamino)phenyl]propyl}piperidine-1-carboxylic acid tert-butyl ester

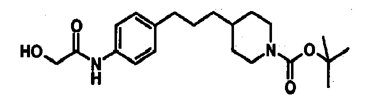

Hydrogenolysis of 4-{3-[4-(2-benzyloxyacetylamino)phenyl]propyl}piperidine-1-carboxylic acid *tert*-butyl ester (**Example 29**) using the method described in **Example 33** afforded the title compound: RT = 3.67 min; *mlz* (ES⁺) = 377.3 [M + H]⁺.

### Example 35: 4-{4-[4-(3-Ethylureido)phenyl]butyl}piperidine-1-carboxylic acid tert-butyl ester

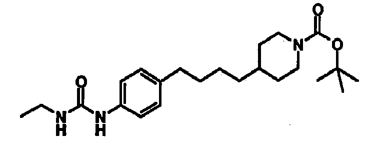

Ethyl isocyanate (26 µL, 360 µmol) was added to a solution of 4-[4-(4-aminophenyl)butyl]piperidine-1-carboxylic acid *tert*-butyl ester (**Preparation 17,** 30 mg, 90 µmol) in CH₂Cl₂ (2 mL) and the reaction stirred 18 h. The solvent was removed to afford the title compound: RT = 4.04 min; *mlz* (ES⁺)= 404.3 [*M* + H]⁺*.*

### Example 36: 4-{4-[4-(3,3-Dimethylureido)phenyl]butyl}piperidine-1-carboxylic acid tert-butyl ester

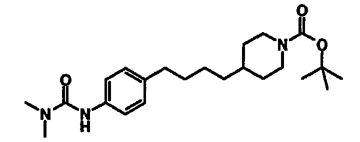

Dimethylcarbamoyl chloride (25 µL, 270 µmol) was added to a solution of 4-[4-(4-aminophenyl)butyl]piperidine-1-carboxylic acid *tert*-butyl ester (**Preparation 17,** 30 mg, 90 µmol) and NEt₃ (70 µL, 500 µmol) in dry THF (2 mL). The resulting mixture was heated at 100°C (sealed tube, microwave) for 20 min, diluted with CH₂Cl₂ (12 mL) and washed with water (2 mL), saturated aqueous NaHCO₃ (2 mL) and brine (2 mL). After filtration through a hydrophobic frit, the solvent was removed and the residue purified by RP-HPLC (CH₃CN-H₂O) to give the title compound: RT = 3.99 min; *mlz* (ES⁺) = 404.4 [*M* + H]⁺.

### Example 37: 4-(4-{4-[(Morpholine-4-carbonyl)amino]phenyl}butyl)piperidine-1-carboxylic acid tert-butyl ester

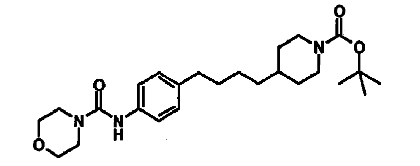

4-[4-(4-Aminophenyl)butyl]piperidine-1-carboxylic acid *tert*-butyl ester (**Preparation 17**) was reacted with 4-morpholinecarbonyl chloride using the same procedure as that described in **Example 36** to give the title urea: RT = 4.01 min; *mlz* (ES⁺) = 446.4 [*M* + H]⁺.

### Example 38: 4-[4-(4-Ethanesulfonylaminophenyl)butyl]piperidine-1-carboxylic acid tert-butyl ester

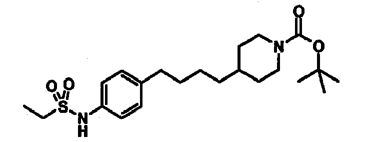

A solution of 4-[4-(4-aminophenyl)butyl]piperidine-1-carboxylic acid *tert*-butyl ester (**Preparation 17,** 40 mg, 120 µmol) and NEt₃ (100 µL, 720 µmol) in dry THF (2 mL) was treated with ethanesulfonyl chloride (47 µL, 500 µmol). After stirring for 48 h, the mixture was diluted with CH₂Cl₂ (12 mL) and washed with water (2 mL), saturated aqueous NaHCO₃ (2 mL) and brine (2 mL). After filtration through a hydrophobic frit, the solvent was removed and the residue purified by column chromatography (IH-EtOAc 4:1) to give the title compound: RT = 4.16 min; *mlz* (ES⁺) = 425.2 [*M* + H]⁺.

### Example 39: 4-[4-(4-Methanesulfonylaminophenyl)butyl]piperidine-1-carboxylic acid tert-butyl ester

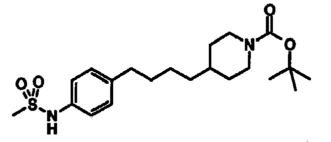

4-[4-(4-Aminophenyl)butyl]piperidine-1-carboxylic acid *tert*-butyl ester (**Preparation 17**) was reacted with methanesulfonyl chloride using the same procedure as that described in **Example 38** to give the title sulfonamide: RT = 4.11 min; *mlz* (ES⁺) = 411.2 [*M* + H]⁺.

### Example 40: 4-[(E)-4-(4-Methylsulfanylphenyl)but-3-enyl]piperidine-1-carboxylic acid tert-butyl ester

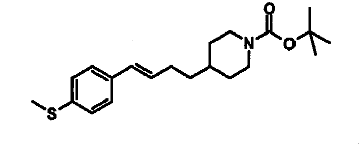

Sodium hydride (102 mg of a 60% dispersion in oil, 2.55 mmol) was suspended in anhydrous DME and a solution of 4-(3-oxopropyl)piperidine-1-carboxylic acid *tert*-butyl ester (439 mg, 1.82 mmol) in anhydrous DME (1.6 mL) added. The resulting slurry was stirred for 5 min and a solution of (4-methylsulfanylbenzyl)phosphonic acid diethyl ester in DME (1.5 mL) introduced. The reaction mixture was heated under gentle reflux for 2 h then allowed to stand overnight at rt whereupon it was poured into water (20 mL) and extracted with EtOAc (2 × 50 mL). The combined organic phases were dried (MgSO₄), the solvent removed under reduced pressure and the residue purified by column chromatography (IH-EtOAc 9:1) to afford the title compound: RT = 4.89 min; *m*/*z* (ES⁺) = 362.1 [*M* + H]⁺.

The compounds listed in **Table 5** were prepared using methods similar to those described in **Example 40.**

**Table 5**

| **Eg** | **Structure** | **Name** | **RT (min)** | ***m*/*z* (ES⁺)** |
|---|---|---|---|---|
| **41** | 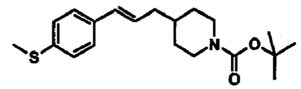 | 3-[(*E*)-3-(4-Methylsulfanyl phenyl)allyl]piperidine-1-carboxylic acid *tert-*butyl ester | 4.66 | 348.1 [*M* + H]⁺ |
| **42** | 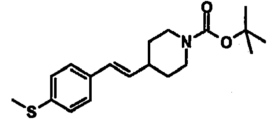 | 4-[(*E*)-2-(4-Methylsulfanyl phenyl)vinyl]piperidine-1-carboxylic acid *tert*-butyl ester | 4.55 | 334.1 [*M* + H]⁺ |

### Example 43 and 44: 4-[(E)-4-(4-Methanesulfinylphenyl)but-3-enyl]piperidine-1-carboxylic acid tert-butyl ester and 4-[(E)-4-(4-methanesulfonylphenyl)but-3-enyl]piperidine-1-carboxylic acid tert-butyl ester

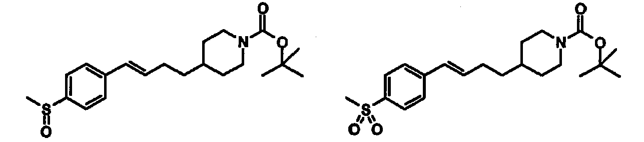

4-[(*E*)-4-(4-Methylsulfanylphenyl)but-3-enyl]piperidine-1-carboxylic acid *tert*-butyl ester (**Example 40**) was oxidised using 1 equivalent of *m*CPBA, according to the method described in **Examples 18 and 19,** to the title sulfoxide: RT = 3.82 min; *m*/*z* (ES⁺) = 378.2 [*M* + H]⁺, and, using two equivalents of *m*CPBA, to the title sulfone: RT = 3.92 min; *m*/*z* (ES⁺) = 394.1 [*M* + H]⁺.

### Examples 45 and 46: 4-[4-(4-Methanesulfinylphenyl)butyl]piperidine-1-carboxylic acid tert-butyl ester and 4-[4-(4-methanesulfonylphenyl)butyl]piperidine-1-carboxylic acid tert-butyl ester

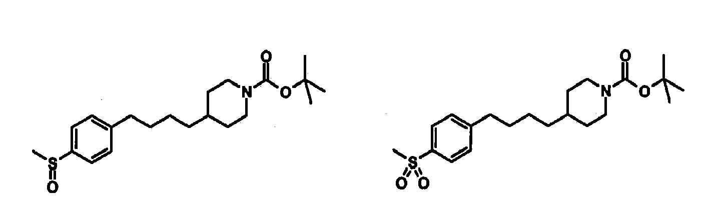

A mixture of 4-[(*E*)-4-(4-methanesulfinylphenyl)but-3-enyl]piperidine-1-carboxylic acid *tert*-butyl ester (**Example 43**) and 4-[(*E*)-4-(4-methanesulfonylphenyl)but-3-enyl]piperidine-1-carboxylic acid *tert*-butyl ester (**Example 44**) was hydrogenated over a Pd catalyst using a method similar to that described in **Example 2** to afford, after separation by column chromatography (IH-EtOAc, 7:3), the title sulfoxide: RT = 3.82 min, *mlz* (ES⁺) = 380.17 [*M* + H]⁺ and the title sulfone: RT = 4.16 min; *mlz* (ES⁺) = 396.16 [*M* + H]⁺.

The compounds listed in **Table 6** were prepared from the corresponding alkenyl sulfides by oxidation to the corresponding sulfoxides or sulfones, using methods described in **Examples 18 and 19,** followed by hydrogenation over a Pd catalyst using the method of **Example 2.**

**Table 6**

| **Eg** | **Structure** | **Name** | **RT (min)** | ***m*/*z* (ES⁺)** |
|---|---|---|---|---|
| **47** | 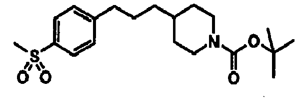 | 3-[3-(4-Methanesulfonyl phenyl)propyl]piperidine-1-carboxylic acid *tert*-butyl ester | 4.04 | 382.1 [*M* + H]⁺ |
| **48** | 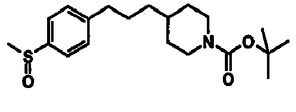 | 3-[3-(4-Methanesulfinylphenyl) propyl]piperidina-1-carboxylic acid *tert*-butyl ester | 3.65 | 366.1 [*M* + H]⁺ |
| **49** | 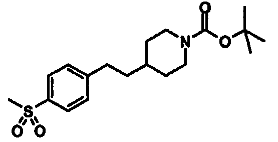 | 4-[2-(4-Methanesulfonyl phenyl)ethyl]piperidine-1-carboxylic acid *tert*-butyl ester | 3.79 | 368.1 [*M* + H]⁺ |

### Example 50: 4-[3,4-Dihydroxy-4-(4-methanesulfonylphenyl)butyl]piperidine-1-carboxylic acid tert-butyl ester

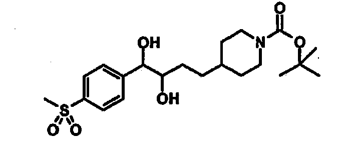

A stirred mixture of water (0.5 mL), acetone (3 mL) and *N*-methylmorpholine-*N*-oxide (31 mg, 265 µmol) was cooled to 0°C and OsO₄ (16 µL of a 2.5% w/v solution in *tert*-butanol, 13 µmol) added. A solution of 4-[4-(4-methanesulfonylphenyl)but-3-enyl]piperidine-1-carboxylic acid *tert*-butyl ester (**Example 44**, 100 mg, 250 µmol) in acetone (1.25 mL) was added dropwise and the resulting mixture stirred overnight at rt. Water (5 mL) and solid sodium thiosulfate (106 mg) were added and, after stirring vigorously for 0.5 h, the mixture was evaporated to dryness. The residue was partitioned between water (20 mL) and EtOAc (50 mL) and the aqueous phase separated and further extracted with EtOAc (3 × 20 mL). The combined organics were washed with brine (20 mL), dried (MgSO₄), evaporated and the residue purified by column chromatography (IH-EtOAc 1:1 to 0:1) to afford the title diol: RT = 3.11 min, *m*/*z* (ES⁺) = 428.2 [M+H]⁺.

### Example 51: 2-{4-[4-(4-Methanesulfonylphenyl)butyl]piperidin-1-yl}-4-trifluoromethylpyrimidine

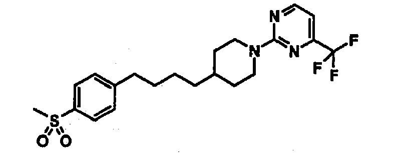

4-[4-(4-Methanesulfonylphenyl)butyl]piperidine (**Preparation 1,** 50 mg, 169µmol) was dissolved in anhydrous 1,4-dioxane (0.6 mL) and 2-chloro-4-trifluoromethylpyrimidine (37 mg, 203 µmol) and DBU (51.5 mg, 338 µmol) added. After stirring for 18 h, the solvent was removed and the residue redissolved in CH₂Cl₂ (10 mL). This solution was washed with water (10 mL), dried by passage through a hydrophobic frit and evaporated. The residue was purified by column chromatography (IH-EtOAc 1:1) to give the title compound: RT = 4.45 min, *m*/*z* (ES⁺) = 441.5 [*M* + H]⁺.

The compounds in **Table 7** were prepared by methods similar to that outlined in **Example 51.**

**Table 7**

| **Eg** | Structure | Name | RT (min) | *m*/*z* (ES⁺) |
|---|---|---|---|---|
| **52** | 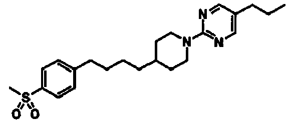 | 2-{4-[4-(4-Methanesulfonyl phenyl)butyl]piperidin-1-yl}-5-propylpyrimidine | 4.09 | 416.1 [*M* + H]⁺ |
| **53** | 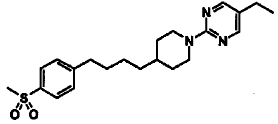 | 5-Ethyl-2-{4-[4-(4-methanesulfonylphenyl)butyl] piperidin-1-yl}pyrimidine | 3.89 | 402.1 [*M* + H]⁺ |
| **54** | 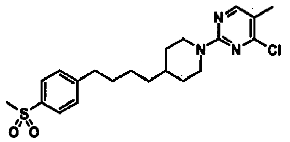 | 4-Chloro-2-{4-[4-(4-methane sulfonylphenyl)butyl]piperidin-1-yl}-5-methylpyrimidine | 4.09 | 422.1 [*M* + H]⁺ |
| 55 | 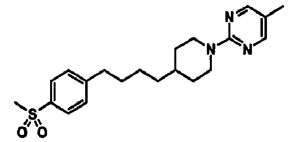 | 2-{4-[4-(4-Methanesulfonyl phenyl)butyl]piperidin-1-yl}-5-methylpyrimidine | 3.67 | 388.1 [*M* + H]⁺ |
| 56 | 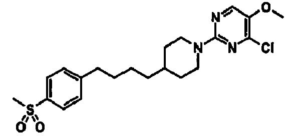 | 4-Chloro-2-{4-[4-(4-methane sulfonylphenyl)butyl]piperidin -1-yl}-5-methoxypyrimidine | 4.04 | 438.0 [*M* + *H*]⁺ |
| 57 | 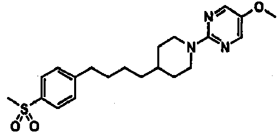 | 2-{4-[4-(4-Methanesulfonyl phenyl)butyl]piperidin-1-yl}-5-methoxypyrimidine | 3.87 | 404.1 [*M* + H]⁺ |
| 58 | 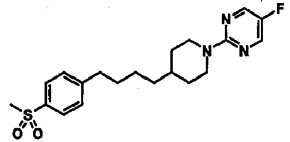 | 5-Fluoro-2-{4-[4-(4-methane sulfonylphenyl)butyl]piperidin -1-yl}pyrimidine | 4.14 | 392.1 [*M* + H]⁺ |
| 59 | 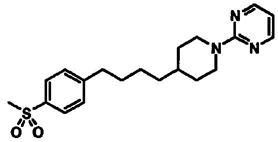 | 2-{4-[4-(4-Methanesulfonyl phenyl)butyl]piperidin-1-yl} pyrimidine | 3.67 | 374.1 [*M* + H]⁺ |

### Example 60: 4-[4-(4-Methanesulfonylphenyl)butyl]-3,4,5,6-tetrahydro-2H-[1,2']bipyridinyl

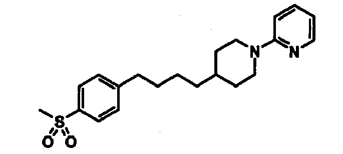

A mixture of 4-[4-(4-methanesulfonylphenyl)butyl]piperidine (**Preparation 1,** 50 mg, 169 µmol), 2-fluoropyridine (65 µL, 760 µmol) and DBU (50 µL, 340 µmol) was stirred at 80°C for 18 h. The reaction mixture was diluted with CH₂Cl₂ (10 mL) and washed with water (2 mL). The organic phase was passed through a hydrophobic frit and the solvent removed to give the title compound: RT = 2.69 min, *mlz* (ES⁺) = 441.5 [*M* + H]⁺.

### Example 61: 4-[4-(4-Methanesulfonylphenyl)butyl]piperidine-1-carboxylic acid ethyl ester

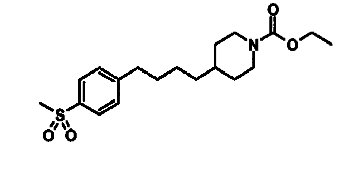

Ethylchloroformate (24.2 µL, 253 µmol) was added to a solution of 4-[4-(4-methanesulfonylphenyl)butyl]piperidine (**Preparation 1,** 50 mg, 169 µmol) and NEt₃ (118 µL, 845 µmol) in anhydrous CH₂Cl₂ (1 ml). After stirring at rt for 1.5 h, the reaction mixture was diluted with CH₂Cl₂ (5 mL) and washed with water (2 mL). The organic phase was passed through a hydrophobic frit and evaporated to afford the title compound: RT = 3.77 min; *m*/*z* (ES⁺) = 368.1 [*M* + H]⁺.

The compounds listed in Table 8 were prepared by reaction of 4-[4-(4-methanesulfonylphenyl)butyl]piperidine with the appropriate chloroformates, using the method described in **Example 61.**

**Table 8**

| **Eg** | **Structure** | **Name** | **RT (min)** | ***m*/*z* (ES)⁺** |
|---|---|---|---|---|
| **62** | 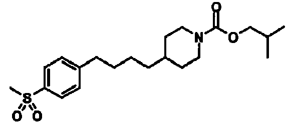 | 4-[4-(4-Methanesulfonyl phenyl)butyl]piperidine-1-carboxylic acid isobutyl ester | 4.05 | 396.2 [*M* + H]⁺ |
| **63** | 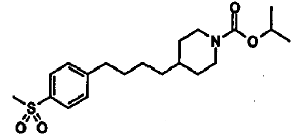 | 4-[4-(4-Methanesulfonyl phenyl)butyl]piperidine-1-carboxylic acid isopropyl ester | 3.92 | 382.2 [*M* + H]⁺ |
| **64** | 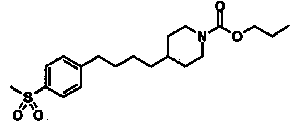 | 4-[4-(4-Methanesulfonyl phenyl)butyl]piperidine-1-carboxylic acid propyl ester | 3.87 | 382.1 [*M* + H]⁺ |
| **65** | 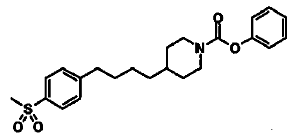 | 4-[4-(4-Methanesulfonyl phenyl)butyl]piperidine-1-carboxylic acid phenyl ester | 4.07 | 416.2 [*M* + H]⁺ |
| **66** | 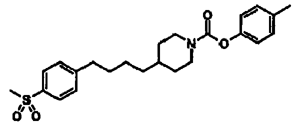 | 4-[4-(4-Methanesulfonyl phenyl)butyl]piperidine-1-carboxylic acid *p*-tolyl ester | 4.17 | 430.2 [*M* + H]⁺ |
| **67** | 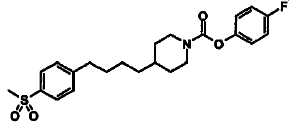 | 4-[4-(4-Methanesulfonyl phenyl)butyl]piperidine-1-carboxylic acid 4-fluorophenyl ester | 4.04 | 434.1 [*M* + H]⁺ |
| 68 | 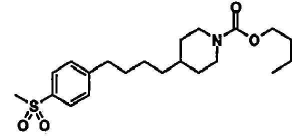 | 4-[4-(4-Methanesulfonyl phenyl)butyl]piperidine-1-carboxylic acid butyl ester | 4.16 | 396.2 [*M* + H]⁺ |
| 69 | 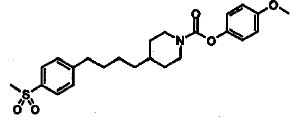 | 4-[4-(4-Methanesulfonyl phenyl)butyl]piperidine-1-carboxylic acid 4-methoxyphenyl ester | 4.01 | 446.2 [*M* + H]⁺ |

### Example 70: 4-[4-(4-Methanesulfonylphenyl)butyl]piperidine-1-carboxylic acid cyclobutyl ester

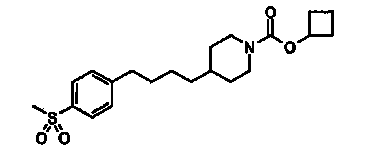

A stirred solution of cyclobutanol (48.7 mg, 676 µmol) in anhydrous THF (4 mL) was treated with a solution of triphosgene (60 mg, 202 µmol) in anhydrous THF (1 mL). After 1 h dry NEt₃ (188 µL, 1.35 mmol) was added and the cloudy mixture stirred a further 20 min before being quickly added to a solution of 4-[4-(4-methanesulfonylphenyl)butyl]piperidine (**Preparation 1,** 50 mg, 169 µmol) in anhydrous THF (2 mL). After stirring for 1 h, the reaction was quenched with water (2 mL) and diluted with CH₂Cl₂ (15 mL). The organic phase was dried by passage through a hydrophobic frit and evaporated to give a residue that was purified by RP-HPLC (CH₃CN-H₂O), giving the title compound: RT = 4.02 min; *m*/*z* (ES⁺) = 394.1 [*M* + H]⁺*.*

The compounds listed in **Table 9** were prepared by reacting the appropriate alcohols with triphosgene and subsequently 4-[4-(4-methanesulfonylphenyl)butyl]piperidine, using the protocol described in **Example 70.**

**Table 9**

| **Eg** | **Structure** | **Name** | **RT (min)** | ***m*/*z* (ES⁺)** |
|---|---|---|---|---|
| **71** | 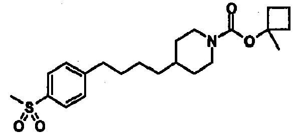 | 4-[4-(4-Methanesulfonylphenyl) butyl]piperidine-1-carboxylic acid 1-methylcyclobutyl ester | 4.12 | 408.2 [*M* + H]⁺ |
| **72** | 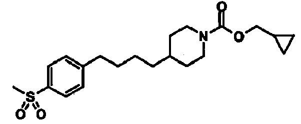 | 4-[4-(4-Methanesulfonylphenyl) butyl]piperidine-1-carboxylic acid cyclopropylmethyl ester | 3.95 | 394.1 [*M* + H]⁺ |
| 73 | 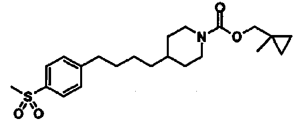 | 4-[4-(4-Methanesulfonylphenyl) butyl]piperidine-1-carboxylic acid 1-methylcyclopropylmethyl ester | 4.05 | 408.2 [*M* + H]⁺ |

### Example 74: 4-Hydroxy-4-[4-(4-methanesulfonylphenyl)butyl]piperidine-1-carboxylic acid tert-butyl ester

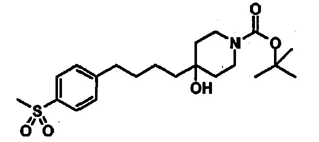

n-Butyllithium (0.938 mL of a 1.6 M solution in hexane, 1.5 mmol) was added in a dropwise fashion to solution of (4-methanesulfonylbenzyl)phosphonic acid diethyl ester (460 mg, 1.5 mmol) in anhydrous THE (3 mL). After stirring at rt for 5 min, a solution of 2-hydroxy-1-oxa-8-azaspiro[4.5]decane-8-carboxylic acid *tert*-butyl ester (**Preparation 13,** 176 mg, 683 µmol) in dry THF (2 mL) was added and the temperature raised to 65°C for 2 h. On cooling, saturated aqueous NH₄Cl (5 mL) was added and the mixture extracted with EtOAc (3 × 30 mL). The organic phase was washed with brine (20 mL), dried (MgSO₄) and evaporated, with the residue being purified by column chromatography (IH-EtOAc 9:1 to 6:1 stepwise) to give 4-hydroxy-4-[(*E*)-4-(4-methanesulfonylphenyl)but-3-enyl]piperidine-1-carboxylic acid *tert*-butyl ester: RT = 3.36 min; *m*/*z* (ES)⁺ = 410.3 [*M* + H]⁺. A sample of this olefin (211 mg, 516 µmol) was dissolved in EtOH (5 mL) and a slurry of Pd (10% on C, 50 mg, 46 µmol) in EtOH (1 mL) added and the atmosphere exchanged for H₂. The mixture was stirred for 18 h then filtered through a pad of Celite, washing through with EtOAc (5 × 5 mL). The combined filtrates were evaporated and the residue purified by column chromatography (IH-EtOAc, 1:1) to afford the title sulfone: RT = 3.36 min, *m*/*z* (ES⁺) = 412.3 [*M* + H]⁺.

### Example 75: 4-[4-(3-Chloro-4-methylsulfanylphenyl)but-3-enyl]piperidine-1-carboxylic acid tert-butyl ester

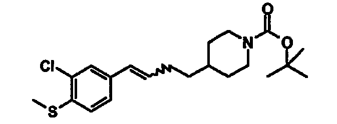

A stirred solution of 4-[3-(diethoxyphosphoryl)propyl]piperidine-1-carboxylic acid *tert-*butyl ester (Preparation 2, 584 mg, 1.61 mmol) in anhydrous THF (5 mL) was cooled to -78°C and *n*-butyllithium (1 mL of a 1.6 M solution in hexanes, 1.6 mmol) added dropwise. After 15 min a solution of 3-chloro-4-methylsulfanylbenzaldehyde (Preparation 4, 300 mg, 1.61 mmol) in dry THF (2 mL) was added *via cannula* and stirring continued for 2 h at -78°C. On quenching with saturated aqueous NH₄Cl (15 mL), the mixture was brought to rt and extracted with EtOAc (3 × 30 mL). The combined organic phases were dried (MgSO₄), evaporated and the residue purified by column chromatography (IH-EtOAc 1:9) to afford 4-[4-(3-chloro-4-methylsulfanylphenyl)-3-(diethoxyphosphoryl)-4-hydroxybutyl]piperidine-1-carboxylic acid *tert*-butyl ester: RT = 4.02 min, *m*/*z* (ES⁺) = 550.1 [*M* + H]⁺. A sample of this material (666 mg, 1.21 mmol) was dissolved in MeOH (5.5 mL) and 4 M aqueous NaOH (5.5 mL) added. The resulting vigorously-stirred mixture was heated to 60°C and sufficient THF (1.5 mL) added to give a clear, homogenous solution. After 18 h the mixture was cooled and acidified to pH 5 using 1 M aqueous HCl, and extracted with CH₂Cl₂ (3 × 25 mL). The combined organic phases were dried (MgSO₄), evaporated and the residue taken up into dry CHCl₃ (5.5 mL). Diisopropylcarbodiimide (380 µL, 2.42 mmol) was added and the solution stirred for 5 h before being evaporated and the residue purified by column chromatography (IH-EtOAc 4:1), to give the title compound: RT = 4.79 min, *m*/*z* (ES⁺) = 396.2 [*M* + H]⁺.

Using the sequence of steps described in **Example 75,** the compounds listed in **Table 10** were prepared by reaction of 4-[3-(diethoxyphosphoryl)propyl]piperidine-1-carboxylic acid *tert-*butyl ester (**Preparation 2**) and the appropriate aldehyde.

**Table 10**

| **Eg** | **Structure** | **RT Name** | **(min) (ES⁺)** | ***m*/*z*** |
|---|---|---|---|---|
| **76** | 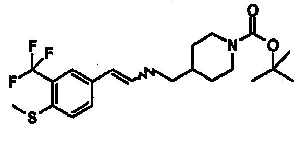 | 4-[4-(4-Methylsulfanyl-3-trifluoromethylphenyl)but-3-enyl]piperidine-1-carboxylic acid *tert*-butyl ester | 4.84 | 430.2 [*M* + H]⁺ |
| **77** | 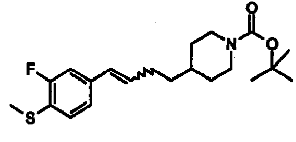 | 4-[4-(3-Fluoro-4-methylsulfanylphenyl)but-3-enyl]piperidine-1-carboxylic acid *tert*-butyl ester | 4.69 | 380.3 [*M* + H]⁺ |
| **78** | 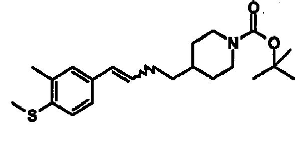 | 4-[4-(3-Methyl-4-methyl sulfanylphenyl)but-3-enyl] piperidine-1-carboxylic acid *tert-*butyl ester | 4.82 | 376.3 [*M* + H]⁺ |
| **79** | 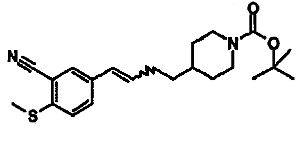 | 4-[4-(3-Cyano-4-methyl sulfanylphenyl)but-3-enyl] piperidine-1-carboxylic acid *tert*-butyl ester | 4.39 | 387.2 [*M* + H]⁺ |

### Example 80 and 81: 4-[4-(3-Chloro-4-methanesulfinylphenyl)butyl]piperidine-1-carboxylic acid tert-butyl ester and 4-[4-(3-chloro-4-methanesulfonylphenyl)butyl]piperidine-1-carboxylic acid tert-butyl ester

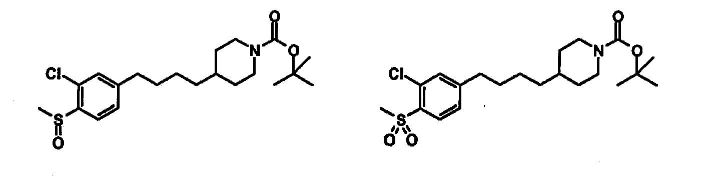

4-[4-(3-Chloro-4-methylsulfanylphenyl)but-3-enyl]piperidine-1-carboxylic acid *tert-*butyl ester (**Example 75**) was oxidised by *m*CPBA, using the procedure described in **Examples 18 and 19,** to afford a mixture of 4-[4-(3-chloro-4-methanesulfinylphenyl)but-3-enyl]piperidine-1-carboxylic acid *tert*-butyl ester: RT = 4.14 min, *m*/*z* (ES⁺) = 412.2 [*M* + H]⁺ and 4-[4-(3-chloro-4-methanesulfonylphenyl)but-3-enyl]piperidine-1-carboxylic acid *tert*-butyl ester: RT = 4.14 min, *m*/*z* (ES⁺) = 428.2 [*M* + H]⁺. This mixture of sulfoxide and sulfone was hydrogenated over a Pd catalyst using the procedure described in **Example 2** to afford, after separation by column chromatography, the title sulfoxide: RT = 4.29 min, *m*/*z* (ES⁺) = 414.2 [*M* + H]⁺ and the title sulfone: RT = 4.26 min, *m*/*z* (ES⁺) = 430.2 [*M* + H]⁺.

The sulfoxides and sulfones listed in Table 11 were prepared from the corresponding sulfides using the series of steps described in Examples 80 and 81.

**Table 11**

| **Eg** | **Structure** | **Name** | **RT (min)** | ***m*/*z* (ES⁺)** |
|---|---|---|---|---|
| **82** | 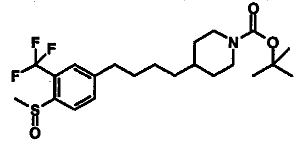 | 4-[4-(4-Methanesulfinyl-3-trifluoromethylphenyl)butyl] piperidine-1-carboxylic acid *tert*-butyl ester | 4.24 | 448.2 [*M* + H]⁺ |
| **83** | 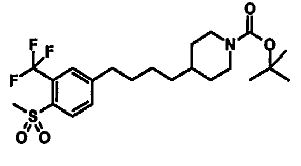 | 4-[4-(4-Methanesulfonyl-3-trifluoromethylphenyl)butyl] piperidine-1-carboxylic acid *tert*- butyl ester | 4.17 | 464.3 [*M* + H]⁺ |
| **84** | 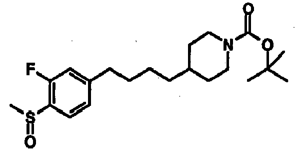 | 4-[4-(3-Fluoro-4-methane sulfinylphenyl)butyl]piperidine-1-carboxylic acid *tert*-butyl ester | 4.09 | 398.2 [*M* + H]⁺ |
| 85 | 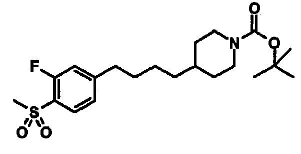 | 4-[4-(3-Fluoro-4-methane sulfonylphenyl)butyl] piperidine-1-carboxylic acid *tert*-butyl ester | 4.24 | 414.2 [*M* + H]⁺ |
| 86 | 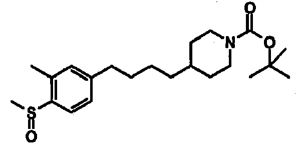 | 4-[4-(4-Methanesulfinyl-3-methylphenyl)butyl]piperidine-1-carboxylic acid *tert*-butyl ester | 3.99 | 394.3 [*M* + H]⁺ |
| 87 | 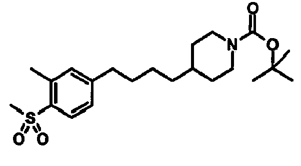 | 4-[4-(4-Methanesulfonyl-3-methylphenyl)butyl]piperidine-1-carboxylic acid *tert*-butyl ester | 4.01 | 410.3 [*M* + H]⁺ |
| 88 | 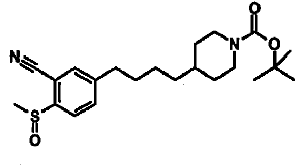 | 4-[4-(3-Cyano-4-methane sulfinylphenyl)butyl]piperidine-1-carboxylic acid *tert*-butyl ester | 3.92 | 405.3 [*M* + H]⁺ |
| 89 | 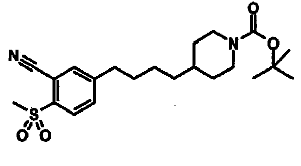 | 4-[4-(3-Cyano4-methane sulfonylphenyl)butyl]piperidine-1-carboxylic acid *tert*-butyl ester | 3.94 | 421.3 [*M* + H]⁺ |

### Example 90: 4-[4-Hydroxy-4-(4-methylsulfanylphenyl)butyl]piperidine-1-carboxylic acid tert-butyl ester

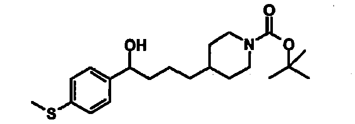

A solution of 4-methanesulfanylmagnesium bromide (0.3 mL of a 1.29 M solution in THF, 390 µmol) was added to a stirred solution of 4-(4-oxobutyl)piperidine-1-carboxylic acid *tert*-butyl ester (**Preparation 11**, 70 mg, 274 µmol) in anhydrous THF (3 mL). After 0.5 h, the reaction was quenched by the addition of 1 M aqueous HCl (0.5 mL) and diluted with ether (15 mL), washed with brine (2 mL) and dried (MgSO₄). Following removal of the solvent, the residue was purified by column chromatography (IH-EtOAc 2:1) to afford the title alcohol: RT = 4.02 min; *mlz* (ES⁺) = 380.2 [*M* + H]⁺.

### Example 91 and 92: 4-[4-Hydroxy-4-(4-methanesulfinylphenyl)butyl]piperidine-1-carboxylic acid tert-butyl ester and 4-[4-hydroxy-4-(4-methanesulfonylphenyl)butyl]piperidine-1-carboxylic acid tert-butyl ester

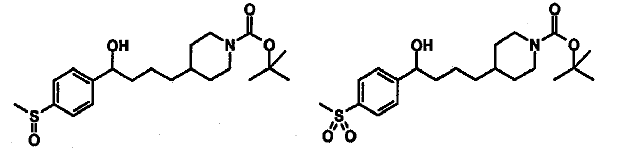

A sample of 4-[4-hydroxy-4-(4-methylsulfanylphenyl)butyl]piperidine-1-carboxylic acid *tert*-butyl ester (**Example 90**) was oxidised by mCPBA, using a similar procedure to that described in **Examples 18 and 19,** to afford the title sulfoxide: RT = 3.32 min; *mlz* (ES⁺) = 396.1 [*M* + H]⁺ and the title sulfone: RT = 3.52 min; *mlz* (ES⁺) = 412.1 [*M* + H]⁺.

### Example 93: 4-[4-(4-Methanesulfinylphenyl)-4-oxobutyl]piperidine-1-carboxylic acid tert-butyl ester

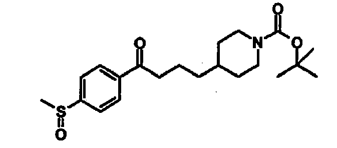

A solution of 4-[4-hydroxy-4-(4-methanesulfinylphenyl)butyl]piperidine-1-carboxylic acid *tert*-butyl ester (**Example 91,** 17 mg, 43 µmol) in CH₂Cl₂ (1.5 mL) was treated with solid Dess-Martin periodinane (36.5 mg, 86 µmol). After stirring for 1.5 h, the mixture was diluted with EtOAc (11 mL), washed with saturated aqueous Na₂CO₃ (1.5 mL) and brine (1.5 mL) then dried (MgSO₄). The sovent was removed and the residue purified by column chromatography (EtOAc) to give the title compound: RT = 3.64 min; *m*/*z* (ES⁺) = 394.2 [M + H]⁺.

### Example 94: 4-[4-(4-Methanesulfonylphenyl)-4-oxobutyl]piperidine-1-carboxylic acid tert-butyl ester

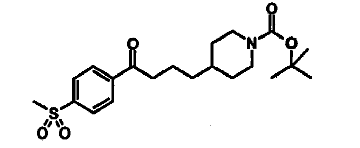

4-[4-Hydroxy-4-(4-methanesulfonylphenyl)butyl]piperidine-1-carboxylic acid *tert*-butyl ester (**Example 92**) was oxidised, using a similar method to that of **Example 93,** to afford the title compound: RT = 3.79 min; *m*/*z* (ES⁺) = 410.1 [*M* + H]⁺*.*

### Example 95: 4-[4-(4-Fluoromethanesulfinylphenyl)-4-hydroxybutyl]piperidine-1-carboxylic acid tert-butyl ester

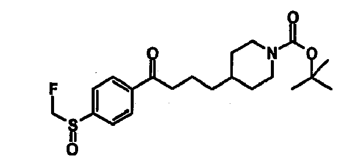

Neat (diethylamino)sulphur trifluoride (DAST) (23 µL, 178 µmol) was added to a solution of 4-[4-(4-methanesulfinylphenyl)-4-oxobutyl]piperidine-1-carboxylic acid *tert*-butyl ester (**Example 93,** 35 mg, 89 µmol) in dry CH₂Cl₂ (0.5 mL) under argon and the mixture stirred for 6 h at rt. Further DAST (23 µL, 178 µmol) was added and the mixture heated at 35°C for a further 18 h. CH₂Cl₂ (2 mL) and water (0.5 mL) were added and the mixture diluted with EtOAc (20 mL). After washing with water (2 mL), brine (2 mL) and drying (MgSO₄), the solvent was removed and the residue purified by column chromatography (IH-EtOAc 4:1) to afford 4-[4-(4-fluoromethylsulfanylphenyl)-4-oxobutyl]piperidine-1-carboxylic acid tert-butyl ester: RT = 4.20 min; *m*/*z* (ES⁺) = 396.2 [*M* + H]⁺. A sample of this sulfide (28 mg, 70.8 µmol) was dissolved in CH₂Cl₂ (2 mL) and *m*CPBA (15.8 mg of 77% purity, 71µmol) in CH₂Cl₂ (1 mL) added. After stirring overnight, the solvent was evaporated and the residue dissolved in EtOAc (20 mL), which was washed with saturated aqueous Na₂CO₃ (2 × 3 mL), water (3 mL) and brine (3 mL). After drying (MgSO₄) the solvent was evaporated and the residue purified by column chromatography (Et₂O then EtOAc) to afford the title compound: RT = 3.69 min; *m*/*z* (ES⁺) = 412.2 [*M* + H]⁺.

### Example 96: 4-[4,4-Difluoro-4-(4-methanesulfonylphenyl)butyl]piperidine-1-carboxylic acid tert-butyl ester

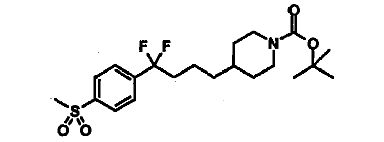

A sample of 4-[4-(4-methanesulfonylphenyl)-4-oxobutyl]piperidine-1-carboxylic acid *tert*-butyl ester (**Example 94,** 27 mg, 66 µmol) was weighed into a small flask, which was flushed with nitrogen, and neat (diethylamino)sulphur trifluoride (DAST) (200 µL, 1.5 mmol) introduced. The resulting solution was stirred at rt for 60 h then quenched by the careful addition of saturated aqueous Na₂CO₃ (2 mL). After extraction into EtOAc (15 mL), the organic phase was washed with brine (2 mL), dried (MgSO₄) and evaporated. The was residue purified by preparative tlc (IH-EtOAc 2:1) to give the title compound: RT = 4.01 min; *mlz* (ES⁺) = 432.3 [M + H]⁺.

### Example 97: 4-[4-(4-Methylsulfanylphenyl)butyryl]piperidine-1-carboxylic acid tert-butyl ester

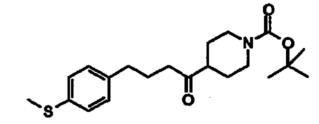

Sodium metal (127 mg, 5.52 mmol) was dissolved in dry EtOH (15 mL) and the resulting stirred solution cooled to 0°C. 4-(2-Ethoxycarbonylacetyl)piperidin-1-carboxylic acid *tert*-butyl ester (1.65 g, 5.52 mmol) was added, the mixture warmed to rt for 10 min, and a dispersion of 1-(2-bromoethyl)-4-methylsulfanylbenzene (1.5 g, 5.52 mmol) in EtOH (2 mL) added. The reaction was heated under reflux for 85 h, cooled and the ethanol removed in vacuo. The residue was dissolved in EtOAc (100 mL), washed with water (25 mL) and brine (25 mL). After drying (MgSO₄) the solvent was removed and the residue purified by column chromatography (IH-EtOAc 9:1 then 7:1) to afford 4-[2-ethoxycarbonyl-4-(4-methylsulfanylphenyl)butyryl]piperidine-1-carboxylic acid *tert*-butyl ester: RT = 4.20 min; *m*/*z* (ES⁺) = 450.2 [*M* + H]⁺. A sample of this ester (897 mg, 2.0 mmol) was dissolved in MeOH (16 mL) and a solution of KOH (224 mg, 4 mmol) in water (6.5 mL) added. After heating under reflux for 2 h, the MeOH was removed and the aqueous phase extracted with EtOAc (50 mL) and dried (MgSO₄). Removal of the solvent afford the title thioether: RT = 4.14 min; *mlz* (ES⁺) = 378.3 [*M* + H]⁺.

### Example 98: 4-[4-(4-Methylsulfanylphenyl)-2-oxobutyl]piperidine-1-carboxylic acid tert-butyl ester

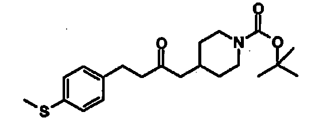

Using the methods described in **Example 97,** 1-bromomethyl-4-methylthiobenzene was reacted with 4-(3-ethoxycarbonyl-2-oxopropyl)piperidine-1-carboxylic acid *tert*-butyl ester (**Preparation 14**) to afford, 4-[3-ethoxycarbonyl-4-(4-methylsulfanylphenyl)-2-oxobutyl]piperidine-1-carboxylic acid *tert*-butyl ester: RT = 4.20 min; *mlz* (ES⁺) = 450.2 [*M* + H]⁺. This material was hydrolysed and decarboxylated to give the title compound: RT = 4.14 min; *mlz* (ES⁺) = 378.2 *[M+* H]⁺.

### Example 99: 4-[4-(3-Fluoro-4-methylsulfanylphenyl)butyryl]piperidine-1-carboxylic acid tert-butyl ester

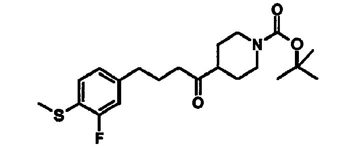

Using the methods described in **Example 97,** 4-(2-bromoethyl)-2-fluoro-1-methylsulfanylbenzene (Preparation 24) was reacted with 4-(2-ethoxycarbonylacetyl)-piperidine-1-carboxylic acid *tert*-bulyl ester, and the product hydrolysed and decarboxylated to give the title compound: RT = 4.24 min; *mlz* (ES⁺) = 396.1 *[M+* H]⁺.

### Example 100: 4-[1-Hydroxy-4-(4-methylsulfanylphenyl)butyl]piperidine-1-carboxylic acid tert-butyl ester

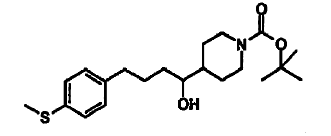

A solution of 4-[4-(4-methylsulfanylphenyl)butyryl]piperidine-1-carboxylic acid *tert-*butyl ester (Example 97, 370 mg, 981 µmol) in EtOH (7 mL) was treated with sodium borohydride (56 mg, 1.47 mmol). After stirring for 2 h, the solvent was removed and the residue taken up in EtOAc (40 mL), washed with water (5 mL) and brine (5 mL) then dried (MgSO₄), and evaporated to give the title alcohol: RT = 3.97 min; *mlz* (ES⁺) = 380.3 [*M* + H]⁺.

### Example 101: 4-[2-Hydroxy-4-(4-methylsulfanylphenyl)butyl]piperidine-1-carboxylic acid tert-butyl ester

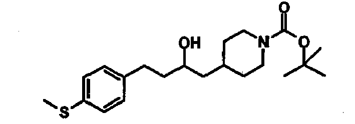

4-[4-(4-Methylsulfanylphenyl)-2-oxobutyl]piperidine-1-carboxylic acid *tert*-butyl ester (**Example 98**) was reduced using sodium borohydride in a manner analogous to that described in **Example 100** to give the title alcohol: RT = 3.99 min; *m*/*z* (ES⁺) = 380.2 [*M* + H]⁺.

### Example 102: 4-[4-(3-Fluoro-4-methylsulfanylphenyl)-1-hydroxybutyl]piperidine-1-carboxylic acid tert-butyl ester

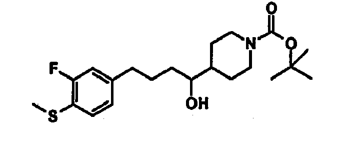

4-[4-(3-Fluoro-4-methylsulfanylphenyl)butyryl]piperidine-1-carboxylic acid *tert*-butyl ester (**Example 99**) was reduced using sodium borohydride, in a manner analogous to that described in **Example 100,** to give the title alcohol: RT = 4.19 min; *mlz* (ES⁺) = 398.1 *[M+* H]⁺.

### Example 103: 4-[4-(3-Fluoro-4-methylsulfanylphenyl)-1-methoxybutyl]piperidine-1-carboxylic acid tert-butyl ester

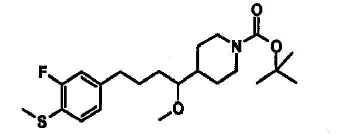

A stirred mixture of NaH (40.6 mg of a 60% dispersion in mineral oil, 1.0 mmol), 4-[4-(3-fluoro-4-methylsulfanylphenyl)-1-hydroxybutyl]piperidine-1-carboxylic acid *tert*-butyl ester (Example 102, 130 mg, 327 µmol), and MeI (0.15 mL, 2.4 mmol) in anhydrous THF (3 mL) was heated at 90°C under microwave irradiation for 2 h, and then at 100°C under microwave irradiation for 1.5 h. The reaction was purified by column chromatography to furnish the title compound: RT = 4.51 min; *mlz* (ES⁺) = 412.1 [*M* + H]⁺*.*

The sulfoxides and sulfones listed in Table 12 were prepared by oxidising the corresponding sulfides with *m*CPBA using the method described in **Examples 18 and 19.**

**Table 12**

| **Eg** | **Structure** | **Name** | **RT (min)** | ***m*/*z* (ES⁺)** |
|---|---|---|---|---|
| **104** | 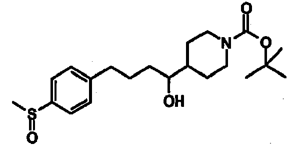 | 4-[1-Hydroxy-4-(4-methane sulfinylphenyl)butyl]piperidine-1-carboxylic acid *tert-*butyl ester | 3.24 | 396.2 [*M* + H]⁺ |
| **105** | 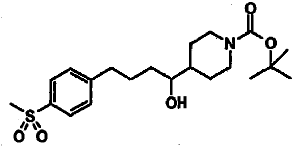 | 4-[1-Hydroxy-4-(4-methane sulfonylphenyl)butyl]piperidine-1-carboxylic acid *tert*-butyl ester | 3.40 | 412.3 [*M* + H]⁺ |
| **106** | 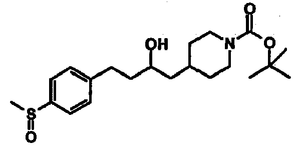 | 4-[2-Hydroxy-4-(4-methane sulfmylphenyl)butyl]piperidine-1-carboxylic acid *tert*-butyl ester | 3.24 | 396.3 [*M* + H]⁺ |
| **107** | 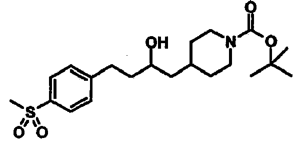 | 4-[2-Hydroxy-4-(4-methane sulfonylphenyl)butyl]piperidine-1-carboxylic acid *tert*-butyl ester | 3.39 | 412.3 [*M* + H]⁺ |
| **108** | 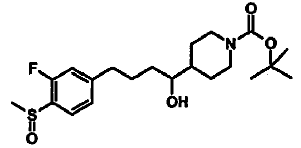 | 4-[4-(3-Fluoro-4-methanesufinylphenyl)-1-hydroxybutyl]piperidine-1-carboxylic acid *tert*-butyl ester | 3.39 | 414.1 [*M* + H]⁺ |
| **109** | 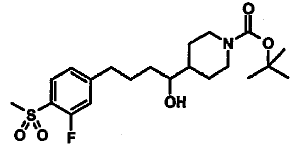 | 4-[4-(3-Fluoro-4-methanesulfonylphenyl)-1-hydroxybutyl]piperidine-1-carboxylic acid *tert*-butyl ester | 3.57 | 430.1 [*M* + H]⁺ |
| **110** | 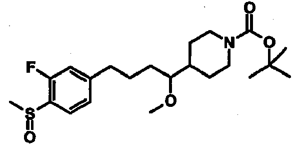 | 4-[4-(3-Fluoro-4-methanesulfinylphenyl)-1-methoxybutyl]piperidine-1-carboxylic acid *tert*-butyl ester | 3.74 | 428.0 [*M* + H]⁺ |
| **111** | 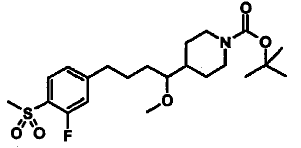 | 4-[4-(3-Fluoro-4-methanesufonylphenyl)-1-methoxybutyl]piperidine-1-carboxylic acid *tert*-butyl ester | 390 | 444.0 [*M* + H]⁺ |

The compounds listed in **Table 13** were produced by oxidation of the corresponding alcohol by Dess-Martin periodinane, according to the procedure outlined in **Example 93.**

**Table 13**

| **Eg** | **Structure** | **Name** | **RT (min)** | ***m*/*z* (ES⁺)** |
|---|---|---|---|---|
| **112** | 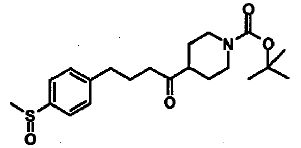 | 4-[4-(4-Methanesulfinylphenyl) butyryl]piperidine-1-carboxylic acid *tert*-butyl ester | 3.39 | 394.3 [*M* + H]⁺ |
| **113** | 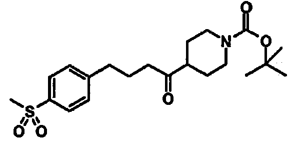 | 4-[4-(4-Methanesulfonylphenyl) butyryl]piperidine-1-carboxylic acid *tert*-butyl ester | 3.59 | 410.2 [*M* + H]⁺ |
| **114** | 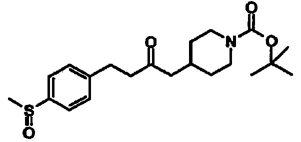 | 4-[4-(4-Methanesulfinylphenyl)-2-oxo-butyl]piperidine-1- ester carboxylic acid *tert*-butyl ester | 3.26 | 394.2 [*M* + H]⁺ |
| **115** | 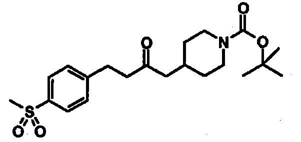 | 4-[4-(4-Methanesulfonylphenyl)-2-oxo-butyl]piperidine-1- ester carboxylic acid *tert*-butyl ester | 3.56 | 410.3 [*M* + H]⁺ |
| **116** | 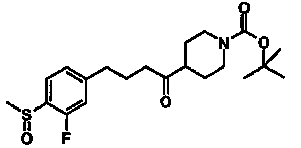 | 4-[4-(3-Fluoro-4-methanesulfinylphenyl)butyryl]-piperidine-1-carboxylic acid *tert-*butyl ester | 3.52 | 412.1 [*M +* H]⁺ |
| **117** | 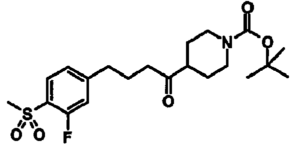 | 4-[4-(3-Fluoro-4-methanesulfonylphenyl)butyryl]-piperidine-1-carboxylic acid *tert-* butyl ester | 3.74 | 428.1 [*M* + H]⁺ |

### Example 118: 4-[3-(4-Cyanophenyl)propyl]piperidine-1-carboxylic acid tert-butyl ester

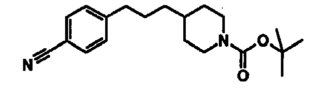

A solution of (4-cyanobenzyl)triphenylphosphonium chloride (201 mg, 480 µmol) in anhydrous THE (5 mL) was cooled to 0°C and lithium bis(trimethylsilyl)amide (530 µL of a 1.0 M solution in THF, 530 µmol) added dropwise. After stirring for 45 min, solid 4-(2-oxoethyl)piperidine-1-carboxylic acid *tert*-butyl ester (100 mg, 440 µmol) was added in one portion and the temperature raised to ambient. After 18 h the reaction was diluted with EtOAc (30 mL), washed with water (5 mL), brine (5 mL) and dried (MgSO₄). The solvent was evaporated under reduced pressure and the residue purified by column chromatography to afford a mixture of (*E*)- and (*Z*)- 4-[3-(4-cyanophenyl)allyl]piperidine-1-carboxylic acid *tert*-butyl esters. This mixture was hydrogenated over a Pd catalyst using the method outlined in **Example 2** to afford the title compound: RT = 4.89 min; δ_{H} (CD₃OD) 1.00 (dq, 2H), 1.25 (m, 2H), 1.44 (s, 9H), 1.39-1.48 (m, 1H), 1.57-1.66 (m, 4H), 2.53 (t, 2H), 2.69 (t, 2H), 4.01 (d, 2H), 7.02 (d, 2H), 7.05 (d, 2H).

### Example 119: 4-[4-(4-Cyanophenyl)butyl]piperidine-1-carboxylic acid tert-butyl ester

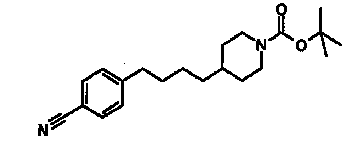

(4-Cyanobenzyl)triphenylphosphonium chloride was reacted with 4-(3-oxopropyl)piperidine-1-carboxylic acid tert-butyl ester using a similar procedure to that described in **Example 118** to afford the title compound: RT = 4.97 min; δ_{H} (CDCl₃) 1.07 (dq, 2H), 1.23-1.30 (m, 2H), 1.31-1.40 (m, 3H), 1.47 (s, 9H), 1.56-1.66 (m, 4H), 2.58 (t, 2H), 2.65 (t, 2H), 4.06 (br s, 2H), 7.07 (d, 2H), 7.10 (d, 2H).

### Example 120: 4-{3-[(4-Methylsulfanylphenyl)-(2-nitrobenzenesulfonyl)amino]propyl} piperidine-1-carboxylic acid tert-butyl ester

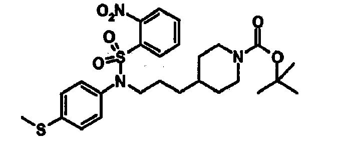

A mixture of *N*-(4-methylsulfanylphenyl)-2-nitrobenzenesulfonamide (**Preparation 8**, 0.6 g, 1.85 mmol), 4-(3-hydroxypropyl)piperidine-1-carboxylic acid *tert*-butyl ester (672 mg, 2.76 mmol), triphenylphosphine (724 mg, 2.76 mmol) and di-*tert*-butylazodicarboxylate (636 mg, 2.76 mmol) under argon was dissolved in anhydrous toluene (10 mL). After stirring at rt for 48 h, the solvent was removed and the residue purified by column chromatography (IH-EtOAc, 2:1 then 1:1) to afford the title sulfonamide: RT = 4.51 min; *m*/*z* (ES⁺) = 550.2 [*M* + H]⁺.

The compounds listed in **Table 14** were prepared using a method similar to that described in **Example 120.**

**Table 14**

| **Eg** | **Structure** | **Name** | **RT (min)** | ***m*/*z* (ES⁺)** |
|---|---|---|---|---|
| **121** | 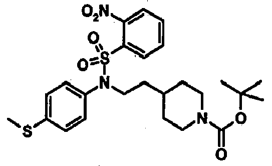 | 4-{2-[(4-Methylsulfanylphenyl)-(2-nitrobenzenesulfonyl)amino] ethyl}piperidine-1-carboxylic acid *tert*-butyl ester | 4.41 | 536.1 [*M* + H]⁺ |
| **122** | 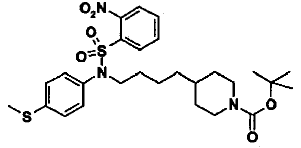 | 4-{4-[(4-Methylsulfanylphenyl)-(2-nitrobenzenesulfonyl)amino] butyl}piperidine-1-carboxylic acid *tert*-butyl ester | 4.55 | 564.1 [*M* + H]⁺ |

### Example 123: 4-[3-(4-Methylsulfanylphenylamino)propyl]piperidine-1-carboxylic acid tert-butyl ester

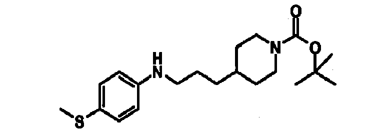

Solid cesium carbonate (261 mg, 800 µmol) was added to a stirred solution of 4-{3-[(4-methylsulfanylphenyl)-(2-nitrobenzenesulfonyl)amino]propyl}piperidine-1-carboxylic acid *tert-*butyl ester **(Example 120,** 219 mg, 400 µmol) and thiophenol (88 mg, 800 µmol) in CH₃CN (1.0 mL). After 1 h the mixture was diluted with ether (50 mL), washed with water (2 × 5 mL) and brine (5 mL) then dried (MgSO₄) The solvent was removed and the residue purified by column chromatography (IH-EtOAc 2:1) to afford the title compound: RT = 4.01 min; *m*/*z* (ES⁺) = 365.2 [*M* + H]⁺.

### Example 124: 4-[2-(4-Methylsulfanylphenylamino)ethyl]piperidine-1-carboxylic acid tert-butyl ester

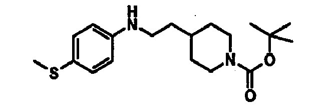

4-{2-[(4-Methylsulfanylphenyl)-(2-nitrobenzenesulfonyl)amino]ethyl}piperidine-1-carboxylic acid *tert*-butyl ester was treated with cesium carbonate and thiophenol in the same way as described in **Example 123** to afford the title compound: RT = 3.74 min; *m*/*z* (ES⁺) = 351.2 [*M* + H]⁺.

### Example 125: 4-[3-(3-Fluoro-4-methylsulfanylphenylamino)propyl]piperidine-1-carboxylic acid tert-butyl ester

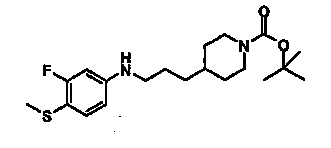

Powdered 4A molecular sieves (100 mg) were suspended in a solution of 4-(3-oxopropyl)piperidine-1-carboxylic acid *tert*-butyl ester (50 mg, 210 µmol) in anhydrous CH₂Cl₂ (3.5 mL) and 3-fluoro-4-methylsulfanylphenylamine (33 mg, 210 µmol) added. After stirring for 20 min, sodium triacetoxyborohydride (58 mg, 270 µmol) was added and stirring continued for 24 h. The reaction mixture was partitioned between saturated aqueous NaHCO₃ (15 mL) and Et₂O (25 mL) and the organic phase separated, washed with brine (5 mL), dried (MgSO₄) and evaporated. The residue was purified by column chromatography (IH-EtOAc 3:1) to afford the title compound: RT = 4.36 min; *m*/*z* (ES⁺) = 383.3 [*M* + H]⁺.

The sulfoxides and sulfones listed in **Table 15** were synthesised by oxidising the corresponding sulfides with *m*CPBA, using the procedure described in **Examples 18 and 19.**

**Table 15**

| **Eg** | **Structure** | **Name** | **RT (min)** | ***m*/*z* (ES⁺)** |
|---|---|---|---|---|
| **126** | 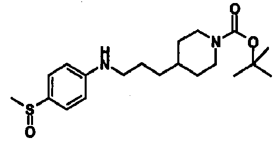 | 4-[3-(4-Methanesulfinylphenyl amino)propyl]piperidine-1-carboxylic acid *tert*-butyl ester | 3.56 | 381.2 [*M* + H]⁺ |
| **127** | 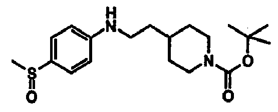 | 4-[2-(4-Methanesulfinylphenyl amino)ethyl]piperidine-1-carboxylic acid *tert*-butyl ester | 3.37 | 367.2 [*M* + H]⁺ |
| **128** | 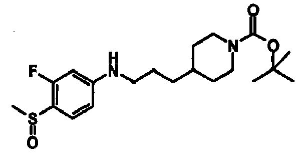 | 4-[3-(3-Fluoro-4-methane sulfinylphenylamino)propyl] piperidine-1-carboxylic acid *tert*-butyl ester | 3.65 | 399.2 [*M* + H]⁺ |
| **129** | 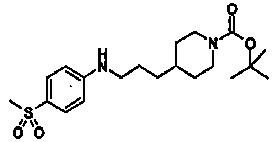 | 4-[3-(4-Methanesulfonylphenyl amino)propyl]piperidine-1-carboxylic acid *tert*-butyl ester | 3.76 | 397.2 [*M* + H]⁺ |
| **130** | 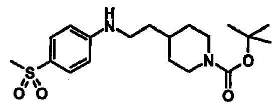 | 4-[2-(4-Methanesulfonylphenyl amino)ethyl]piperidine-1-carboxylic acid *tert*-butyl ester | 3.61 | 383.2 [*M* + H]⁺ |
| **131** | 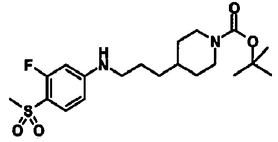 | 4-[3-(3-Fluoro-4-methane sulfonylphenylamino)propyl] piperidine-1-carboxylic acid *tert*-butyl ester | 3.77 | 415.3 [*M* + H]⁺ |
| **132** | 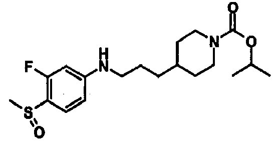 | 4-[3-(3-Fluoro-4-methanesulfinylphenylamino)-propyl]piperidine-1-carboxylic acid isopropyl ester | 3.49 | 385.1 [*M* + H]⁺ |
| **133** | 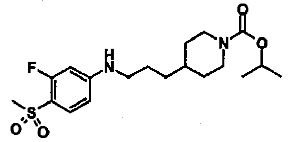 | 4-[3-(3-Fluoro-4-methanesulfonylphenylamino)-propyl]piperidine-1-carboxylic acid isopropyl ester | 3.51 | 401.0 [*M* + H]⁺ |

### Example 134: 4-{3-[Methyl(4-methylsulfanylphenyl)amino]propyl}piperidine-1-carboxylic acid tert-butyl ester

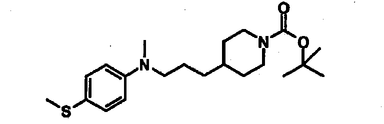

A solution of 4-[3-(4-methylsulfanylphenylamino)propyl]piperidine-1-carboxylic acid *tert*-butyl ester (**Example 123,** 90 mg, 247 µmol) in THF (2.5 mL) was added dropwise over 4 min to a stirred, ice-cooled solution of 10% aqueous H₂SO₄ (0.5 mL) and formaldehyde (241 µL of a 37 wt% solution in water, 2.96 mmol) in THF (1.25 mL). Solid sodium borohydride (65.5 mg, 1.73 mmol) was added in 10 mg portions over 4-5 min and the reaction warmed to rt. After stirring for 15 min, the mixture was poured into 0.2 M aqueous NaOH (20 mL) and extracted with EtOAc (2 × 25 mL). The combined organic phases were washed with brine (10 mL), dried (MgSO₄) and evaporated. The residue was filtered through a small plug of silica (IH-EtOAc 4:1) to afford the title compound: RT = 3.90 min; *m*/*z* (ES⁺) = 379.2 [*M* + H]⁺.

The compounds in **Table 16** were synthesised using the method described in **Example 134.**

**Table 16**

| **Eg** | **Structure** | **Name** | **RT (min)** | ***m*/*z* (ES⁺)** |
|---|---|---|---|---|
| **135** | 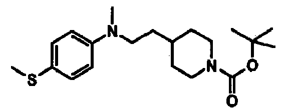 | 4-{2-[Methyl(4-methylsulfanyl phenyl)amino]ethyl}piperidine-1-carboxylic acid *tert*-butyl ester | 4.14 | 365.2 [*M* + H]⁺ |
| **136** | 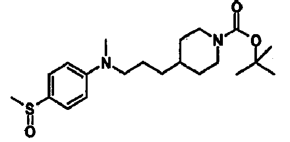 | 4-{3-[(4-Methanesulfinyl phenyl)methylamino]propyl} piperidin-1-carboxylic acid *tert*-butyl ester | 3.70 | 395.2 [*M* + H]⁺ |
| **137** | 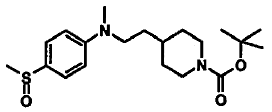 | 4-{2-[(4-Methanesulfinyl phenyl)methylamino]ethyl} piperidine-1-carboxylic acid *tert*-butyl ester | 3.57 | 381.2 [*M* + H]⁺ |
| **138** | 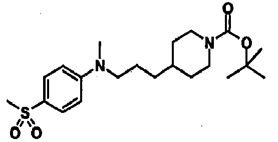 | 4-{3-[(4-Methanesulfonyl phenyl)methylamino]propyl}) piperidine-1-carboxylic acid *tert*-butyl ester | 3.92 | 411.2 [*M* + H]⁺ |
| **139** | 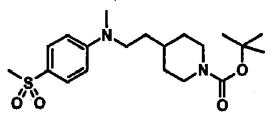 | 4-{2-[(4-Methanesulfonyl phenyl)methylamino]ethyl} piperidine-1-carboxylic acid *tert*-butyl ester | 3.72 | 397.1 [*M* + H]⁺ |

### Example 140 and 141: 4-[4-(4-Methanesulfinylphenylamino)butyl]piperidine-1-carboxylic acid tert-butyl ester and 4-[4-(4-methanesulfonylphenylamino)butyl]pipefidine-1-carboxylic acid tert-butyl ester

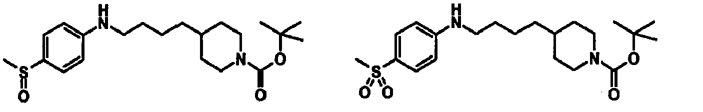

Using the method described in **Examples 18 and 19,** 4-{4-[(4-methylsulfanylphenyl)-(2-nitrobenzenesulfonyl)amino]butyl}piperidine-1-carboxylic acid *tert*-butyl ester **(Example 122)** was oxidised to 4-{4-[(4-methanesulfinylphenyl)-(2-nitrobenzenesulfonyl)amino]butyl}-piperidine-1-carboxylic acid *tert*-butyl ester: RT = 3.97 min; *mlz* (ES⁺) = 580.2 [*M* + H]⁺ and to 4-{4-[(4-methanesulfonylphenyl)-(2-nitrobenzenesulfonyl)amino]butyl}piperidine-1-carboxylic acid *tert*-butyl ester: RT = 4.07 min; *m*/*z* (ES⁺) = 596.1 [*M* + H]⁺.

In each case the resulting sulfoxide or sulfone was treated with cesium carbonate and thiophenol in CH₃CN, in the manner described in **Example 123**, to afford either the title sulfoxide: RT = 3.77 min; *m*/*z* (ES⁺) = 395.2 [*M* + H]⁺ or the title sulfone RT = 3.87 min; *m*/*z* (ES⁺) = 411.2 [*M* + H]⁺.

### Example 142: 4-[3-(4-Methylsulfanylphenoxy)propyl]piperidine-1-carboxylic acid tert-butyl ester

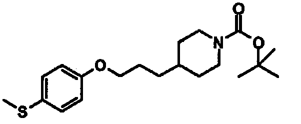

A suspension of sodium hydride (231 mg of a 60% dispersion in oil, 5.8 mmol) in anhydrous THF (4 mL) was cooled to 0°C and a solution of 4-(methylthio)phenol (771 mg, 5.5 mmol) in THF (2 mL) added in a dropwise fashion. After stirring for 0.5 h, a solution of 4-(3-methanesulfonyloxypropyl)piperidine-1-carboxylic acid *tert*-butyl ester (**Preparation 9**, 322 mg, 1 mmol) in anhydrous THF (3 mL) was introduced *via cannula* and the resulting colourless solution heated at 65°C for 16 h. After cooling, the mixture was diluted with ether (100 mL) and washed sequentially with 2 M aqueous NaOH (10 mL) water (10 mL), 2 M aqueous NaOH (10 mL) and brine (10 mL). The solvent was removed and the residue purified by column chromatography (IH-EtOAc 4:1) to afford the title compound: RT = 4.57 min; *m*/*z* (ES⁺) = 366.2 [*M* + H]⁺.

The compounds listed in **Table 17** were synthesised from the appropriate 4-(methylsulfanyl)phenol and the corresponding mesylate, using a similar procedure to that described in **Example 142.**

**Table 17**

| **Eg** | **Structure** | **Name** | **RT (min)** | ***m*/*z* (ES⁺)** |
|---|---|---|---|---|
| **143** | 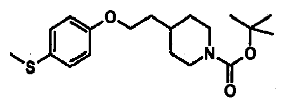 | 4-[2-(4-Methylsulfanylphenoxy) ethyl]piperidine-1-carboxylic acid *tert*-butyl ester | 4.36 | 352.2 [*M* + H]⁺ |
| **144** | 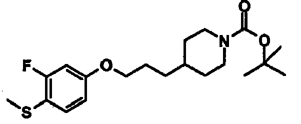 | 4-[3-(3-Fluoro-4-methylsulfanyl phenoxy)propyl]piperidine-1-carboxylic acid *tert*-butyl ester | 4.84 | 384.2 [*M* + H]⁺ |
| **145** | 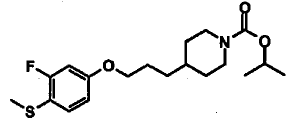 | 4-[3-(3-Fluoro-4-methylsulfanyl-phenoxy)propyl]piperidine-1-carboxylic acid isopropyl ester | 4.42 | 370.0 [*M* + H]⁺ |

The compounds in **Table 18** were produced by oxidation of the corresponding sulfide either to the sulfoxide or the sulfone using the method of **Examples 18 and 19.**

**Table 18**

| **Eg** | **Structure** | **Name** | **RT (min)** | ***m*/*z* (ES⁺)** |
|---|---|---|---|---|
| **146** | 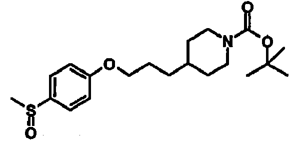 | 4-[3-(4-Methanesulfinylphenoxy) propyl]piperidine-1-carboxylic acid *tert*-butyl ester | 3.77 | 382.1 [*M* + H]⁺ |
| **147** | 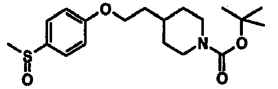 | 4-[2-(4-Methanesulfinylphenoxy) ethyl]piperidine-1-carboxylic acid *tert*-butyl ester | 3.57 | 368.1 [*M* + H]⁺ |
| **148** | 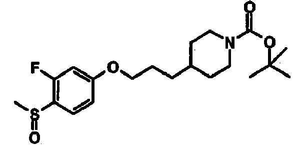 | 4-[3-(3-Fluoro-4-methanesulfinyl phenoxy)propyl]piperidine-1-carboxylic acid *tert*-butyl ester | 3.98 | 400.2 [*M* + H]⁺ |
| **149** | 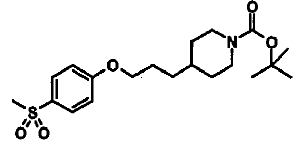 | 4-[3-(4-Methanesulfonyl phenoxy)propyl]piperidine-1-carboxylic acid *tert*-butyl ester | 3.89 | 398.1 [*M* + H]⁺ |
| **150** | 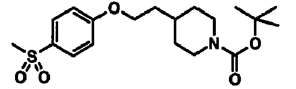 | 4-[2-(4-Methanesulfonyl phenoxy)ethyl]piperidine-1-carboxylic acid *tert*-butyl ester | 3.72 | 384.1 [*M* + H]⁺ |
| **151** | 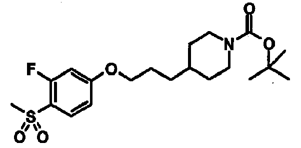 | 4-[3-(3-Fluoro-4-methane sulfonylphenoxy)propyl] piperidine-1-carboxylic acid *tert-* butyl ester | 3.94 | 416.2 [*M* + H]⁺ |
| **152** | 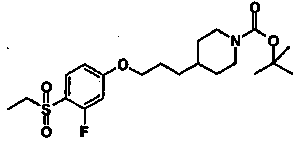 | 4-[3-(4-Ethanesulfonyl-3-fluoro-phenoxy)propyl]piperidine-1-carboxylic acid *tert*-butyl ester | 4.01 | 430.1 [*M* + H]⁺ |
| **153** | 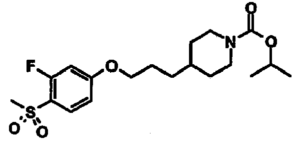 | 4-[3-(3-Fluoro-4-methanesulfonylphenoxy)-propyl]piperidine-1-carboxylic acid isopropyl ester | 3.69 | 402.0 [*M* + H]⁺ |

### Example 154: 4-[3-(5-Methanesulfonylpyridin-2-yloxy)propyl]piperidine-1-carboxylic acid tert-butyl ester

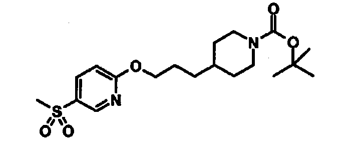

A stirred solution of 4-(3-hydroxypropyl)piperidine-1-carboxylic acid *tert*-butyl ester (1.29 g, 5.3 mmol) in THF was cooled to 0°C and sodium hydride (233 mg of a 60% dispersion in oil, 5.83 mmol) was added portionwise. After 15 min, solid 2-bromo-5-methylsulfonyl-pyridine was introduced and the resulting mixture heated under reflux for 20 h. The solvent was removed and the residual material dissolved in EtOAc (150 mL), washed with saturated aqueous NaHCO₃ (20 mL), brine (20 mL) and dried (MgSO₄). Removal of the solvent and purification of the residue afforded the title aryl ether: RT = 3.87 min; *m*/*z* (ES⁺) = 399.1 [*M* + H]⁺.

### Example 155: 4-[3-(5-Cyanopyridin-2-yloxy)propyl]piperidine-1-carboxylic acid tert-butyl ester

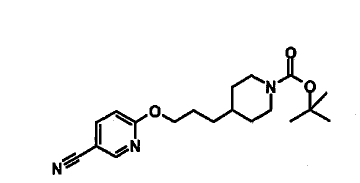

2-Chloro-5-cyanopyridine was reacted with 4-(3-hydroxypropyl)piperidine-1-carboxylic acid *tert*-butyl ester using the procedure described in **Example 154** to give the title compound: RT = 3.86 min; *m*/*z* (ES⁺) = 346.3 [*M* + H]⁺.

### Example 156: 4-[3-(4-Carboxy-3-fluorophenoxy)propyl]piperidine-1-carboxylic acid tert-butyl ester

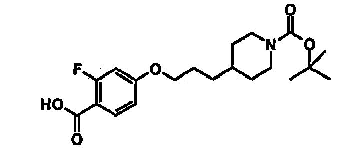

A mixture of 2-fluoro-4-hydroxybenzoic acid methyl ester (**Preparation 15**, 800 mg, 4.7 mmol), 4-(3-hydroxypropyl)piperidine-1-carboxylic acid *tert*-butyl ester (1.04 g, 4.27 mmol) and triphenylphosphine (1.23 g, 4.7 mmol) was dissolved in anhydrous THF (40 mL) and cooled to 0°C. Neat diisopropylazodicarboxylate (924 µL, 4.7 mmol) was introduced and the resulting solution brought to rt and stirred for 6 h. More triphenylphosphine (615 mg, 2.35 mmol) and diisopropylazodicarboxylate (462 µL, 2.35 mmol) were added and stirring continued for a further 18 h. The reaction mixture was diluted with EtOAc (200 mL), washed with saturated aqueous Na₂CO₃ (40 mL) and dried (MgSO₄) The solvent was removed and the resulting oil triturated with Et₂O-IH to precipitate triphenylphosphine oxide, which was removed by filtration. The filtrate was evaporated and the residue purified by column chromatography (IH-EtOAc 4:1) to give 4-[3-(3-fluoro-4-methoxycarbonylphenoxy)propyl]piperidino-1-carboxylic acid *tert*-butyl ester: RT = 4.45 min; *m*/*z* (ES⁺) = 396.3 [*M* + H]⁺. A sample of this methyl ester (1.04 g, 2.63 mmol) was dissolved in MeOH (20 mL) and LiOH.H_{2O} (1.1 g, 26.3 mmol) in water (5 mL) added. After stirring for 20 h, the methanol was evaporated and water (30 mL) added, which was acidified to pH 4 using 2 M aqueous HCl. The resulting suspension was extracted into EtOAc (3 × 70 mL), and the combined extracts dried (MgSO₄) and evaporated to give the title acid: RT = 3.94 min; *m*/*z* (ES⁺) = 382.3 [*M* + H]⁺.

### Example 157: 4-[3-(4-Carboxyphenoxy)propyl]piperidine-carboxylic acid tert-butyl ester

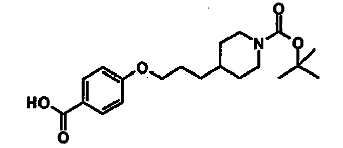

A solution of methyl-4-hydroxybenzoate (1.82 g, 12 mmol) in anhydrous THF (50 mL) was treated with sodium hydride (480 mg of a 60% dispersion in oil, 12 mmol) and stirred for 30 min. 4-(2-Methanesulfonyloxypropyl)piperidine-1-carboxylic acid *tert*-butyl ester (**Preparation 9**, 700 mg, 2.18 mmol), dissolved in dry THF (20 mL), was added and the reaction heated under reflux for 3 days. On cooling, Et₂O (150 mL) was added and the mixture washed with 2 M aqueous NaOH (3 × 30 mL) and brine (30 mL) then dried (MgSO₄). Following removal of the solvent, the crude residue was redissolved in MeOH (10 mL) and LiOH.H₂O (894 mg, 21.8 mmol) in water (2.5 mL) was added. This mixture was stirred at rt for 18 h, the methanol removed *in vacuo* and the remaining aqueous solution was acidified to pH 3 using 2 M aqueous HCl. The resulting mixture was extracted into Et₂O (2 × 40 mL); the combined organics were then extracted with 2 M aqueous NaOH (3 × 20 mL) and these combined aqueous phases acidified to pH 3 using conc HCl. The precipitated solid was extracted into Et₂O (3 × 30 mL), the combined organics dried (MgSO₄) and the solvent evaporated to give the title acid: RT = 3.76 min; *m*/*z* (ES⁻) = 362.5 [*M* - H]⁻.

### Example 158: 6-[3-(1-tert-Butoxycarbonylpiperidin-4-yl-propoxy]nicotinic acid

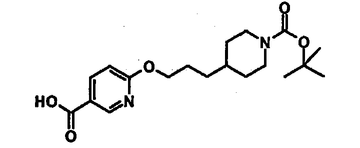

A mixture of 4-[3-(5-cyanopyridin-2-yloxy)propyl]piperidine-1-carboxylic acid *tert*-butyl ester **(Example 155,** 2.90 g, 8.40 mmol), 2 M aqueous NaOH (25 mL, 50 mmol) and EtOH (50 mL) was heated under reflux for 48 h. The EtOH was evaporated and the remaining aqueous phase acidified to pH 5 using conc HCl. The precipitated solid was extracted into EtOAc (200 mL) which was dried (MgSO₄) and evaporated. The resulting solid was dissolved in 50% saturated aqueous Na₂CO₃ (30 mL), was washed with EtOAc (20 mL) and, using conc HCl, acidified to pH 5. After extraction with EtOAc (2 × 70 mL) the combined organics were dried (MgSO₄) and evaporated to afford the title acid: RT = 3.57 min; *m*/*z* (ES⁺) = 365.2 [*M* + H]⁺.

### Example 159: 4-[3-(4-Carboxy-3-fluorophenoxy)propyl]piperidine-1-carboxylic acid isopropyl ester

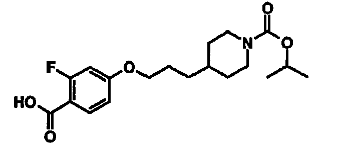

This carboxylic acid was prepared employing procedures similar to those outlined in **Example 156:** RT = 3.64 min; *m*/*z* (ES⁺) = 368.1 [*M* + H]⁺.

### Example 160: 4-[3-(4-Carboxy-3,5-difluorophenoxy)propyl]piperidine-1-carboxylic acid tert-butyl ester

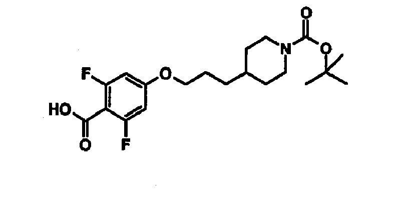

Reaction of 4-bromo-3,5-difluorophenol with 4-(3-hydroxypropyl)piperidine-1-carboxylic acid *tert*-butyl ester employing the Mitsunobu reaction, as outlined in **Example 156,** furnished 4-[3-(4-bromo-3,5-difluorophenoxy)propyl]piperidine-1-carboxylic acid *tert*-butyl ester: RT = 4.61 min. A solution of this aryl bromide (2.28 g, 5.2 mmol) in anhydrous THF (10 mL) was added to a stirred solution of n-BuLi (4.2 mL of a 2.5 M solution in hexanes, 10.5 mmol) in anhydrous THF (10 mL) at-78 °C. After 40 min, CO₂ (g) was bubbled through the reaction mixture as it was allowed to warm to ambient temperature. The reaction was quenched with H₂O, the THF removed under reduced pressure and EtOAc added. The mixture was extracted with water. The combined aqueous extracts were acidified to pH2 with 1M HCl and then extracted with EtOAc. The organic layer was washed with brine and dried (MgSO₄). Filtration, solvent evaporation, and flash chromatography (EtOAc-Hexane-AcOH, 100:100:1) furnished the title compound: RT = 3.84 min; *m*/*z* (ES⁺) = 400.1 [*M* + H]⁺.

### Example 161: 4-[3-(4-Carboxy-3-fluorophenylamino)propyl]piperidine-1-carboxylic acid tert-butyl ester

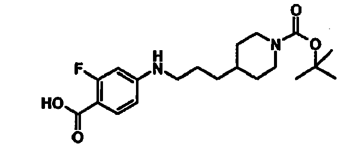

NaH (364 mg of a 60% dispersion in mineral oil, 9.1 mmol) was added to a stirred solution of methyl 4-amino-2-fluorobenzoate (1.03 g, 6.1 mmol) in anhydrous THF (10 mL). After 30 min, a solution of 4-(2-methanesulfonyloxypropyl)piperidine-1-carboxylic acid *tert-*butyl ester (**Preparation 9**, 1.95 g, 6.1 mmol) was added and the reaction heated under reflux for 5 d, before being quenched with H₂O (20 mL) and extracted with EtOAc (100 mL). The organic layer was washed with saturated aqueous Na₂CO₃ and brine and dried (MgSO₄). Filtration, solvent evaporation, and flash chromatography (EtOAc-IH, 3:7) furnished 4-[3-(3-fluoro-4-methoxycarbonylphenylamino)propyl]piperidine-1-carboxylic acid *tert*-butyl ester: *m*/*z* (ES⁺) = 395.2 [*M* + H]⁺. A mixture of this ester (300 mg, 0.76 mmol) and LiOH·H₂O (319 mg, 7.6 mmol) in MeOH-H₂O (12.5 mL, 4:1) was heated under reflux for 20 h. The MeOH was removed in vacuo, then the remainder was acidified to pH5 with 0.5M HCl, before being extracted with EtOAc. The organic extracts were washed with brine, before being dried, filtered and concentrated to furnish the title compound: RT = 3.72 min; *m*/*z* (ES⁻) = 379.5 [*M* - H]⁻.

### Example 162: 4-[3-(4-Ethylcarbamoyl-3-fluorophenoxy)propyl]piperidine-1-carboxylic acid tert-butyl ester

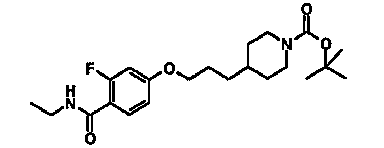

A solution of 4-[3-(4-carboxy-3-fluorophenoxy)propyl]piperidine-1-carboxylic acid *tert-*butyl ester **(Example 156,** 100 mg, 260 µmol) and diisopropylethylamine (113 µL, 650 µmol) in anhydrous THF (5 mL) was treated firstly with HATU (119 mg, 300 µmol) and then, after stirring for 1 h, ethylamine (130 µL of a 2 M solution in THF, 260 µmol). Stirring was continued for a further 18 h and the mixture then diluted with CH₂Cl₂ (30 mL). After washing with saturated aqueous Na₂CO₃ (5 mL), the organic phase was dried (MgSO₄), evaporated and the resuidue purified by column chromatography (IH-EtOAc 7:3) to afford the title compound: RT = 4.07 min; *m*/*z* (ES⁺) = 409.2 [*M* + H]⁺.

The amides listed in **Table 19** were prepared by reacting either 4-[3-(4-carboxy-3-fluorophenoxy)propyl]piperidine-1-carboxylic acid *tert*-butyl ester **(Example 156)**, 4-[3-(4-carboxyphenoxy)propyl]piperidine-1-carboxylic acid *tert*-butyl ester **(Example 157)**, 6-[3-(1-*tert*-butoxycarbonylpiperidin-4-ylpropoxy]nicotinic acid **(Example 158),** 4-[3-(4-carboxy-3-fluorophenoxy)propyl]piperidine-1-carboxylic acid isopropyl ester **(Example 159),** 4-[3-(4-carboxy-3,5-difluorophenoxy)propyl]piperidine-1-carboxylic acid *tert*-butyl ester **(Example 160)**, or 4-[3-(4-carboxy-3-fluorophenylamino)propyl]piperidine-1-carboxylic acid *tert*-butyl ester **(Example 161)** with the appropriate amine, using a procedure similar to that described in **Example 162.**

**Table 19**

| **Eg** | **Structure** | **Name** | **RT (min)** | ***m*/*z* (ES⁺)** |
|---|---|---|---|---|
| **163** | 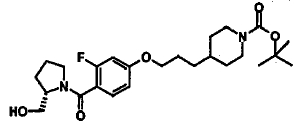 | 4-{3-[3-Fluoro-4-((*S*)-2-hydroxy methylpyrrolidine-1-carbonyl) phenoxy]propyl}piperidine-1-carboxylic acid *tert*-butyl ester | 3.76 | 465.2 [*M* + H]⁺ |
| **164** | 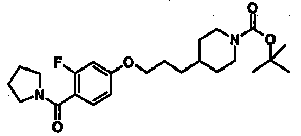 | 4-{3-[3-Fluoro-4-(pyrrolidine-1-carbonyl)phenoxy]propyl} piperidine-1-carboxylic acid *tert-* butyl ester | 3.92 | 435.2 [*M* + H]⁺ |
| **165** | 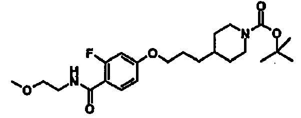 | 4-{3-[3-Fluoro4-(2-methoxy ethylcarbamoyl)phenoxy]propyl} piperidine-1-carboxylic acid *tert-* butyl ester | 4.01 | 439.2 [*M* + H]⁺ |
| **166** | 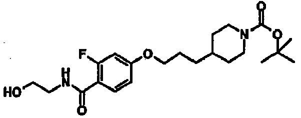 | 4-{3-[3-Fluoro-4-(2-hydroxyethyl carbamoyl)phenoxy]propyl} piperidine-1-carboxylic acid *tert*- butyl ester | 3.62 | 425.2 [*M* + H]⁺ |
| **167** | 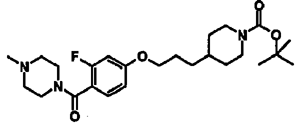 | 4-{3-[3-Fluoro-4-(4-methyl piperazine-1-carbonyl)phenoxy] propyl}piperidine-1-carboxylic acid *tert*-butyl ester | 2.99 | 464.2 [*M* + H]⁺ |
| **168** | 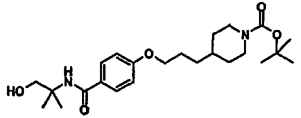 | 4-{3-[4-(2-Hydroxy-1,1-dimethyl ethylcarbamoyl)phenoxy]propyl} piperidine-1-carboxylic acid *tert*-butyl ester | 3.74 | 435.3 [*M* + H]⁺ |
| **169** | 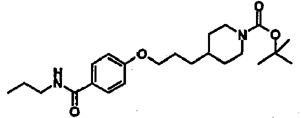 | 4-[3-(4-Propylcarbamoyl phenoxy)propyl]piperidine-1-carboxylic acid *tert*-butyl ester | 3.90 | 405.3 [*M* + H]⁺ |
| **170** | 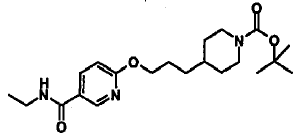 | 4-[3-(5-Ethylcarbamoylpyridin-2-yloxy)propyl]piperidine-1-carboxylic acid *tert*-butyl ester | 3.72 | 392.2 [*M* + H]⁺ |
| **171** | 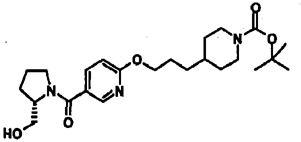 | 4-{3-[5-((*S*)-2-Hydroxymethyl pyrrolidine-1-carbonyl)pyridine -2-yloxy]propyl}piperidine-1-carboxylic acid *tert*-butyl ester | 3.54 | 448.3 [*M* + H]⁺ |
| **172** | 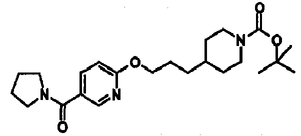 | 4-{3-[5-(Pyrrolidine-1-carbonyl) pyridin-2-yloxy]propyl} piperidine-1-carboxylic acid *tert*-butyl ester | 3.81 | 418.3 [*M* + H]⁺ |
| **173** | 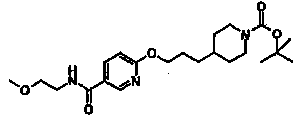 | 4-{3-[5-(2-Methoxyethyl carbamoyl)pyridin-2-yloxy] propyl}piperidine-1-carboxylic acid *tert*-butyl ester | 3.62 | 422.3 [*M* + H]⁺ |
| **174** | 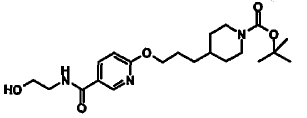 | 4-{3-[5-(2-Hydroxyethyl carbamoyl)pyridin-2-yloxy] propyl}piperidine-1-carboxylic acid *tert*-butyl ester | 3.27 | 408.3 [*M* + H]⁺ |
| **175** | 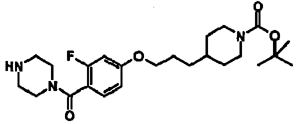 | 4-{3-[3-Fluoro-4-(piperazine-1-carbonyl)phenoxy]propyl}-piperidine-1-carboxylic acid *tert*-butyl ester | 2.89 | 438.1 [*M* + H]⁺ |
| **176** | 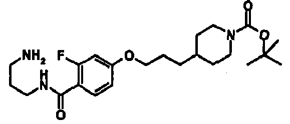 | 4-{3-[4-(3-Amino-propylcarbamoyl)-3-fluoro-phenoxy]propyl}piperidine-1-carboxylic acid *tert*-butyl ester | 2.82 | 450.2 [*M* + H]⁺ |
| **177** | 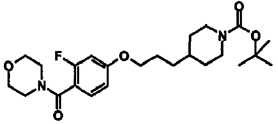 | 4-{3-[3-Fluoro-4-(morpholine-4-carbonyl)phenoxy]propyl}-piperidine-1-carboxylic acid *tert*-butyl ester | 3.92 | 451.2 [*M* + H]⁺ |
| **178** | 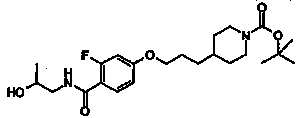 | 4-{3-[3-Fluoro-4-(2-hydroxy-propylcarbamoyl)phenoxy]-propyl}piperidine-1-carboxylic acid *tert*-butyl ester | 3.74 | 439.2 [*M* + H]⁺ |
| **179** | 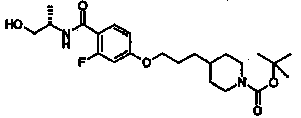 | 4-{3-[3-Fluoro-4-((*S*)-2-hydroxy-1-methylethylcarbamoyl)-phenoxy]propyl}piperidine-1-carboxylic acid *tert*-butyl ester | 3.70 | 439.2 [*M* + H]⁺ |
| **180** | 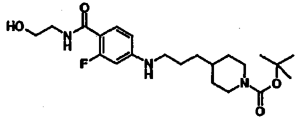 | 4-{3-[3-Fluoro4-(2-hydroxy-ethylcarbamoyl)phenylamino]-propyl}piperidine-1-carboxylic acid *tert*-butyl ester | 3.56 | 424.1 [*M* + H]⁺ |
| **181** | 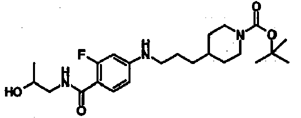 | 4-{3-[3-Fluoro-4-(2-hydroxy-propylcarbamoyl)phenylamino]-propyl}piperidine-1-carboxylic acid *tert*-butyl ester | 3.61 | 438.1 [*M* + H]⁺ |
| **182** | 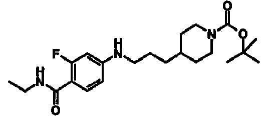 | 4-[3-(4-Ethylcarbamoyl-3-fluoro-phenylamino)propyl]piperidine-1-carboxylic acid *tert*-butyl ester | 3.95 | 408.1 [*M* + H]⁺ |
| **183** | 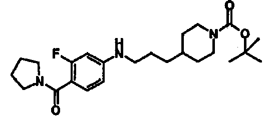 | 4-{3-[3-Fluoro-4-(pyrrolidine-1-carbonyl)phenylamino]propyl}-piperidine-1-carboxylic acid *tert*butyl ester | 3.94 | 434.1 [*M* + H]⁺ |
| **184** | 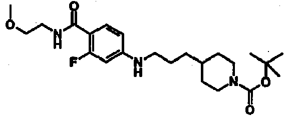 | 4-{3-[3-Fluoro-4-(2-methoxy ethylcarbamoyl)phenylamino]-propyl}piperidine-1-carboxylic acid *tert*-butyl ester | 3.81 | 438.1 [*M* + H]⁺ |
| **185** | 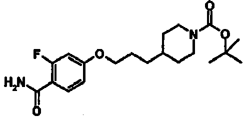 | 4-[3-(4-Carbamoyl-3-fluoro-phenoxy)propyl]piperidine-1-carboxylic acid *tert*-butyl ester | 3.67 | 381.1 [*M* + H]⁺ |
| **186** | 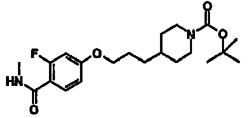 | 4-[3-(3-Fluoro-4-methylcarbamoylphenoxy)-propyl]piperidine-1-carboxylic acid *tert*-butyl ester | 3.86 | 395.1 [*M* + H]⁺ |
| **187** | 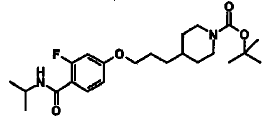 | 4-[3-(3-Fluoro-4-isopropylcarbamoylphenoxy)-propyl]piperidine-1-carboxylic acid *tert*-butyl ester | 3.99 | 423.1 [*M* + H]⁺ |
| **188** | 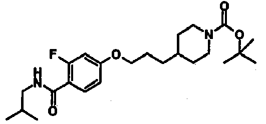 | 4-[3-(3-Fluoro-4-isobutylcarbamoylphenoxy)-propyl]piperidine-1-carboxylic acid *tert*-butyl ester | 4.17 | 437.1 [*M* + H]⁺ |
| **189** | 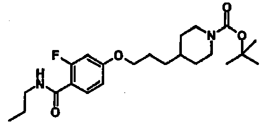 | 4-[3-(3-Fluoro-4-propylcarbamoylphenoxy)-propyl]piperidine-1-carboxylic acid *tert-*butyl ester | 3.90 | 423.1 [*M* + H]⁺ |
| **190** | 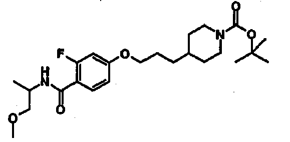 | 4-{3-[3-Fluoro-4-(2-methoxy-1-methylethylcarbamoyl)-phenoxy]propyl}piperidine-1-carboxylic acid *tert*-butyl ester | 3.99 | 433.1 [*M* + H]⁺ |
| **191** | 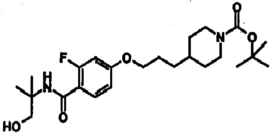 | 4-{3-[3-Fluoro-4-(2-hydroxy-1,1-dimethylethylcarbamoyl)-phenoxy]propyl}piperidine-1-carboxylic acid *tert*-butyl ester | 3.79 | 453.1 [*M* + H]⁺ |
| **192** | 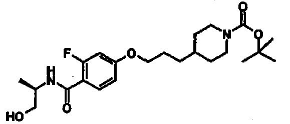 | 4-{3-[3-Fluoro4-((*R*)2-hydroxy-1-methylethylcarbamoyl)-phenoxy]propyl}piperidine-1-carboxylic acid *tert*-butyl ester | 3.61 | 439.1 [*M* + H]⁺ |
| **193** | 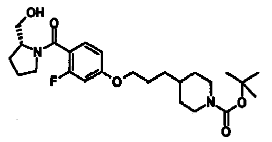 | 4-{3-[3-Fluoro-4-((*R*)-2-hydroxymethylpyrrolidine-1-carbonyl)phenoxy]propyl}-piperidine-1-carboxylic acid *tert*-butyl ester | 3.61 | 465.1 [*M* + H]⁺ |
| **194** | 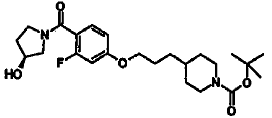 | 4-{3-[3-Fluoro-4-((*S*)-3-hydroxy-pyrrolidine-1-carbonyl)-phenoxy]propyl}piperidine-1-carboxylic acid *tert*-butyl ester | 3.49 | 451.1 [*M* + H]⁺ |
| **195** | 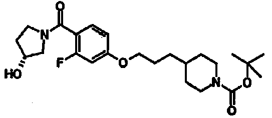 | 4-{3-[3-Fluoro-4-((*R*)-3-hydroxy-pyrrolidine-1-carbonyl)-phenoxy]propyl}piperidine-1-carboxylic acid *tert*-butyl ester | 3.45 | 451.1 [*M* + H]⁺ |
| **196** | 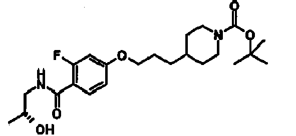 | 4-{3-[3-Fluoro-4-((*R*)-2-hydroxy-propylcarbamoyl)phenoxy]-propyl}piperidine-1-carboxylic acid *tert*-butyl ester | 3.65 | 439.1 [*M* + H]⁺ |
| **197** | 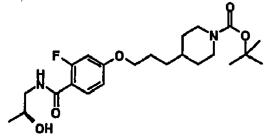 | 4-{3-[3-Fluoro-4-((*S*)-2-hydroxy-propylcarbamoyl)phenoxy]-propyl}piperidine-1-carboxylic acid *tert*-butyl ester | 3.59 | 439.1 [*M* + H]⁺ |
| **198** | 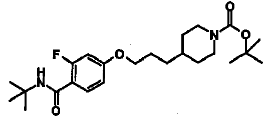 | 4-[3-(4-tert-Butylcarbamoyl-3-fluorophenoxy)propyl]-piperidine-1-carboxylic acid *tert*-butyl ester | 4.24 | 437.1 [*M* + H]⁺ |
| **199** | 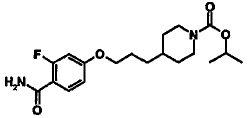 | 4-[3-(4-Carbamoyl-3-fluoro-phenoxy)propyl]piperidine-1-carboxylic acid isopropyl ester | 3.52 | 367.1 [*M* + H]⁺ |
| **200** | 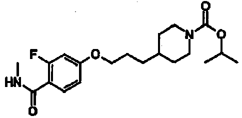 | 4-[3-(3-Fluoro-4-methylearbamoylphenoxy)-propyl]piperidine-1-carboxylic acid isopropyl ester | 3.56 | 381.1 [*M* + H]⁺ |
| **201** | 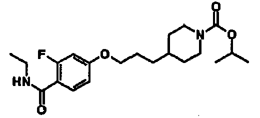 | 4-[3-(4-Ethylcarbamoyl-3-fluoro-phenoxy)propyl]piperidine-1-carboxylic acid isopropyl ester | 3.77 | 395.1 [*M* + H]⁺ |
| **202** | 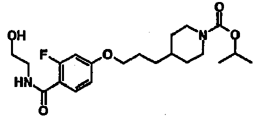 | 4-{3-[3-Fluoro-4-(2-hydroxy-ethylcarbamoyl)phenoxy]-propyl}piperidina-1-carboxylic acid isopropyl ester | 3.38 | 411.1 [*M* + H]⁺ |
| **203** | 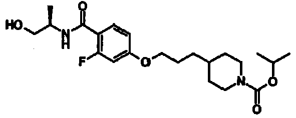 | 4-{3-[3-Fluoro-4-((*R*)-2-hydroxy-1-methylethylcarbamoyl)-phenoxy]propyl}piperidine-1-carboxylic acid isopropyl ester | 3.44 | 425.1 [*M* + H]⁺ |
| **204** | 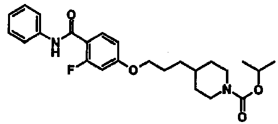 | 4-[3-(3-Fluoro-4-phenylcarbamoylphenoxy)-propyl]piperidine-1-carboxylic acid isopropyl ester | 4.01 | 443.1 [*M* + H]⁺ |
| **205** | 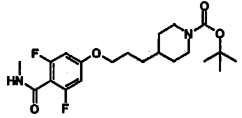 | 4-[3-(3,5-Difluoro-4-methylcarbamoylphenoxy)-propyl]piperidine-1-carboxylic acid *tert*-butyl ester | 3.62 | 413.1 [*M* + H]⁺ |
| **206** | 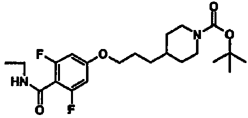 | 4-[3-(4-Ethylcarbamoyl-3,5-difluorophenoxy)propyl]-piperidino-1-carboxylic acid *tert-* butyl ester | 3.76 | 427.1 [*M* + H]⁺ |
| **207** | 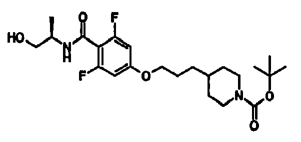 | 4-{3-[3,5-Difluoro-4-((R)-2-hydroxy-1-methyl-ethylcarbamoyl)phenoxy]-propyl}piperidine-1-carboxylic acid *tert*-butyl ester | 3.59 | 457.1 [*M* + H]⁺ |
| **208** | 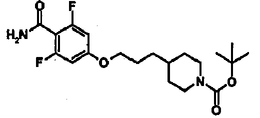 | 4-[3-(4-Carbamoyl-3,5-difluorophenoxy)propyl]piperidine-1-carboxylic acid *tert*-butyl ester | 3.61 | 399.1 [*M* + H]⁺ |
| **209** | 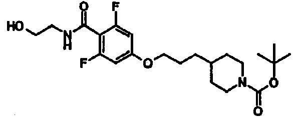 | 4-{3-[3,5-Difluoro-4-(2-hydroxy-ethylcarbamoyl)phenoxy]-propyl}piperidine-1-carboxylic acid *tert*-butyl ester | 3.37 | 443.1 [*M* + H]⁺ |

### Example 210:4-[2-(4-Methylsulfanylbenzyloxy)ethyl]piperidine-1-carboxylic acid tert-butyl ester

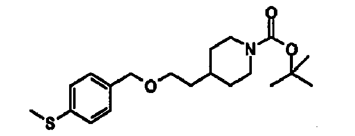

Powdered KOH (449 mg, 8 mmol) was suspended in anhydrous toluene (20 mL) and (4-methylthio)benzyl chloride (345 mg, 2 mmol) added in one portion. After stirring for 10 min, 4-(2-hydroxyethyl)piperidina-1-carboxylic acid *tert-*butyl ester (688 mg, 3 mmol) was added followed by tris[2-(2-methoxyethoxy)ethyl]amine (64 µL, 200 µmol) and the resulting mixture heated under gentle reflux for 18 h. The mixture was cooled, diluted with toluene and washed with water (30 mL). The aqueous phase was extracted with toluene (2 x 20 mL) and the combined organics washed with brine (30 mL), dried (MgSO₄) and evaporated to afford the title compound: RT = 4.47 min; *m*/*z* (ES⁺) = 366.2 [*M* + H]⁺.

### Example 211: 4-[2-(4-Methylsulfanylphenyl)ethoxymethyl]piperidine-1-carboxylic acid tertbutyl ester

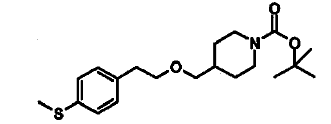

2-(4-Methylsulfanylphenyl)ethanol (100 mg, 595 µmol) was dissolved in dry DMF (4 mL), cooled to 0°C, and sodium hydride (36 mg of a 60 % dispersion in oil, 900 µmol) added. The mixture was warmed to rt and, after stirring for 1 h, tetra-*n*-butylammonium iodide (22 mg, 60 µmol) and 4-methanesulfonyloxymethylpiperidine-1-carboxylic acid *tert*-butyl ester (210 mg, 717 µmol) were added. Stirring was continued for 18 h and the reaction quenched by the addition of water (1 mL). Following dilution with EtOAc (30 mL) the organic phase was washed with water (5 mL) and brine (5 mL) then dried (MgSO₄) Removal of the solvent and purification of the residual oil by column chromatography (IH-EtOAc 6:1) afforded the title ether: RT = 4.16 min; *mlz* (ES⁺) = 366.2 [*M* + H]⁺.

### Example 212 and 213: 4-[2-(4-Methanesulfinylbenzyloxy)ethyl]piperidine-1-carboxylic acid tert-butyl ester and 4-[2-(4-methanesulfonylbenzyloxy)ethyl]piperidine-1-carboxylic acid tert-butyl ester

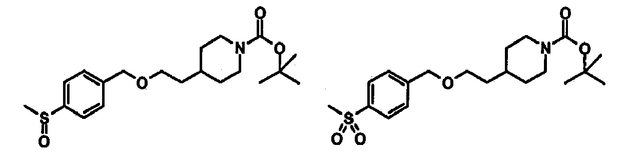

4-[2-(4-Methylsulfanylbenzyloxy)ethyl]piperidine-1-carboxylic acid *tert*-butyl ester **(Example 210)** was oxidised by *m*CPBA using the method described in **Examples 18 and 19** to give the title sulfoxide: RT = 3.49 min; *mlz* (ES⁺) = 382.2 [*M* + H]⁺, and the title sulfone: RT = 3.65 min; *mlz* (ES⁺) = 398.2 [*M* + H]⁺.

### Example 214 and 215: 4-[2-(4-Methanesulfinylphenyl)ethoxymethyl]piperidine-1-carboxylic acid tert-butyl ester and 4-[2-(4-methanesulfonylphenyl)ethoxymethyl]piperidine-1-carboxylic acid tert-butyl ester

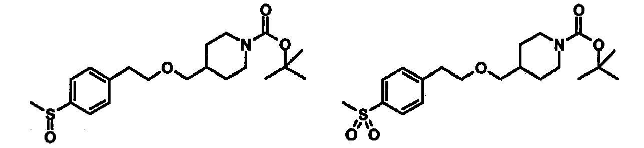

4-[2-(4-Methylsulfanylphenyl)ethoxymethyl]piperidine-1-carboxylic acid *tert*-butyl ester **(Example 211)** was oxidised using the procedure described in **Examples 18 and 19.** Purification afforded the title sulfoxide: RT = 3.40 min; *mlz* (ES⁺) = 382.2 [*M* + H]⁺ and the title sulfone: RT = 3.59 min; *mlz* (ES⁺) = 398.3 [*M* + H]⁺.

### Example 216: 4-[(E)-3-(4-Methanesulfonylphenyl)allyloxy]piperidine-1-carboxylic acid tert-butyl ester

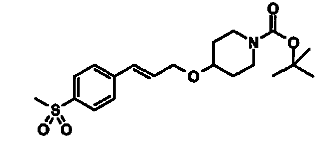

Sodium hydride (34 mg of a 60% dispersion in oil, 860 µmol) was added in one portion to a stirred solution of (4-methanesulfonylbenzyl)phosphonic acid diethyl ester (263 mg, 860 µmol) in dry DME (7 mL). After 30 min, a solution of 4-(2-oxoethoxy)piperidine-1-carboxylic acid *tert*-butyl ester **(Preparation 16**, 149 mg, 610 µmol) in dry DME (2 mL) was introduced and stirring continued for 3 h. Water (5 mL) was added and the mixture extracted into EtOAc (3 x 20 mL). The combined organics were washed with brine (5 mL), dried (MgSO₄) and evaporated to give a crude product, which was purified by flash chromatography (IH-EtOAc 7:3 then 3:2), affording the title olefin: RT = 3.44 min; *m*/*z* (ES⁺) = 396.3 [*M* + H]⁺.

### Example 217: 4-[3-(4-Methanesulfonylphenyl)propoxy]piperidine-1-carboxylic acid tert-butyl ester

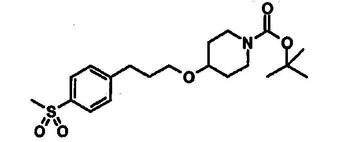

A sample of 4-[(*E*)-3-(4-methanesulfonylphenyl)allyloxy]piperidine-1-carboxylic acid *tert*-butyl ester **(Example 216)** in EtOH was hydrogenated over a Pd catalyst using the method described in **Example 2,** to give the title compound: RT = 3.54 min; *mlz* (ES⁺) = 398.3 [*M* + H]⁺.

### Example 218: 4-[2-(2,3-Difluorobenzyloxy)ethyl]piperidine-1-carboxylic acid tert-butyl ester

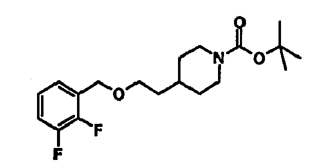

A stirred solution of (2,3-difluorophenyl)methanol (50 mg, 350 µmol) in anhydrous THF (2 mL) was treated with *t*BuOK (47 mg, 42 µmol) and then 4-(2-methanesulfonyloxy-ethyl)piperidine-1-carboxylic acid *tert*-butyl ester **(Preparation 10)** was added. The resulting mixture was heated under reflux for 12 h, cooled and poured into saturated aqueous NH₄Cl, and extracted with EtOAc (20 mL). The organic phase was washed with brine (5 mL), dried (MgSO₄) and evaporated. The residue was purified by column chromatography (IH-EtOAc, 4:1) to afford the title compound: RT = 4.39 min; *mlz* (ES⁺) = 356.1 [*M* + H]⁺.

### Example 219: 4-[2-(3,4-Difluorobenzyloxy)ethyl]piperidine-1-carboxylic acid tert-butyl ester

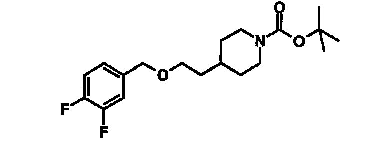

Using the procedure described in **Example 218,** (3,4-difluorophenyl)methanol was converted to the title compound: RT = 4.30 min; *m*/*z* (ES⁺) = 356.1 [*M* + H]⁺.

### Example 220: 4-[3-(4-Methanesulfonylphenylsulfanyl)propyl]piperidine-1-carboxylic acid tert-butyl ester

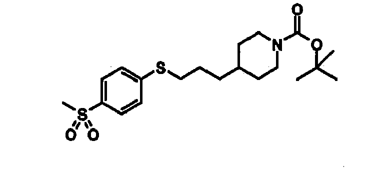

In a dried flask under argon, sodium hydride (121 mg of a 60% dispersion in oil, 3.02 mmol) was suspended in dry THF (4 mL) and cooled in an ice bath. A solution of 4-methanesulfonylbenzenethiol (582 mg, 2.83 mmol) in dry THF (3 mL) was added and the mixture stirred for 0.5 h. A solution of 4-(3-methanesulfonyloxypropyl)piperidine-1-carboxylic acid *tert*-butyl ester **(Preparation 9,** 359 mg, 1.12 mmol) in dry THF (3 mL) was added slowly and the resulting slurry heated at 65°C for 18 h. On cooling, the mixture was diluted with ether (60 mL), washed with 2 M aqueous NaOH (2 x 10 mL), water (60 mL) and brine (50 mL) then dried (MgSO₄) Removal of the solvent and purification of the residue by column chromatography (IH-EtOAc 3:1) afforded the title compound: RT = 3.99 min; *m*/*z* (ES⁺) = 414.2 [*M* + H]⁺.

### Examples 221 and 222: 4-[3-(4-Methanesulfonylbenzenesulfinyl)propyl]piperidine-1-carboxylic acid tert-butyl ester 4-[3-(4-methanesulfonylbenzenesulfonyl)propyl]piperidino-1-carboxylic acid tert-butyl ester

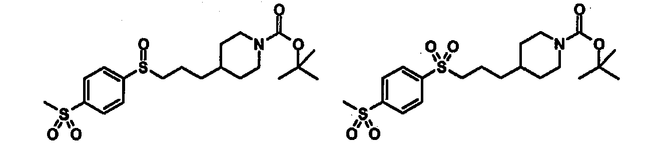

A sample of 4-[3-(4-methanesulfonylphenylsulfanyl)propyl]piperidine-1-carboxylic acid *tert*-butyl ester **(Example 220)** was oxidised using *m*CPBA, according to the procedure described in **Examples 18 and 19,** to the title sulfoxide: RT = 3.36 min; *mlz* (ES⁺) = 430.3 [*M* + H]⁺ and to the title sulfone: RT = 3.59 min; *m*/*z* (ES⁺) = 446.3 [*M* + H]⁺.

### Example 223: 4-[3-(3-Fluoro-4-methylsulfanylphenylcarbamoyl)propyl]piperidine-1-carboxylic acid tert-butyl ester

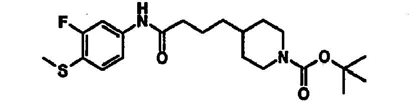

A solution of 4-(3-carboxypropyl)piperidine-1-carboxylic acid *tert*-butyl ester (200 mg, 737 µmol), EDCI (141 mg, 737 µmol), HOBt (99 mg, 737 µmol), and DIPEA (0.38 mL, 2.2 mmol) in anhydrous DMF (10 mL) was stirred at 20°C for 10 min, before being treated with 3-fluoro-4-methylsulfanylaniline (105 mg, 670 µmol). After 112 h, the reaction was concentrated in vacuo and the residue dissolved in EtOAc. The organic layer was washed with saturated aqueous Na₂CO₃, and dried (MgSO₄). Filtration, solvent evaporation, and flash chromatography (IH-EtOAc, 3:2) furnished the title compound: RT = 3.97 min; *m*/*z* (ES⁺) = 411.1 [*M* + H]⁺.

### Examples 224 and 225: 4-[3-(3-Fluoro-4-methanesulfinylphenylcarbamoyl)propyl]piperidine-1-carboxylic acid tert-butyl ester and 4-[3-(3-Fluoro-4-methariesulfonylphenylcarbamoylr propyl]piperidine-1-carboxylic acid tert-butyl ester

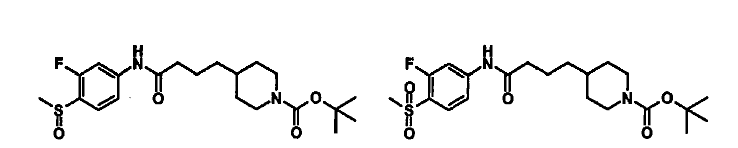

A sample of 4-[3-(3-fluoro-4-methylsulfanylphenylcarbamoyl)propyl]piperidine-1-carboxylic acid *tert*-butyl ester **(Example 223)** was oxidised using *m*CPBA, according to the procedure described in **Examples 18 and 19,** to the title sulfoxide: RT = 3.34 min; *m*/*z* (ES⁺) = 427.1 [*M* + H]⁺ and to the title sulfone: RT = 3.61 min; *mlz* (ES⁺) = 443.1 [*M* + H]⁺.

### Example 226: 4-[2-(3-Fluoro-4-methanesulfonylphenylcarbamoyl)ethyl]piperidine-1-carboxylic acid tert-butyl ester

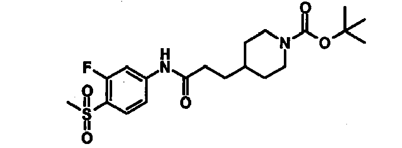

The title compound was prepared from 3-fluoro-4-methylsulfanylaniline and 4-(2-carboxyethyl)piperidino-1-carboxylic acid *tert*-butyl ester employing procedures similar to those described in **Examples 223, 224,** and **225:** RT = 3.45 min; *mlz* (ES⁺) = 446.1 [*M* + NH₄]⁺.

The sulfoxides and sulfones listed in **Table 20** were prepared by a two-step sequence: 1) Mitsunobu reaction of the appropriate phenol with the appropriate alcohol employing a protocol similar to that of **Example 156;** 2) Oxidation, as outlined in **Examples 18** and **19.**

**Table 20**

| **Eg** | **Structure** | **Name** | **RT (min)** | ***m*/*z* (ES⁺)** |
|---|---|---|---|---|
| **227** | 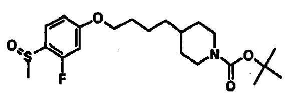 | 4-[4-(3-Fluoro-4-methanesulfinylphenoxy)butyl]-piperidine-1-carboxylic acid *tert-* butyl ester | 3.49 | 414.1 [*M* + H]⁺ |
| **228** | 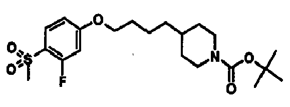 | 4-[4-(3-Fluoro-4-methanesulfonylphenoxy)butyl]-piperidine-1-carboxylic acid butyl ester | 3.62 | 430.1_{.} [*M* + H]⁺ |
| **229** | 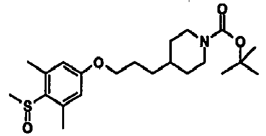 | 4-[3-(4-Methanesulfinyl-3,5-dimethylphenoxy)propyl]-piperidine-1-carboxylic acid *tert-* butyl ester | 3.81 | 410.1 [*M* + H]⁺ |
| **230** | 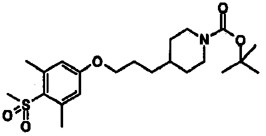 | 4-[3-(4-Methanesulfonyl-3,5-dimethylphenoxy)propyl]-piperidine-1-carboxylic acid *tert-* butyl ester | 4.11 | 426.1 [*M* + H]⁺ |
| **231** | 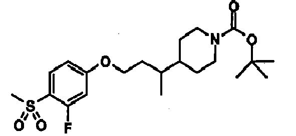 | 4-[3-(3-Fluoro-4-methanesulfonylphenoxy)-1-methylpropyl]piperidine-1-carboxylic acid *tert*-butyl ester | 3.99 | 430.0 [*M* + H]⁺ |

### Example 232: 4-[3-(3-Fluoro-4-methanesulfonylphenoxy)-2-methylpropyl]piperidine-1-carboxylic acid tert-butyl ester

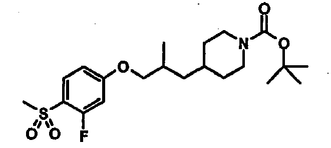

Prepared from 3-fluoro4-methylsulfanylphenol and 4-(3-hydroxy-2-methylpropyl)piperidine-1-carboxylic acid *tert*-butyl ester via the two-step Mitsunobu-oxidation sequence exemplified by **Examples 156, 18** and **19:** δ_{H} (CDCl₃) 1.04 (m, 2H), 1.15-1.27 (m, 2H), 1.35-1.50 (m, 10H), 1.56 (s, 3H), 1.65-1.75 (br m, 2H), 2.02-2.12 (m, 1H), 2.65-2.75 (br, 2H), 3.19 (s, 3H), 3.78-3.86 (m, 2H), 4.05-4.16 (m, 2H), 6.71-6.81 (m, 2H), 7.86 (t, 1H).

The sulfoxides and sulfones listed in **Table 21** were prepared by a two-step sequence: 1) Palladium-catalysed thioether formation, as outlined in **Preparation 19;** 2) Oxidation, as outlined in **Examples 18** and **19.**

**Table 21**

| **Eg** | **Structure** | **Name** | **RT (min)** | ***m*/*z* (ES⁺)** |
|---|---|---|---|---|
| **233** | 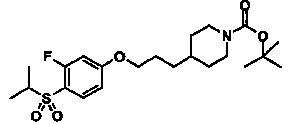 | 4-{3-[3-Fluoro-4-(propane-2-sulfonyl)phenoxy]propyl}-piperidina-1-carboxylic acid *tert-* butyl ester | 4.01 | 444.1 [*M* + H]⁺ |
| **234** | 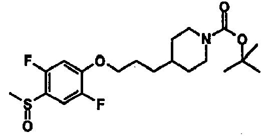 | 4-[3-(2,5-Diffuoro-4-methanesulfinylphenoxy)-propyl]piperidine-1-carboxylic acid *tert*-butyl ester | 3.89 | 418.0 [*M* + H]⁺ |
| **235** | 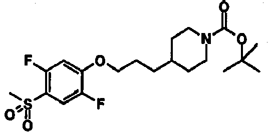 | 4-[3-(2,5-Difluoro-4-methanesulfonylphenoxy)-propyl]piperidine-1-carboxylic acid *tert*-butyl ester | 4.02 | 434.2 [*M* + H]⁺ |
| **236** | 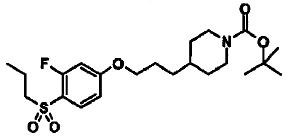 | 4-{3-[3-Fluoro-4-(propane-1-sulfonyl)phenoxy]propyl}-piperidine-1-carboxylic acid *tert*- butyl ester | 4.14 | 444.1 [*M* + H]⁺ |
| **237** | 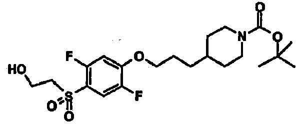 | 4-{3-[2,5-Difluoro-4-(2-hydroxy-ethanesulfonyl)phenoxy]-propyl}piperidine-1-carboxylic acid *tert*-butyl ester | 3.76 | 464.0 [*M* + H]⁺ |
| **238** | 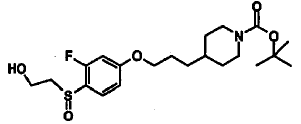 | 4-{3-[3-Fluoro-4-(2-hydroxy-ethanesulfinyl)phenoxy]propyl}-piperidine-1-carboxylic acid *tert-* butyl ester | 3.56 | 430.1 [*M* + H]⁺ |
| **239** | 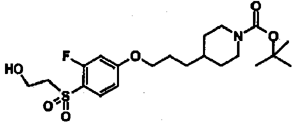 | 4-{3-[3-Fluoro-4-(2-hydroxy-ethanesulfonyl)phenoxy]-propyl}piperidine-1-carboxylic acid *tert*-butyl ester | 3.65 | 446.0 [*M* + H]⁺ |
| **240** | 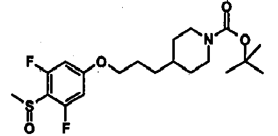 | 4-[3-(3,5-Diftuoro-4-methanesulfinylphenoxy)-propyl]piperidine-1-carboxylic acid *tert*-butyl ester | 3.64 | 418.0 [*M* + H]⁺ |
| **241** | 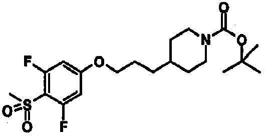 | 4-[3-(3,5-Diffuoro-4-methanesulfonylphenoxy)-propyl]piperidine-1-carboxylic acid *tert*-butyl ester | 3.89 | 434.0 [*M* + H]⁺ |
| **242** | 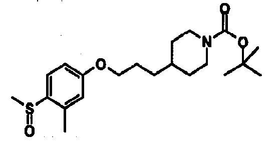 | 4-[3-(4-Methanesulfinyl-3-methylphenoxy)propyl]-piperidine-1-carboxylic acid *tert-* butyl ester | 3.72 | 396.1 [*M* + H]⁺ |
| **243** | 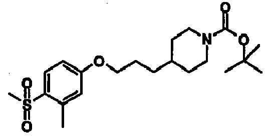 | 4-[3-(4-Methanesulfonyl-3-methylphenoxy)propyl]-piperidine-1-carboxylic acid *tert-* butyl ester | 4.01 | 412.1 [*M* + H]⁺ |
| **244** | 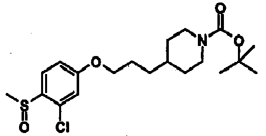 | 4-[3-(3-Chloro-4-methanesulfinylphenoxy)-propyl]piperidine-1-carboxylic acid *tert*-butyl ester | 3.89 | 416.0 [*M* + H]⁺ |
| **245** | 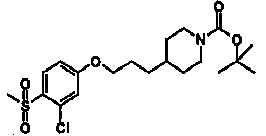 | 4-[3-(3-Chloro-4-methanesulfonylphenoxy)-propyl]piperidine-1-carboxylic acid *tert*-butyl ester | 4.04 | 432.0 [*M* + H]⁺ |
| **246** | 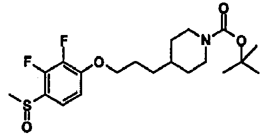 | 4-[3-(2,3-Diffuoro-4-methanesulfinylphenoxy)-propyl]piperidine-1-carboxylic acid *tert*-butyl ester | 3.77 | 418.1 [*M* + H]⁺ |
| **247** | 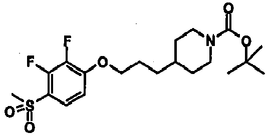 | 4-[3-(2,3-Diffuoro-4-methanesulfonylphenoxy)-propyl]piperidine-1-carboxylic acid *tert*-butyl ester | 3.94 | 434.1 [*M* + H]⁺ |
| **248** | 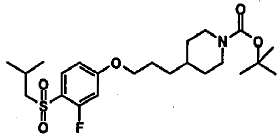 | 4-{3-[3-Fluoro-4-(2-methyl-propane-1-sulfonyl)phenoxy]-propyl}piperidine-1-carboxylic acid *tert*-butyl ester | 4.12 | 458.1 [*M* + H]⁺ |

The compounds listed in **Table 22** were prepared by a two-step sequence: 1) Deprotection of 4-[3-(3-fluoro-4-methanesulfinylphenoxy)propyl]piperidine-1-carboxylic acid *tert*-butyl ester **(Example 148)** or 4-[3-(3-fluoro-4-methanesulfonylphenoxy)propyl]piperidine-1-carboxylic acid *tert*-butyl ester **(Example 151),** by a protocol similar to that outlined in **Preparation 1;** 2) Carbamate synthesis via protocols delineated in **Examples 61** and **70.**

**Table 22**

| **Eg** | **Structure** | **Name** | **RT (min)** | ***m*/*z* (ES⁺)** |
|---|---|---|---|---|
| **249** | 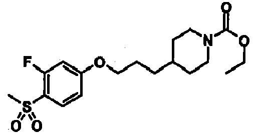 | 4-[3-(3-Fluoro-4-methanesulfonylphenoxy)-propyl]piperidine-1-carboxylic acid ethyl ester | 3.62 | 388.0 [*M* + H]⁺ |
| **250** | 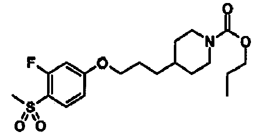 | 4-[3-(3-Fluoro-4-methanesulfonylphenoxy)-propyl]piperidine-1-carboxylic acid propyl ester | 3.64 | 402.0 [*M* + H]⁺ |
| **251** | 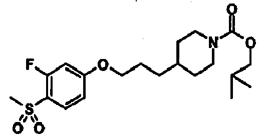 | 4-[3-(3-Fluoro-4-methanesulfonylphenoxy)-propyl]piperidine-1-carboxylic acid isobutyl ester | 3.92 | 416.0 [*M* + H]⁺ |
| **252** | 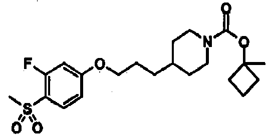 | 4-[3-(3-Fluoro-4-methanesulfonylphenoxy)-propyl]piperidine-1-carboxylic acid 1-methylcyclobutyl ester | 3.87 | 428.0 [*M* + H]⁺ |
| **253** | 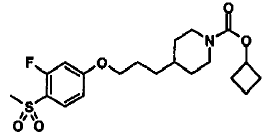 | 4-[3-(3-Fluoro-4-methanesulfonylphenoxy)-propyl]piperidine-1-carboxylic acid cyclobutyl ester | 3.87 | 414.0 [*M* + H]⁺ |
| **254** | 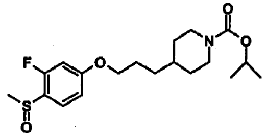 | 4-[3-(3-Fluoro-4-methanesulfinylphenoxy)propyl]-piperidine-1-carboxylic acid isopropyl ester | 3.67 | 386.0 [*M* + H]⁺ |
| **255** | 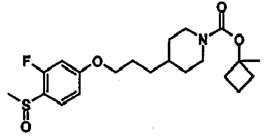 | 4-[3-(3-Fluoro-4-methanesulfmylphenoxy)propyl]-piperidine-1-carboxylic acid 1-methylcyclobutyl ester | 3.89 | 412.1 [*M* + H]⁺ |

### Example 256: 4-[3-(2-Fluoro-4-methanesulfonylphenoxy)propyl]piperidine-1-carboxylic acid tert-butyl ester

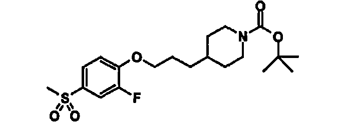

A stirred mixture of NaH (31.2 mg of a 60% dispersion in mineral oil, 1.3 mmol), 2-fluoro-4-methanesulfonylphenol (244 mg, 1.28 mmol) and 4-(3-methanesulfonyloxypropyl)piperidine-1-carboxylic acid *tert*-butyl ester **(Preparation 9,** 380 mg, 1.21 mmol) in anhydrous DMF (3 mL) was heated at 70°C for 6 h. The DMF was removed under reduced pressure and the residue taken up in Et₂O (100 mL). The solution was washed with 2M NaOH (10 mL) and brine (10 mL) and dried (MgSO₄). Filtration, solvent evaporation, column chromatography (IH-EtOAc, 1:1), and recrystallisation from Et₂O-CH₂Cl₂-IH furnished the title compound: RT = 3.77 min; *m*/*z* (ES⁺) = 416.0 [*M* + H]⁺.

### Example 257: 4-[3-(3-Fluoro-4-sulfamoylphenoxy)propyl]piperidine-1-carboxylic acid tert-butyl ester

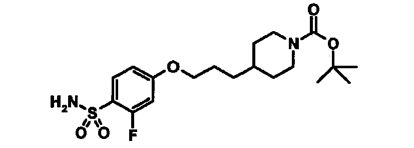

Prepared by Mitsunobu condensation of 2-fluoro-4-hydroxybenzenesulfonamide **(Preparation 25)** with 4-(3-hydroxypropyl)piperidine-1-carboxylic acid *tert*-butyl ester, using a procedure similar to that outlined in **Preparation 19:** δ_{H} (CDCl₃) 1.10-1.22 (m, 2H), 1.40-1.50 (m, 12H), 1.65-1.75 (m, 2H), 1.80-1.90 (m, 2H), 2.65-2.80 (m, 2H), 4.01 (t, 2H), 4.05-4.20 (br, 2H), 4.96 (s, 2H), 6.70-6.80 (m, 2H), 7.83 (t, 1H); RT = 3.70 min; *mlz* (ES⁻) = 415.4 [*M -* H]⁻.

### Example 258: 4-[3-(4-Methanesulfonylphenoxy)butyl]piperidine-1-carboxylic acid tert-butyl ester

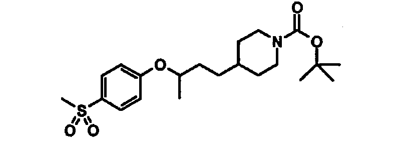

Prepared by Mitsunobu condensation of 4-methylsulfonylphenol with 4-(3-hydroxybutyl)piperidine-1-carboxylic acid *tert*-butyl ester (**Preparation 30**), using a procedure similar to that outlined in **Preparation 19:** RT = 3.92 min; *mlz* (ES⁻) = 412.1 [*M* + H]⁺.

The compounds in **Table 23** were prepared from 4-[3-(4-amino-3-fluorophenoxy)-propyl]piperidine-1-carboxylic acid *tert*-butyl ester **(Preparation 31)** using methods similar to those of **Examples 20, 35, 36** and **38.**

**Table 23**

| **Eg** | **Structure** | **Name** | **RT (min)** | ***m*/*z* (ES⁺)** |
|---|---|---|---|---|
| **259** | 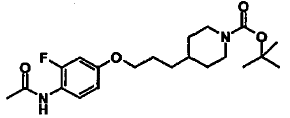 | 4-[3-(4-Acetylamino-3-fluoro-phenoxy)propyl]piperiduie-1carboxylic acid *tert*-butyl ester | 3.76 | 395.1 [*M* + H]⁺ |
| **260** | 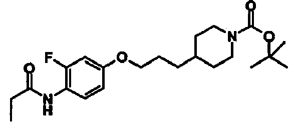 | 4-[3-(3-Fluoro-4-propionylaminophenoxy)-propyl]piperidine-1-carboxylic acid *tert*-butyl ester | 3.79 | 409.1 [*M* + H]⁺ |
| **261** | 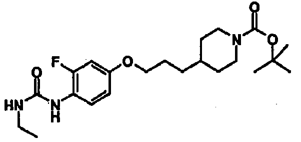 | 4-{3-[4-(3-Ethylureido)-3-fluoro-phenoxy]propyl}piperidine-1-carboxylic acid *tert*-butyl ester | 3.62 | 424.1 [*M* + H]⁺ |
| **262** | 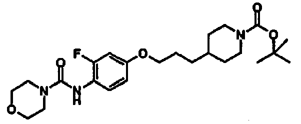 | 4-(3-{3-Fluoro-4-[(morpholine-4-carbonyl)amino]phenoxy}-propyl)piperidine-1-carboxylic acid *tert*-butyl ester | 3.64 | 466.1 [*M* + H]⁺ |
| **263** | 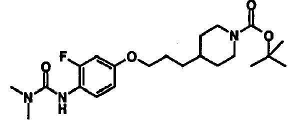 | 4-{3-[4-(3,3-Dimethylureido)-3-fluorophenoxy]propyl}-piperidine-1-carboxylic acid *tert-* butyl ester | 3.76 | 424.1 [*M* + H]⁺ |
| **264** | 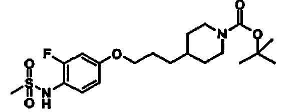 | 4-[3-(3-Fluoro-4-methanesulfonylaminophenoxy)-propyl]piperidine-1-carboxylic acid *tert*-butyl ester | 3.77 | 431.1 [*M* + H]⁺ |

**Example 265:** 4-{3-[4-(Dimethylphosphinoyl)-3-fluorophenoxy]propyl)piperidine-1-carboxylic acid *tert*-butyl ester 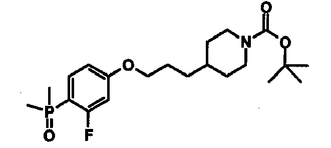 *n*-BuLi (0.84 mL of a 1.6M solution in hexane, 1.34 mmol) was added dropwise to a stirred solution of 4-[3-(4-bromo-3-fluorophenoxy)propyl]piperidine-1-carboxylic acid *tert-*butyl ester (see **Preparation 19,** 560 mg, 1.34 mmol) in anhydrous THF (10 mL) at -78°C. After 45 min, a solution of Me₂P(O)Cl (100 mg, 0.89 mmol) in anhydrous THF (1 mL) was added. The reaction was warmed to -30°C over 1 h, then H₂O (1 mL) was added. The mixture was partitioned between EtOAc (100 mL) and brine (100 mL). The organic extracts were dried (Na₂SO₄), filtered, concentrated and piurified by column chromatography (MeOH-EtOAc, 1:9) to yield the title compound: RT = 3.65 min; *m*/*z* (ES⁺) = 414.1 [*M* + H]⁺.

The biological activity of the compounds of the invention may be tested in the following assay systems:

### Yeast Reporter Assay

The yeast cell-based reporter assays have previously been described in the literature (e.g. see Miret J. J. et al, 2002, J. Biol. Chem., 277:6881-6887; Campbell R.M. et al,1999, Bioorg. Med. Chem. Lett., 9:2413-2418; King K. et al, 1990, Science, 250:121-123); WO 99/14344; WO 00/12704; and US 6,100,042). Briefly, yeast cells have been engineered such that the endogenous yeast G-alpha (GPA1) has been deleted and replaced with G-protein chimeras constructed using multiple techniques. Additionally, the endogenous yeast GPCR, Ste3 has been deleted to allow for heterologous expression of a mammalian GPCR of choice. In the yeast, elements of the pheromone signaling transduction pathway, which are conserved in eukaryotic cells (for example, the mitogen-activated protein kinase pathway), drive the expression of Fus1. By placing β-galactosidase (LacZ) under the control of the Fus1 promoter (Fus1p), a system has been developed whereby receptor activation leads to an enzymatic read-out.

Yeast cells were transformed by an adaptation of the lithium acetate method described by Agatep et al, (Agatep, R. et al, 1998, Transformation of Saccharomyces cerevisiae by the lithium acetate/single-stranded carrier DNA/polyethylene glycol (LiAc/ss-DNA/PEG) protocol. Technical Tips Online, Trends Journals, Elsevier). Briefly, yeast cells were grown overnight on yeast tryptone plates (YT). Carrier single-stranded DNA (10µg), 2µg of each of two Fuslp-LacZ reporter plasmids (one with URA selection marker and one with TRP), 2µg of GPR119 (human or mouse receptor) in yeast expression vector (2µg origin of replication) and a lithium acetate/ polyethylene glycol/ TE buffer was pipetted into an Eppendorf tube. The yeast expression plasmid containing the receptor/ no receptor control has a LEU marker. Yeast cells were inoculated into this mixture and the reaction proceeds at 30°C for 60min. The yeast cells were then heat-shocked at 42°C for 15min. The cells were then washed and spread on selection plates. The selection plates are synthetic defined yeast media minus LEU, URA and TRP (SD-LUT). After incubating at 30°C for 2-3 days, colonies that grow on the selection plates were then tested in the LacZ assay.

In order to perform fluorimetric enzyme assays for β-galactosidase, yeast cells carrying the human or mouse GPR119 receptor were grown overnight in liquid SD-LUT medium to an unsaturated concentration (i.e. the cells were still dividing and had not yet reached stationary phase). They were diluted in fresh medium to an optimal assay concentration and 90µl of yeast cells added to 96-well black polystyrene plates (Costar). Compounds, dissolved in DMSO and diluted in a 10% DMSO solution to 10X concentration, were added to the plates and the plates placed at 30°C for 4h. After 4h, the substrate for the β-galactosidase was added to each well. In these experiments, Fluorescein di (β-D-galactopyranoside) was used (FDG), a substrate for the enzyme that releases fluorescein, allowing a fluorimetric read-out. 20µl per well of 500µM FDG/2.5% Triton X100 was added (the detergent was necessary to render the cells permeable). After incubation of the cells with the substrate for 60min, 20µl per well of 1M sodium carbonate was added to terminate the reaction and enhance the fluorescent signal. The plates were then read in a fluorimeter at 485/535nm.

The compounds of the invention give an increase in fluorescent signal of at least ∼ 1.5-fold that of the background signal (i.e. the signal obtained in the presence of 1% DMSO without compound). Compounds of the invention which give an increase of at least 5-fold may be preferred.

### cAMP Assay

A stable cell line expressing recombinant human GPR119 was established and this cell line was used to investigate the effect of compounds of the invention on intracellular levels of cyclic AMP (cAMP). The cell monolayers were washed with phosphate buffered saline and stimulated at 37°C for 30min with various concentrations of compound in stimulation buffer plus 1% DMSO. Cells were then lysed and cAMP content determined using the Perkin Elmer AlphaScreen^{™} (Amplified Luminescent Proximity Homogeneous Assay) cAMP kit. Buffers and assay conditions were as described in the manufacturer's protocol.

Compounds of the invention produced a concentration-dependent increase in intracellular cAMP level and generally had an EC₅₀ of <10µM. Compounds showing and EC₅₀ of less than 1µM in the cAMP assay may be preferred.

### In vivo feeding study

The effect of compounds of the invention on body weight and food and water intake was examined in freely-feeding male Sprague-Dawley rats maintained on reverse-phase lighting. Test compounds and reference compounds were dosed by appropriate routes of administration (e.g. intraperitoneally or orally) and measurements made over the following 24 h. Rats were individually housed in polypropylene cages with metal grid floors at a temperature of 21±4°C and 55±20% humidity. Polypropylene trays with cage pads were placed beneath each cage to detect any food spillage. Animals were maintained on a reverse phase light-dark cycle (lights off for 8 h from 09.30-17.30 h) during which time the room was illuminated by red light. Animals had free access to a standard powdered rat diet and tap water during a two week acclimatization period. The diet was contained in glass feeding jars with aluminum lids. Each lid had a 3-4 cm hole in it to allow access to the food. Animals, feeding jars and water bottles were weighed (to the nearest 0.1 g) at the onset of the dark period. The feeding jars and water bottles were subsequently measured 1, 2, 4, 6 and 24 h after animals were dosed with a compound of the invention and any significant differences between the treatment groups at baseline compared to vehicle-treated controls.

Selected compounds of the invention showed a statistically significant hypophagic effect at one or more time points at a dose of ≤ 100mg/kg.

### Anti-diabetic effects of compounds of the invention in an in-vitro model of pancreatic beta cells (MIT-T15)

### Cell Culture

HIT-T15 cells (passage 60) were obtained from ATCC, and were cultured in RPMI1640 medium supplemented with 10% fetal calf serum and 30nM sodium selenite. All experiments were done with cells at less than passage 70, in accordance with the literature, which describes altered properties of this cell line at passage numbers above 81 (Zhang HJ, Walseth TF, Robertson RP. Insulin secretion and cAMP metabolism in HIT cells. Reciprocal and serial passage-dependent relationships. Diabetes. 1989 Jan;38(1):44-8).

### cAMP assay

HIT-T15 cells were plated in standard culture medium in 96-well plates at 100,000 cells/ 0.1ml/ well and cultured for 24 hr and the medium was then discarded. Cells were incubated for 15min at room temperature with 100µl stimulation buffer (Hanks buffered salt solution, 5mM HEPES, 0.5mM IBMX, 0.1 % BSA, pH 7.4). This was discarded and replaced with compound dilutions over the range 0.001, 0.003, 0.01, 0.03, 0.1, 0.3,1, 3, 10, 30 µM in stimulation buffer in the presence of 0.5% DMSO. Cells were incubated at room temperature for 30min. Then 75ul lysis buffer (5mM HEPES, 0.3% Tween-20,0.1% BSA, pH 7.4) was added per well and the plate was shaken at 900 rpm for 20 min. Particulate matter was removed by centrifugation at 3000rpm for 5min, then the samples were transferred in duplicate to 384-well plates, and processed following the Perkin Elmer AlphaScreen cAMP assay kit instructions. Briefly 25µl reactions were set up containing 8µl sample, 5µl acceptor bead mix and 12µl detection mix, such that the concentration of the final reaction components is the same as stated in the kit instructions. Reactions were incubated at room temperature for 150min, and the plate was read using a Packard Fusion instrument. Measurements for cAMP were compared to a standard curve of known cAMP amounts (0.01, 0.03, 0.1, 0.3, 1, 3,10, 30,100, 300,1000 nM) to convert the readings to absolute cAMP amounts. Data was analysed using XLfit 3 software.

Representative compounds of the invention were found to increase cAMP at an EC₅₀ of less than 10 µM. Compounds showing an EC₅₀ of less than 1 µM in the cAMP assay may be preferred.

### Insulin secretion assay

HIT-T15 cells were plated in standard culture medium in 12-well plates at 106 cells/ 1 ml/ well and cultured for 3 days and the medium was then discarded. Cells were washed x 2 with supplemented Krebs-Ringer buffer (KRB) containing 119 mM NaCl, 4.74 mM KCl, 2.54 mM CaCl₂, 1.19 mM MgSO₄, 1.19 mM KH2PO4,25 mM NaHCO₃, 10mM HEPES at pH 7.4 and 0.1% bovine serum albumin. Cells were incubated with 1ml KRB at 37°C for 30 min which was then discarded. This was followed by a second incubation with KRB for 30 min, which was collected and used to measure basal insulin secretion levels for each well. Compound dilutions (0, 0.1, 0.3, 1, 3,10 uM) were then added to duplicate wells in 1ml KRB, supplemented with 5.6 mM glucose. After 30 min incubation at 37°C samples were removed for determination of insulin levels. Measurement of insulin was done using the Mercodia Rat insulin ELISA kit, following the manufacturers instructions, with a standard curve of known insulin concentrations. For each well insulin levels were corrected by subtraction of the basal secretion level from the pre-incubation in the absence of glucose. Data was analysed using XLfit 3 software.

Representative compounds of the invention were found to increase insulin secretion at an EC₅₀ of less than 10 µM. Compounds showing an EC₅₀ of less than 1µM in the insulin secretion assay may be preferred.

### Oral Glucose Tolerance Tests

The effects of compounds of the invention on oral glucose (Glc) tolerance were evaluated in male C57B1/6 or male *ob*/*ob* mice. Food was withdrawn 5 h before administration of Glc and remained withdrawn throughout the study. Mice had free access to water during the study. A cut was made to the animals' tails, then blood (20 µL) was removed for measurement of basal Glc levels 45 min before administration of the Glc load. Then, the mice were weighed and dosed orally with test compound or vehicle (20% aqueous hydroxypropyl-*β*-cyclodextrin or 25% aqueous Gelucire 44/14) 30 min before the removal of an additional blood sample (20 µL) and treatment with the Glc load (2-5 g kg⁻¹ p.o.). Blood samples (20 µL) were then taken 25, 50, 80, 120, and 180 min after Glc administration. The 20 µL blood samples for measurement of Glc levels were taken from the cut tip of the tail into disposable micro-pipettes (Dade Diagnostics Inc., Puerto Rico) and the sample added to 480 µL of haemolysis reagent. Duplicate 20 µL aliquots of the diluted haemolysed blood were then added to 180 µL of Trinders glucose reagent (Sigma enzymatic (Trinder) colorimetric method) in a 96-well assay plate. After mixing, the samples were left at rt for 30 min before being read against Glc standards (Sigma glucose/urea nitrogen combined standard set). Representative compounds of the invention statistically reduced the Glc excursion at doses ≤100 mg kg⁻¹.

## Claims

1. A compound of formula (I), or a pharmaceutically acceptable salt thereof: 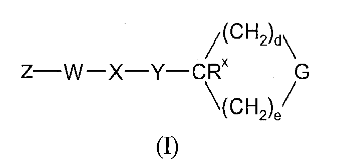
wherein Z is phenyl or a 5- or 6-membered heteroaryl group containing up to four heteroatoms selected from O, N and S, any of which may be optionally substituted by one or more substituents selected from halo, C₁₋₄ alkyl, C₁₋₄ fluoroalkyl, C₁₋₄ hydroxyalkyl, C₂₋₄ alkenyl, C₂₋₄ alkynyl, C₃₋₇ cycloalkyl, aryl, OR¹, CN, NO₂ -(CH₂)ⱼ-S(O)ₘR¹, -(CH₂)ⱼ-C(O)NR¹R¹¹, NR¹R¹¹, NR²C(O)R¹, NR²C(O)NR¹R¹¹, NR²SO₂R¹, SO₂NR¹R¹¹, C(O)R², C(O)OR², -P(O)(CH₃)₂, -(CH₂)ⱼ-(4- to 7-membered heterocyclyl) or -(CH₂)ⱼ-(5- to 6-membered heteroaryl); provided that Z is not optionally substituted 3- or 4-pyridyl;
m is 0, 1 or 2;
j is 0, 1 or 2;
W and Y are independently a bond, an unbranched or a branched C₁₋₄ alkylene optionally substituted by hydroxy or C₁₋₃alkoxy, or an unbranched or a branched C₂₋₄ alkenylene;
X is selected from CH₂, O, S, CH(OH), CH(halogen), CF₂, C(O), C(O)O, C(O)S, SC(O), C(O)CH₂S, C(O)CH₂C(OH), C(OH)CH₂C(O), C(O)CH₂C(O), OC(O), NR⁵, CH(NR⁵R⁵⁵), C(O)NR², NR² C(O), S(O) and S(O)₂;
R^{x} is hydrogen or hydroxy;
G is CHR³, N-C(O)OR⁴, N-C(O)NR⁴R⁵, N-C₁₋₄alkylene-C(O)OR⁴, N-C(O)C(O)OR⁴, N-S(O)₂R⁴ N-C(O)R⁴ or N-P(O)(O-Ph)₂; or N-heterocyclyl or N-heteroaryl, either of which may optionally be substituted by one or two groups selected from C₁₋₄ alkyl, C₁₋₄ alkoxy or halogen;
R¹ and R¹¹ are independently hydrogen, C₁₋₄ alkyl, which may optionally be substituted by halo, hydroxy, C₁₋₄ alkoxy-, aryloxy-, arylC₁₋₄ alkoxy-, C₁₋₄ allcylthio-, C₃₋₇ heterocyclyl,
-C(O)OR⁷ or N(R²)₂; or may be C₃₋₇ cycloalkyl, aryl, heterocyclyl or heteroaryl, wherein the cyclic groups may be substituted with one or more substituents selected from halo, C₁₋₄ alkyl, C₁₋₄ fluoroalkyl, OR⁶, CN, SO₂CH₃, N(R²)₂ and NO₂; or taken together R¹ and R¹¹ may form a 5- or 6-membered heterocyclic ring optionally substituted by hydroxy, C₁₋₄ alkyl or C₁₋₄ hydroxyalkyl and optionally containing a further heteroatom selected from O and NR²; or R¹¹ is C₁₋₄ alkyloxy-;
R² are independently hydrogen or C₁₋₄ alkyl; or a group N(R²)₂ may form a 4- to 7-membered heterocyclic ring optionally containing a further heteroatom selected from O and NR²;
R³ is C₃₋₆ alkyl;
R⁴ is C₁₋₈ alkyl, C₂₋₈ alkenyl or C₂₋₈ alkynyl, any of which may be optionally substituted by one or more substituents selected from halo, NR⁵R⁵⁵, OR⁵, C(O)OR⁵, OC(O)R⁵ and CN, and may contain a CH₂ group that is replaced by O or S; or a C₃₋₇cycloalkyl, aryl, heterocyclyl, heteroaryl, C₁₋₄alkyleneC₃-₇cycloalkyl, C₁₋₄alkylenearyl, C₁₋₄alkyleneheterocyclyl or C₁₋₄alkyleneheteroaryl, any of which may be substituted with one or more substituents selected from halo, C₁₋₄ alkyl, C₁₋₄ fluoroalkyl, OR⁵, CN, NR⁵R⁵⁵, SO₂Me, NO₂ and C(O)OR⁵;
R⁵ and R⁵⁵ are independently hydrogen or C₁₋₄alkyl; or taken together R⁵ and R⁵⁵ may form a 5- or 6-membered heterocyclic ring; or a group NR⁵ may represent NS(O)₂-(2-NO₂-C₆H₄);
R⁶ is hydrogen, C₁₋₂ alkyl or C₁₋₂ fluoroalkyl;
R⁷ is hydrogen or C₁₋₄ alkyl;
d is 0, 1, 2 or 3; and
e is 1, 2, 3, 4 or 5, provided that d + e is 2, 3, 4 or 5.

2. A compound according to claim 1, or a pharmaceutically acceptable salt thereof, wherein Z is optionally substituted phenyl or 6-membered hereroaryl.

3. A compound according to claim 2, or a pharmaceutically acceptable salt thereof, wherein Z is phenyl.

4. A compound according to any one of claims 1 to 3, or a pharmaceutically acceptable salt thereof, wherein Z is substituted by one or more halo, C₁₋₄ alkyl, C₁₋₄ fluoroalkyl, C₂₋₄ alkenyl, C₂₋₄ alkynyl, CN, S(O)ₘR¹, NR²C(O)NR¹R¹¹, C(O)NR¹R¹¹, SO₂NR¹R¹¹, COR², COOR² or a 5- or 6-membered heteroaryl groups.

5. A compound according to claim 2, or a pharmaceutically acceptable salt thereof, wherein Z is: 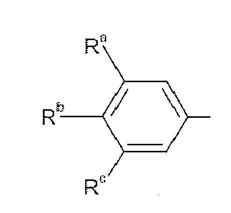 wherein:
R^{a} and R^{c} independently represent hydrogen, fluorine, chlorine, methyl or CN; and
R^{b} represents S(O)ₘR¹, C(O)NR¹R¹¹, SO₂NR¹R¹¹, NR²C(O)R¹, NR²SO₂R¹, NR²C(O)NR¹R¹¹or 5-membered heteroaryl.

6. A compound according to claim 5, or a pharmaceutically acceptable salt thereof, wherein:
R¹ and R¹¹ independently represent hydrogen or C₁₋₄ alkyl which may optionally be substituted by hydroxyl or NH₂, alternatively R¹ and R¹¹ taken together may form a heterocyclic ring, e.g. a 5- or 6-membered heterocyclic ring, optionally substituted with OH or CH₂OH; and
R² are independently hydrogen or C₁₋₄ alkyl; or a group N(R²)₂ may form a 4- to 7-membered heterocyclic ring optionally containing a further heteroatom selected from O and NR².

7. A compound according to any one of claims 1 to 6, or a pharmaceutically acceptable salt thereof, wherein G is N-C(O)OR⁴, N-C(O)NR⁴R⁵ or N-heteroaryl.

8. A compound according to claim 7, or a pharmaceutically acceptable salt thereof, wherein G is N-heteroaryl.

9. A compound according to claim 7, or a pharmaceutically acceptable salt thereof, wherein G is N-C(O)OR⁴.

10. A compound according to any one of claims 1 to 9, or a pharmaceutically acceptable salt thereof, wherein R⁴ represents C₁₋₈ alkyl, C₂₋₈ alkenyl or C₂₋₈ alkynyl optionally substituted by one or more halo atoms or CN, and may contain a CH₂ group that may be replaced by O or S; or a C₃₋₇cycloallcyl, aryl or C₁₋₄alkyleneC₃₋₇cycloalkyl, any of which may be substituted with one or more substituents selected from halo, C₁₋₄ alkyl, C₁₋₄ fluoroalkyl, OR⁵, CN, NR⁵R⁵⁵ , NO₂ or C(O)OC₁₋₄alkyl.

11. A compound according to claim 10, or a pharmaceutically acceptable salt thereof, wherein R⁴ represents C₂₋₅alkyl optionally substituted by one or more halo atoms or CN, and which may contain a CH₂ group that is replaced by O or S, or C₃₋₅cycloalkyl optionally substituted by C₁₋₄ alkyl.

12. A compound according to claim 10 or 11, or a pharmaceutically acceptable salt thereof, wherein the group represented by R⁴ is unsubstituted.

13. A compound according to any one of claims 1 to 12, or a pharmaceutically acceptable salt thereof, wherein d and e each represent 1.

14. A compound according to any one of claims 1 to 12, or a pharmaceutically acceptable salt thereof, wherein d and e each represent 2.

15. A compound according to any one of the preceding claims, or a pharmaceutically acceptable salt thereof, wherein W and Y do not both represent a bond.

16. A compound according to any one of the preceding claims, or a pharmaceutically acceptable salt thereof, wherein -W-X-Y- represents a 4 or 5 atom chain.

17. A compound according to any one of the preceding claims, or a pharmaceutically acceptable salt thereof, wherein W is a bond.

18. A compound according to any one of the preceding claims, or a pharmaceutically acceptable salt thereof, wherein X is CH₂, CF₂, O or NR⁵.

19. A compound according to any one of the preceding claims, or a pharmaceutically acceptable salt thereof, wherein Y is unbranched or a branched C₃₋₄ alkylene optionally substituted by hydroxy or C_{1- 3}alkoxy.

20. A compound according to any one of the preceding claims, or a pharmaceutically acceptable salt thereof, wherein R^{x} is hydrogen.

21. A compound according to claim 1 or a pharmaceutically acceptable salt thereof, of formula (Ic): 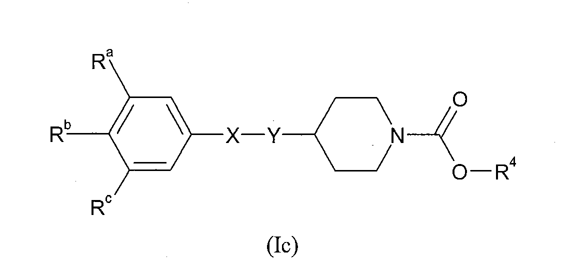 wherein:
R^{a} and R^{c} independently represent hydrogen, fluorine, chlorine, methyl or CN;
R^{b} represents S(O)ₘR¹, C(O)NR¹R¹¹, SO₂NR¹R¹¹ NR²C(O)R¹, NR²SO₂R¹, NR²C(O)NR¹R¹¹ or 5-membered heteroaryl;
X represents CH₂, CF₂, O, NH or C(O);
Y represents an unbranched or a branched C₃₋₄ alkylene group;
R⁴ represents C₂₋₅ alkyl or C₃₋₆ cycloalkyl which may optionally be substituted by methyl;
m represents 1 or 2;
R¹ and R¹¹ independently represent hydrogen or C₁₋₄ alkyl which may optionally be substituted by hydroxyl or NH₂, alternatively R¹ and R¹¹ taken together may form a heterocyclic ring, e.g. a 5- or 6-membered heterocyclic ring, optionally substituted with OH or CH₂OH; and
R² are independently hydrogen or C₁₋₄ alkyl; or a group N(R²)₂ may form a 4- to 7-membered heterocyclic ring optionally containing a further heteroatom selected from O and NR².

22. A compound of formula (I) as defined in any one of Examples 1 to 265, or a pharmaceutically acceptable salt thereof.

23. A pharmaceutical composition comprising a compound according to any one of claims 1 to two, or a pharmaceutically acceptable salt thereof and a pharmaceutically acceptable carrier.

24. A compound according to any one of claims 1 to 22, or a pharmaceutically acceptable salt thereof, for use in the regulation of satiety.

25. A compound according to any one of claims 1 to 22, or a pharmaceutically acceptable salt thereof, for use in the treatment or prevention of obesity.

26. A compound according to any one of claims 1 to 22, or a pharmaceutically acceptable salt thereof, for use in the treatment or prevention of diabetes.

27. A compound according to any one of claims 1 to 22, or a pharmaceutically acceptable salt thereof, for use in the treatment of metabolic syndrome (syndrome X), impaired glucose tolerance, hyperlipidemia, hypertriglyceridemia, hypercholesterolemia, low HDL levels or hypertension.

28. A compound according to any one of claims 1 to 22 or a pharmaceutically acceptable salt thereof, for use as a medicament.

29. A compound of formula (XII): 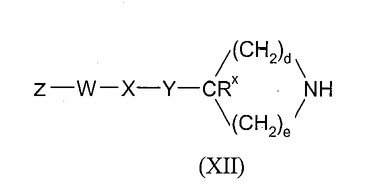 or a salt or protected derivative thereof, wherein the groups Z, W, X, Y, R^{x}, d and e are as defined in claim 1.
